# EUROPEAN PATENT SPECIFICATION

(11) **EP 2 066 269 B1**
(45) Date of publication and mention of the grant of the patent: **08.02.2012**
(21) Application number: 07838995.4
(22) Date of filing: 28.09.2007
(51) Int. Cl.: A61F 2/84, A61F 2/06

(54) **THORACIC AORTIC ANEURYSM REPAIR APPARATUS**
VORRICHTUNG ZUR REPARATUR VON AORTENANEURYSMEN IM BRUSTKORB
APPAREIL PERMETTANT DE RÉPARER UN ANEVRISME DE L'AORTE THORACIQUE

(30) Priority: 28.09.2006 US 848232 P; 28.09.2006 US 848198 P; 28.09.2006 US 848246 P; 28.09.2006 US 848197 P
(43) Date of publication of application: 10.06.2009
(73) Proprietor: Cook Medical Technologies LLC, Bloomington, IN 47404 (US)
(72) Inventor: YI TSENG, David, Princeton Junction, NJ 08550 (US); LLORT, Francisco, M., Skillman, NJ 08558 (US)
(74) Representative: Brun, Philippe Alexandre Georges
(86) International application number: PCT/US2007/020941
(87) International publication number: WO 2008/042266

(56) References cited:
- EP-A- 1 574 169
- WO-A-2005/025206
- DE-U1- 20 115 706
- US-A1- 2006 161 265

## Description

### Technical Field

The present invention relates to a thoracic aortic aneurysm repair apparatus and method.

### Background of the Invention

Conventional expandable endovascular prosthetic implants, such as stents and stent grafts, can be loaded into a catheter for insertion and deployment at a lesion site within a patient's vascular system. The catheter is typically configured to retain the prosthetic implant in an insertion configuration during delivery to the lesion site. At the lesion site, the prosthetic implant may be deployed, for example by retracting a catheter sheath from the prosthetic implant's proximal end (related to a position of the patient's heat) to the distal end.

Prosthetic implants must be accurately placed to sufficiently cover the target lesion site during endovascular treatments or procedures. With many conventional catheters, implant movement during deployment may occur from frictional interference or contact with the catheter sheath as the catheter sheath is retracted from about the implant. Such implant movement is an increased concern when implants having a high foreshortening percentage, such as a braided stent, are deployed. For example, during the deployment of a braided stent having a twenty percent foreshortening percentage, a proximal end and an opposing distal end of the stent tend to converge, which causes the stent to migrate from a desired anchoring position within the target lesion site. Moreover, covering undesired location, such as healthy vessels and/or branch vessels, due to inaccurate implant placement may cause unfavorable clinical consequences, such as branch vessel occlusion and/or restenosis. Unsuccessful attempts to prevent or limit undesirable implant movement during deployment have included applying a lubricious coating to the conventional implant to reduce the frictional contact between the implant and the catheter sheath.

Several additional complications with prosthetic implant placement are associated with certain conventional endovascular procedures, such as thoracic and abdominal endovascular procedures. For example, one phenomenon associated with thoracic stent graft placement is the "wind sock" effect. Due to a high blood flow rate, a volume gradient, and/or a pressure gradient in the thoracic region, the proximal end of the stent graft may be undesirably pushed or moved distally as a result of blood flow and/or the pressure gradient within the thoracic region during initial deployment of the stent graft. Such migration may result in inaccurate positioning of the stent graft with respect to the lesion site. Further, in abdominal aneurysm procedures, an inadequate distance between an edge of the renal artery and an edge of the aneurysm, commonly referred to as a "short neck," may prevent or limit a patient's acceptance of an endovascular treatment or procedure.

When a self-expanding stent graft is deployed within a curved portion of a blood vessel, the stent graft must correspond to and/or accommodate the curvature of the blood vessel. Conventional stent grafts include a plurality of discontinuous or noncontiguous stent elements that overlap each other to approximate the blood vessel curvature. Such element overlap in conventional stent grafts may result in angular deformity of the stent graft and/or an increased potential for structural damage to the stent graft and/or the blood vessel from repetitive pulsatile motion induced by blood flow and/or pressure variations. Conventional stent grafts formed of overlapping stent elements may also cause undesirable kinking or bending of the stent graft as the stent graft approximates the curvature of the vessel, which compromises the blood flow through the stent graft. Other conventional stent grafts that are bent or otherwise curved to approximate the curvature of the blood vessel may separate from the vessel wall because such conventional stent grafts do not smoothly accommodate the curved vessel portion. Such separation may lead to an attachment endoleak, a flap occlusion and/or portions of the stent graft projecting into the graft component of the stent graft and/or into the blood vessel wall, causing damage and/or injury.

US 6,344,054 discloses a prosthesis comprising a graft and an expandable stent underlying the graft, the stent and graft being linked together only at or near their distal ends. The difference in stent length between compressed and expanded radial configurations may exceed the difference in graft length between such configurations. An introducer for such prosthesis comprises an interior sheath axially mounted inside an exterior sheath, with the graft positioned between the interior and exterior sheaths and the stent compressed at least partially inside the interior sheath. A method for deploying the prosthesis using such an introducer may include positioning the introducer distal end upstream relative to proximally flowing endoluminal fluid so that the endoluminal fluid may flow between the graft and inner sheath during stent deployment.

### Summary of the Invention

The present invention seeks to provide an improved stent graft, and an improved stent graft delivery system .

According to an aspect of the present invention, there is provided a stent graft for deployment within a body vessel, including:
a tubular graft provided with a proximal end and a distal end;
a support stent, at least a portion of which lies within the tubular graft, provided with a proximal end and a distal end;
wherein the support stent includes at least one wire forming a first helical wire portion having a first translational direction about an axis of the support stent and a second helical wire portion having a second translational direction about the axis opposite the first translational direction;
wherein when the support stent is in a compressed delivery configuration, the support stent has a first length, and wherein when the support stent is in an expanded deployed configuration, the support stent has a second length which is less than the first length; and
wherein when in the delivery configuration, the support stent is attached to the graft only at or near the proximal end of the graft; the stent graft further including at least one locking mechanism at or near the distal end of the graft and configured to engage the support stent in the deployed configuration, thereby substantially to fix the diameter and length of the support stent in the deployed configuration.

According to an embodiment of the invention, there is provided a delivery system as in claim 15.

### Brief Description of the Drawing

Embodiments of the present invention are described below, by way of example only, with reference to the accompanying drawings, in which:
Figure 1 is a side view of an exemplary stent graft in a deployed configuration having a curvature of about 45°;
Figure 2 is a side view of an exemplary stent graft in a deployed configuration having a curvature of about 60°;
Figure 3 is a side view of an exemplary stent graft in a deployed configuration having a curvature of about 90°;
Figure 4 is a side view of an exemplary stent graft in a deployed configuration having an offset curvature of about 90°;
Figure 5 is a side view of an exemplary stent graft in a deployed configuration having a curvature of about 110°;
Figure 6 is a side view of an exemplary stent graft in a deployed configuration having a curvature of about 130°;
Figure 7 is a perspective view of a proximal end of an exemplary stent graft including an anchor stent;
Figure 8 is a side view of the proximal end of the stent graft shown in Figure 7;
Figure 9 is a perspective view of a distal end of an exemplary graft;
Figure 10 is a side view of an exemplary stent in an arcuate initial configuration;
Figure 11 is a side view of a partially deployed stent with the graft removed to show characteristics of the stent;
Figure 12 is an exploded perspective view of an exemplary repair system;
Figure 13 is a side view of the repair system shown in Figure 1 2 in an initial insertion position at a lesion site;
Figure 14 is a side view of the repair system shown in Figure 1 2 with an outer sheath retracted;
Figure 15 is a side view of the repair system shown in Figure 12 with an anchor stent deployed at a lesion site;
Figure 16 is a side view of the repair system shown in Figure 12 with an inner sheath retracted;
Figure 17 is an enlarged view of a portion of the repair system shown in Figure 16;
Figure 18 is a side view of the repair system shown in Figure 12 in a final deployed position at the lesion site;
Figure 19 is a schematic side view of a stent graft positioned with respect to a lesion site in a compressed insertion configuration;
Figure 20 is a schematic side view of the stent graft shown in Figure 19 with a distal end of the stent graft in a deployed configuration;
Figure 21 is a schematic side view of the stent graft shown in Figure 19 in a deployed configuration;
Figure 22 is a schematic side view of a stent graft positioned with respect to a lesion site in a compressed insertion configuration;
Figure 23 is a schematic side view of the stent graft shown in Figure 22 with a distal end of the stent graft in a deployed configuration;
Figure 24 is a schematic side view of the stent graft shown in Figure 22 in a deployed configuration;
Figure 25 is a front view of a portion of another delivery device;
Figure 26 is a perspective view of a delivery device suitable for use with the stent graft shown in Figure 22;
Figure 27 is a perspective view of a capture mechanism suitable for use with the delivery device shown in Figure 26;
Figure 28 is a perspective view of a nose cone suitable for use with the delivery device shown in Figure 26;
Figure 29 is a side view of an exemplary delivery system with a retraction element drawn;
Figure 30 is a side view of the delivery system shown in Figure 29 with a first locking element drawn;
Figure 31 is a sectional view of the delivery system shown in Figure 30 at sectional line A-A;
Figure 32 is a side view of an exemplary delivery system with a first retraction element drawn;
Figure 33 is a side view of the delivery system shown in Figure 32 with a second retraction element drawn;
Figure 34 is a sectional view of the delivery system shown in Figure 32 at sectional line B-B;
Figure 35 is a perspective view of an exemplary delivery system in an initial position;
Figure 36 is a perspective view of the delivery system shown in Figure 35 with a first retraction element drawn;
Figure 37 is a perspective view of the delivery system shown in Figure 35 with a second retraction element drawn;
Figure 38 is a perspective view of a portion of the delivery system shown in Figure 35;
Figure 39 is a sectional view of the portion of the delivery system shown in Figure 38;
Figure 40 is a perspective view of the delivery system shown in Figure 35 with the housing removed;
Figure 41 is a perspective view of the delivery system shown in Figure 35 with the housing removed;
Figure 42 is a sectional view of a portion of the delivery system shown in Figure 38 in another embodiment;
Figure 43 is a perspective view of a portion of the delivery system shown in Figure 38 in another embodiment;
Figure 44 is a perspective view of a portion of the delivery system shown in Figure 38 in another embodiment;
Figure 45 is a perspective view of a portion of the delivery system shown in Figure 38 in another embodiment;
Figure 46 is a perspective view of a portion of the delivery system shown in Figure 38 in another embodiment;
Figure 47 is a side view of an exemplary delivery system with a first retraction element drawn;
Figure 48 is a side view of the delivery system shown in Figure 47 with a second retraction element drawn;
Figure 49 is a side view of the delivery system shown in Figure 47 with a third retraction element drawn;
Figure 50 is a side view of an exemplary delivery system in an initial position;
Figure 51 is a sectional view of the delivery system shown in Figure 50;
Figure 52 is a partial secondary side view of the delivery system shown in Figure 50 with a retraction element drawn to an intermediate position;
Figure 53 is a partial sectional side view of the delivery system shown in Figure 50 with a retraction element drawn to a final position;
Figure 54 is a side view of an exemplary delivery system in an initial position;
Figure 55 is a sectional view of the delivery system shown in Figure 54;
Figure 56 is a partial sectional side view of the delivery system shown in Figure 54 with an outer sheath retracted;
Figure 57 is a partial sectional side view of the delivery system shown in Figure 54 with a retraction element drawn;
Figure 58 is a perspective view of a portion of the delivery system shown in Figure 54 in another embodiment;
Figure 59 is a partial sectional side view of an exemplary delivery system in an unlocked, initial position;
Figure 60 is a partial sectional side view of the delivery system shown in Figure 59 with an outer sheath retraction element drawn;
Figure 61 is a partial sectional side view of the delivery system shown in Figure 59 with a graft retraction element drawn;
Figure 62 is a side view of the delivery system shown in Figure 59 with an inner sheath retraction element drawn;
Figure 63 is a perspective view of an exemplary delivery system in an initial position;
Figure 64 is a perspective view of the delivery system shown in Figure 63 with an outer sheath retraction element drawn;
Figure 65 is a perspective view of the delivery system shown in Figure 63 with an inner sheath retraction element drawn;
Figure 66 is a sectional view of a portion of the delivery system shown in Figure 64;
Figure 67 is a side view of a portion of the delivery system shown in Figure 63 with the housing removed;
Figure 68 is a side view of a portion of the delivery system shown in Figure 65 with the housing removed;
Figure 69 is a perspective view of a portion of the delivery system shown in Figure 63 with a portion of the housing removed;
Figure 70 is a side view of an exemplary delivery system in an initial position;
Figure 71 is a side view of the delivery system shown in Figure 70 with an outer sheath retraction element drawn;
Figure 72 is a side view of the delivery system shown in Figure 70 with an inner sheath retraction element drawn;
Figure 73 is a perspective view of an exemplary delivery system in an initial position;
Figure 74 is a perspective view of the delivery system shown in Figure 73 with an outer sheath retraction element drawn;
Figure 75 is a perspective view of the delivery system shown in Figure 73 with an inner sheath retraction element drawn;
Figure 76 is a side view of a portion of the delivery system shown in Figure 73 with the housing removed;
Figure 77 is a side view of a portion of the delivery system shown in Figure 75 with the housing removed;
Figure 78 is a partial sectional side view of a portion of the delivery system shown in Figure 74;
Figure 79 is a partial sectional side view of a portion of the delivery system shown in Figure 74;
Figure 80 is a perspective view of a portion of the delivery system shown in Figure 73 with the housing removed;
Figure 81 is atop view of an exemplary delivery system in an initial position;
Figure 82 is a side view of the delivery system shown in Figure 81;
Figure 83 is a top view of the delivery system shown in Figure 81 with an outer sheath retraction element drawn;
Figure 84 is a top view of the delivery system shown in Figure 81 with an inner sheath retraction element drawn;
Figure 85 is a perspective view of an exemplary delivery system in an initial position;
Figure 86 is a perspective view of the delivery system shown in Figure 85 with an outer sheath retraction element drawn;
Figure 87 is a perspective view of the delivery system shown in Figure 85 with an inner sheath retraction element drawn;
Figure 88 is a sectional view of a portion of the delivery system shown in Figure 86;
Figure 89 is a side view of a portion of the delivery system shown in Figure 86 with the housing removed;
Figure 90 is a side view of a portion of the delivery system shown in Figure 87 with the housing removed;
Figure 91 is a side view of an exemplary graft release mechanism;
Figure 92 is a side view of an exemplary graft release mechanism;
Figure 93 is a side view of the graft release mechanism shown in Figure 92 with an outer sheath retracted;
Figure 94 is a side view of an exemplary graft release mechanism;
Figure 95 is a sectional side view of the graft release mechanism shown in Figure 94;
Figure 96 is a sectional side view of the graft release mechanism shown in Figure 94 with a retaining ring retracted;
Figure 97 is a side view of an exemplary graft release mechanism;
Figure 98 is a sectional side view of the graft release mechanism shown in Figure 97 with a retaining ring retracted;
Figure 99 is a sectional side view of the graft release mechanism shown in Figure 98 with a graft in a delivery configuration;
Figure 100 is a sectional side view of the anchor stent release mechanism shown in Figure 98 with a graft in a deployed configuration;
Figure 101 is aside view of an exemplary graft release mechanism;
Figure 102 is a side view of the anchor stent release mechanism shown in Figure 101 with a graft partially deployed;
Figure 103 is a side view of the graft release mechanism shown in Figure 101 with a graft partially deployed in another embodiment;
Figure 104 is a sectional side view of an exemplary support member advancement mechanism;
Figure 105 is a sectional side view of the support member advancement mechanism shown in Figure 104;
Figure 106 is a sectional side view of an exemplary support member advancement mechanism in an initial position;
Figure 107 is a sectional side view of the support member advancement mechanism shown in Figure 106, in a final position;
Figure 108 is a sectional side view of an exemplary support member advancement mechanism in an initial position;
Figure 109 is a sectional side view of the support member advancement mechanism shown in Figure 108 in a final position;
Figure 110 is a sectional side view of an exemplary support member advancement mechanism in an initial position;
Figure 111 is a sectional side view of a portion of the support member advancement mechanism shown in Figure 110;

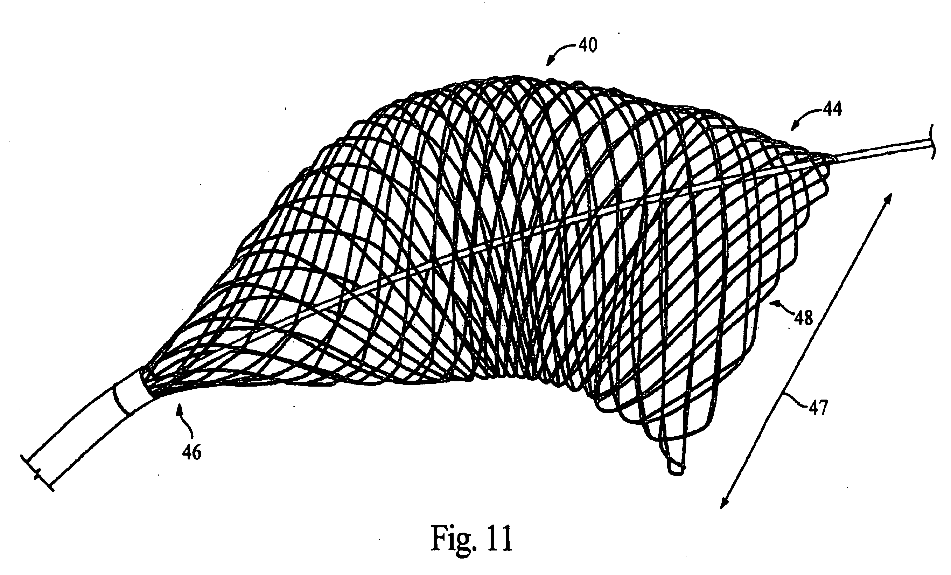

Figure 11 2 is a sectional side view of an exemplary support member advancement mechanism in an initial position;

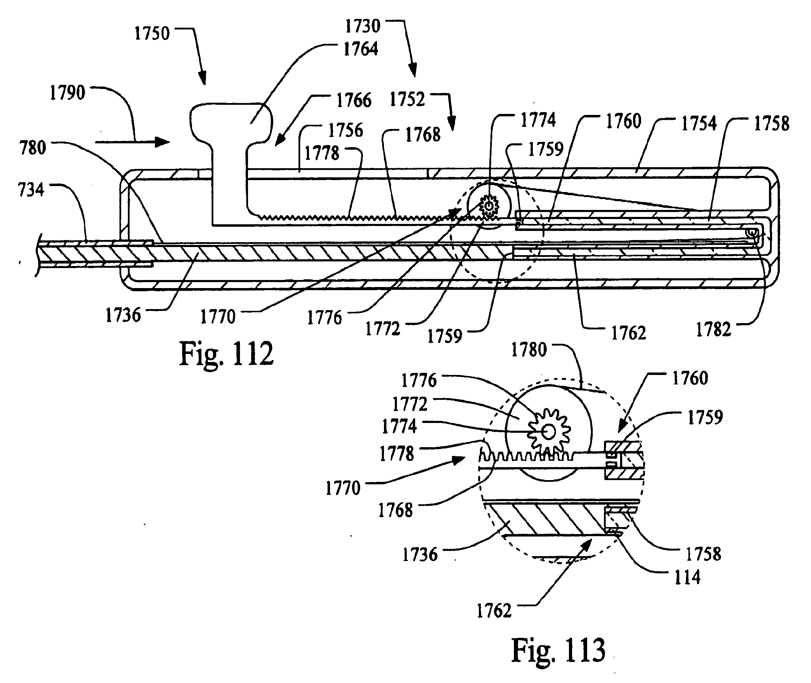

Figure 113 is a sectional side view of a portion of the support member advancement mechanism shown in Figure 112;

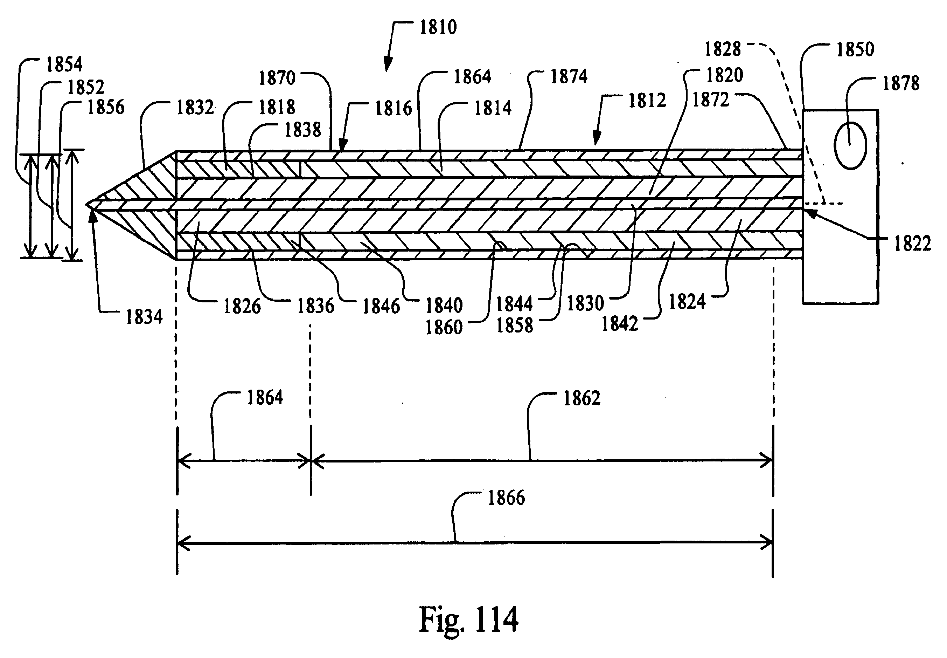

Figure 114 is a partial sectional view of an exemplary prosthesis delivery system;
Figure 11 5 is a partial sectional view of an exemplary prosthesis delivery system before deployment;

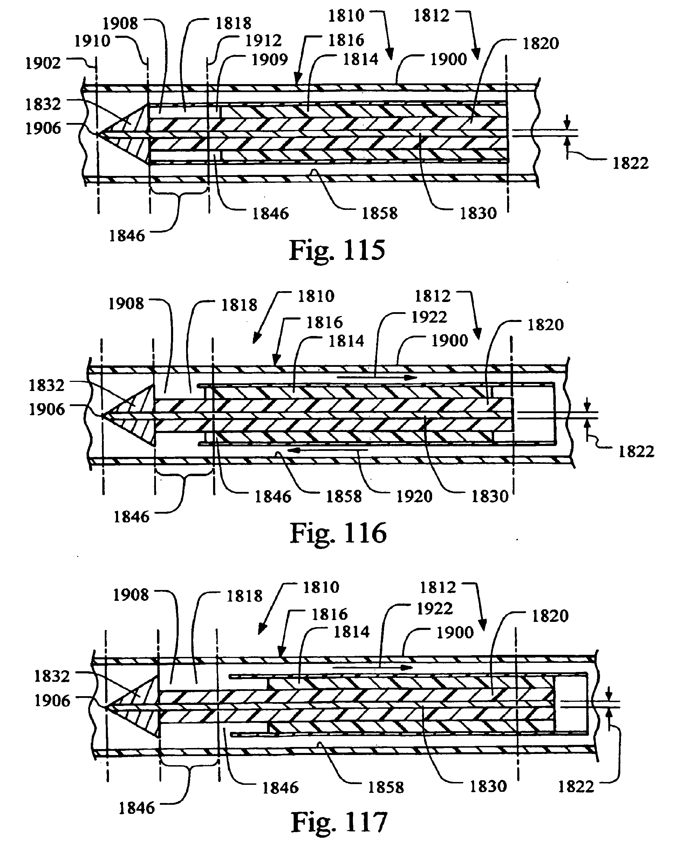

Figure 116 is a partial sectional view of an exemplary prosthesis delivery system during deployment; and
Figure 117 is a partial sectional view of an exemplary prosthesis delivery system after deployment.

### Detailed Description

The preferred examples described below relate to a stent, stent graft, apparatus and a method for repairing and/or treating aneurysms, such as a thoracic aortic aneurysm. The stent and stent graft have an arcuate configuration upon deployment of the stent during an endovascular procedure. More specifically, with the stent or stent graft positioned at a lesion site within a curved portion of a blood vessel, the stent or stent graft is adaptable to the anatomical curvature of the blood vessel.

The apparatus and method not path of the invention taught herein can facilitate precise and accurate positioning of the stent or stent graft at the desired lesion site while preventing or limiting undesirable stent or stent graft movement and/or migration. Further, a post-deployment placement of the stent or stent graft with respect to the lesion site can be accurately predicted or determined to prevent undesirable blockage or occlusion of branch vessels. In one embodiment, the stent graft is deployed from a distal end (related to a position of a patient's heart) to the proximal end of the stent graft. The distal end is commonly referred to as the "bottom" position and the proximal end is commonly referred to as the "up" position. By deploying the stent graft in a "bottom-up" procedure, a distal end of the stent graft is precisely and accurately positioned at the desired lesion site and a post-deployment placement of the stent graft with respect to the lesion site can be accurately predicted or determined to prevent undesirable blockage or occlusion of branch vessels, in particular the celiac artery.

The preferred embodiments are described below in reference to their application in connection with endovascular treatment of thoracic aortic aneurysms and dissections. However, it will be apparent to those skilled in the art and guided by the teachings herein provided that the invention is likewise applicable to any suitable endovascular treatment or procedure including, without limitation, endovascular treatment of abdominal aortic aneurysms.

Unless otherwise defined, all technical and scientific terms used herein have the same meaning as commonly understood by one of ordinary skill in the art to which this invention pertains. In case of conflict, the present document, including definitions, will control. Preferred methods and materials are described below, although methods and materials similar or equivalent to those described herein can be used in the practice or testing of the present invention. The materials, methods, and examples disclosed herein are illustrative only and not intended to be limiting.

### Definitions

As used herein, the term "adaptable" refers to the ability of the stent graft components to move and/or adjust to the curvature of the blood vessel. In one embodiment, the stent has a curvature in the deployed configuration different than the curvature in the initial configuration.

As used herein, references to "endovascular" are to be understood to refer to within blood vessels.

As used herein, the term "body vessel" means any tube-shaped body passage lumen that conducts fluid, including but not limited to blood vessels such as those of the human vasculature system, esophageal, intestinal, billiary, urethral and ureteral passages.

The term "implantable" refers to an ability of a prosthetic implant to be positioned, for any duration of time, at a location within a body, such as within a body vessel. Furthermore, the terms "implantation" and "implanted" refer to the positioning, for any duration of time, of a prosthetic implant at a location within a body, such as within a body vessel.

The term "biocompatible" refers to a material that is substantially non-toxic in the *in vivo* environment of its intended use, and that is not substantially rejected by the patient's physiological system (i.e., is non-antigenic). This can be gauged by the ability of a material to pass the biocompatibility tests set forth in International Standards Organization (ISO) Standard No. 10993 and/or the U.S. Pharmacopeia (USP) 23 and/or the U.S. Food and Drug Administration (FDA) blue book memorandum No. G95-1, entitled "Use of International Standard ISO-10993, Biological Evaluation of Medical Devices Part-1: Evaluation and Testing." Typically, these tests measure a material's toxicity, infectivity, pyrogenicity, irritation potential, reactivity, hemolytic activity, carcinogenicity and/or immunogenicity. A biocompatible structure or material, when introduced into a majority of patients, will not cause a significantly adverse, long-lived or escalating biological reaction or response, and is distinguished from a mild, transient inflammation which typically accompanies surgery or implantation of foreign objects into a living organism.

The term "string" as used herein is a generic term for a continuous strand of material. For example, strings may include, but are not limited to, monofilaments, filaments, fibers, yarns, cords, sutures, threads, and strings.

The term "retraction elements" as used herein is a generic term for any element able to impart motion to a second element coupled thereto. For example, retraction elements may include, but are not limited to, knobs, rotary knobs, levers, grips, slides, handles, shafts, arms, tabs, cranks, slides, pivots, and stems.

The term "locking element" as used herein is a generic term for any element able to limit or otherwise prevent movement of a second element coupled thereto. For example, locking elements may include, but are not limited to, knobs, levers, grips, handles, shafts, arms, cranks, pins, tabs, buttons, poles, pivots, rods, stems, and lockouts.

### Stent and Stent Graft

Stents and stent grafts according to the preferred embodiments may have an arcuate configuration upon deployment during an endovascular procedure, permitting adaptation to the anatomical curvature of the blood vessel due to inherent shape memory of the stent as a result of a secondary annealing process, as described in greater detail below. In one embodiment, the stent is formed or fabricated in an initial configuration having a curvature of about 0° to about 180°, In a particular embodiment, the stent has a curvature of about 180° in the initial configuration. In a deployed configuration, the stent is adaptable to approximate the curvature of the blood vessel portion or lesion site within which the stent is positioned. In one embodiment, the stent has a curvature in the deployed configuration different than the curvature in the initial configuration. At the lesion site, the stent is movable between a compressed and/or deformed insertion configuration and the deployed configuration to adjust to the curvature of the blood vessel.

In one embodiment, an endovascular stent graft or endograft 10 is provided. Stent graft 10 is positioned within a blood vessel, such as a patient's aorta, to reinforce a weak spot or lesion site in the blood vessel at or near an aneurysm. In one embodiment, stent graft 10 is positioned within the blood vessel at a curved portion of the blood vessel, such as at the aortic arch. Stent graft 10 provides strength to the injured or diseased blood vessel at the aneurysm and allows blood to flow through stent graft 10 without further stress and/or trauma to the aneurysm, thus, preventing enlargement and/or rupture of the blood vessel at the lesion site. In a particular embodiment, stent graft 10 includes a braided stent, as described in greater detail below. The braided stent facilitates smoothly approximating a curvature of the blood vessel without introducing additional stress points at the vessel wall at or near the lesion site. Further, because the braided stent is formed in one embodiment by a suitable annealing or heat treating process to an arcuate initial configuration, material straightening stresses on the blood vessel wall are eliminated or reduced. Thus, this further reduces stresses applied by the support stent and/or stent graft against the vessel wall.

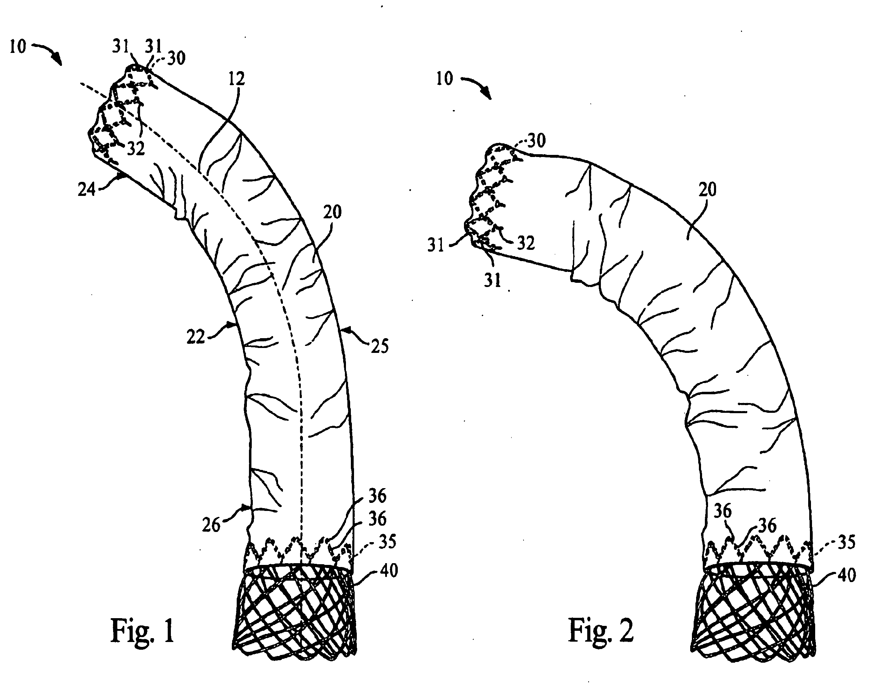

Stent graft 10 defines a longitudinal axis 12 along a length of stent graft 10, as shown in Figure 1. Stent graft 10 has any suitable length corresponding to a length of the lesion site at which the stent graft is to be positioned. Stent graft 10 is properly anchored tightly to an interior wall surface of the blood vessel proximally and/or distally to the lesion site.

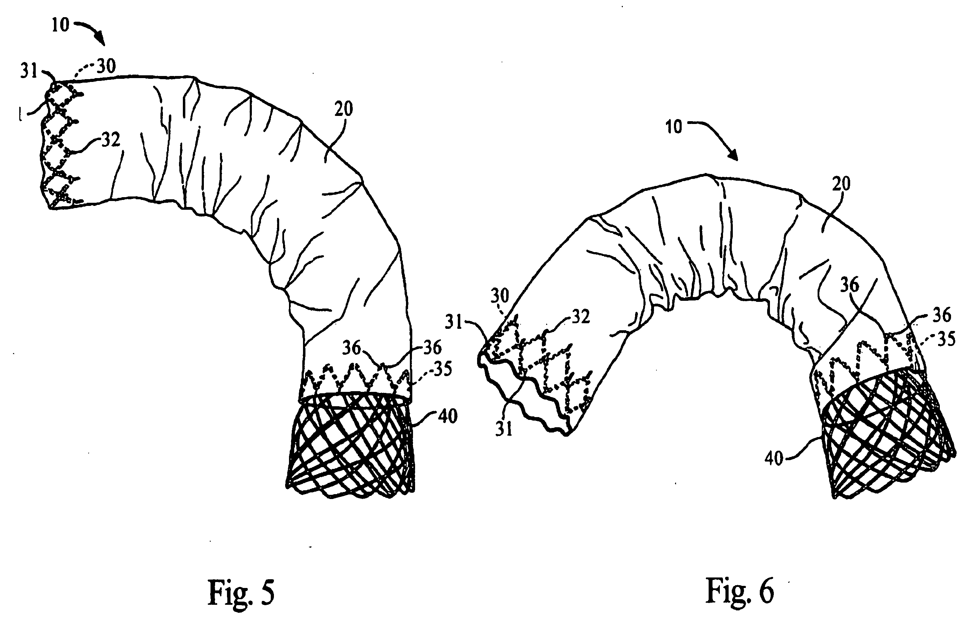

Figures 1-6 show an exemplary stent graft 10 in an arcuate deployed configuration having a curvature of about 0° to about 180°. In one embodiment, in the deployed configuration, stent graft 10 has a curvature substantially similar to the curvature of stent graft 10 in the initial configuration. In another embodiment, in the deployed configuration, stent graft 10 has a curvature different than the curvature of stent graft 10 in the initial configuration. Stent graft 10 is shown in the Figures at various deployed configurations, such as having a curvature of about 45°, as shown in Figure 1, to about 130°, as shown in Figure 6.

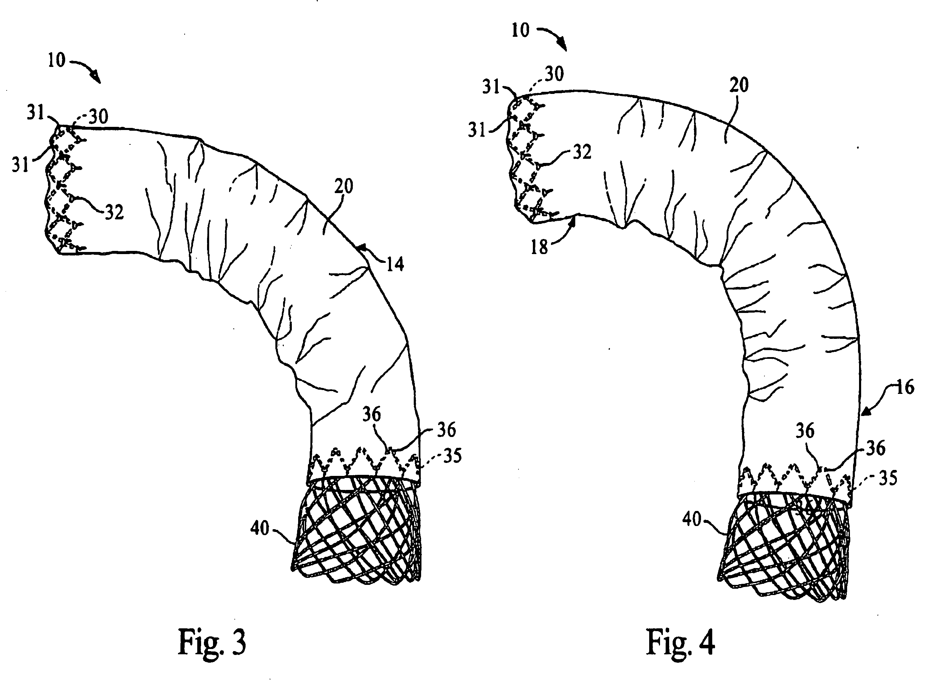

It will be apparent to those skilled in the art and guided by the teachings herein provided that stent graft 10 may have an initial configuration having any suitable curvature and/or a deployed configuration having any suitable curvature, which corresponds at least generally to a curvature of the blood vessel portion within which stent graft 10 is deployed. In other embodiments, in the deployed configuration stent graft 10 has a curvature of about 45° as shown in Figure 1, about 60° as shown in Figure 2, about 90° as shown in Figures 3 and 4, about 110° as shown in Figure 5 or about 130° as shown in Figure 6. Further, in one embodiment, an arcuate portion of stent graft 10 is positioned at a center portion 14 of stent graft 10 as shown in Figure 3, at or near a proximal portion 18 of stent graft 10 as shown in Figure 4 or at or near a distal portion 1 6 of stent graft 10 (not shown).

Further, in a particular embodiment, an external diameter of distal portion 16 of stent graft 10 is different than an external diameter of proximal portion 18 of stent graft 10. The external diameter of distal portion 16 corresponds to an internal diameter of the blood vessel at or near a distal end of the curved blood vessel portion and the external diameter of proximal portion 18 corresponds to an internal diameter of the blood vessel at or near a proximate end of the curved blood vessel portion. In a further particular embodiment, the external diameter of proximal portion 18 is greater than the external diameter of distal portion 16.

### Graft

As shown in Figures 1-6, stent graft 10 includes a graft 20 formed of a suitable biocompatible material. It is apparent to those skilled in the art and guided by the teachings herein provided that graft 20 may include any suitable biocompatible synthetic and/or biological material, which is suitable for facilitating repair to the injured or diseased blood vessel.

Graft 20 has a body 22 that defines a proximal end 24, a midsection 25 and an opposing distal end 26. In one embodiment, body 22 has a tubular configuration and is flexible to adapt to contact an inner surface of the curved blood vessel portion. In a particular embodiment, graft 20 is fabricated from a suitable fabric or cloth material that is flexible to contact an inner surface of the curved blood vessel portion and/or adjust to the curvature of the inner surface.

Referring to Figure 1, proximal end 24 is configured, upon deployment of graft 20 at the lesion site, to contact and/or sealingly anchor to the interior wall surface of the vessel at a proximal anchoring location. Similarly, distal end 26 is configured to contact and/or sealingly anchor to the interior wall surface at a distal anchoring location.

In one embodiment, graft 20 is positioned at the lesion site such that proximal end 24 is positioned proximally with respect to the lesion site. With proximal end 24 sealingly anchored to the interior wall surface, distal end 26 contacts and/or sealingly anchors to the interior wall surface of the vessel at the distal anchoring location positioned distal with respect to the lesion site. In another embodiment, graft 20 is positioned at the lesion site such that distal end 26 contacts or anchors to the interior wall surface distal to the lesion site. With distal end 26 contacting the interior wall surface, proximal end 24 sealingly anchors to the interior wall surface of the vessel at the proximal anchoring location positioned proximal with respect to the lesion site.

### Anchor Stent

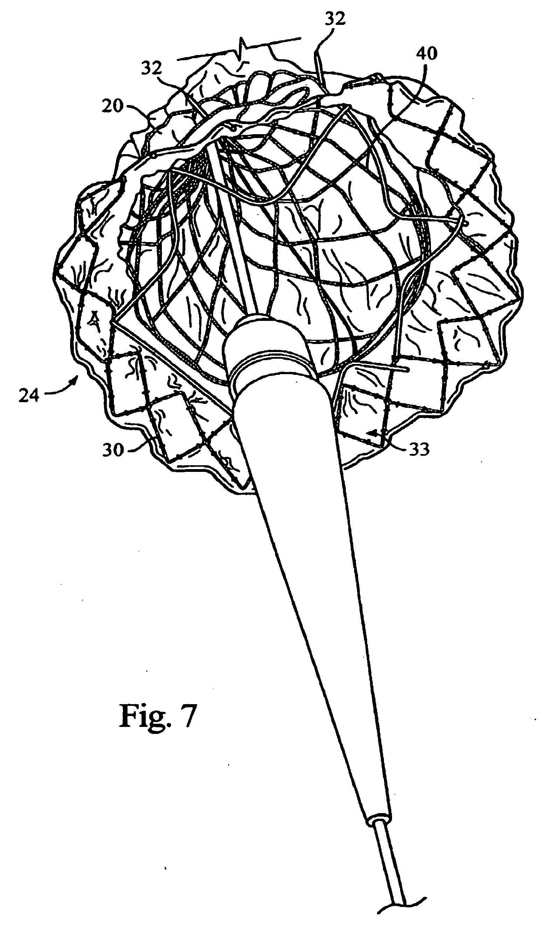

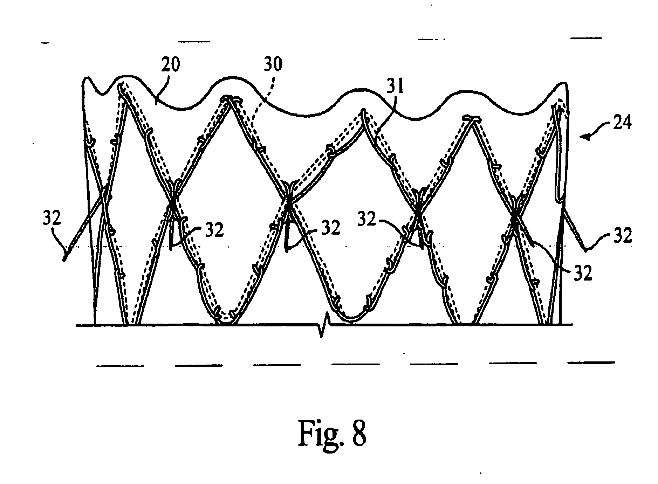

In one embodiment, an anchor stent 30 is coupled to graft 20 using a suitable coupling mechanism, such as a suture or stitching 31. Referring to Figures 7 and 8, anchor stent 30 is coupled to an inner surface of graft 20 at proximal end 24. In this embodiment, anchor stent 30 includes at least one projection, such as a plurality of barbs 32, which extend through graft 20 and outwardly with respect to an outer surface of graft 20. In a particular embodiment, barbs 32 are integrally formed with anchor stent 30. Barbs 32 are configured to penetrate and/or imbed into a blood vessel wall, such as the aortic wall, with stent graft 10 in the deployed configuration for facilitating retaining stent graft 10 accurately and properly positioned at the lesion site. It is apparent to those skilled in the art and guided by the teachings herein provided that any suitable component or mechanism may be incorporated into anchor stent 30 that is configured to interfere with and/or couple to the blood vessel wall to maintain stent graft 10 accurately and properly positioned at the lesion site.

As shown in Figure 7, anchor stent 30 is configured to form a plurality of diamond shaped voids 33. In one embodiment, anchor stent 30, including integrally formed barbs 32, is fabricated using a suitable laser cutting process. However, it will be apparent to those skilled in the art and guided by the teachings herein that any suitable fabrication process may be used to fabricate anchor stent 30 with barbs 32 either coupled to or integrally formed with anchor stent 30.

### Locking Ring

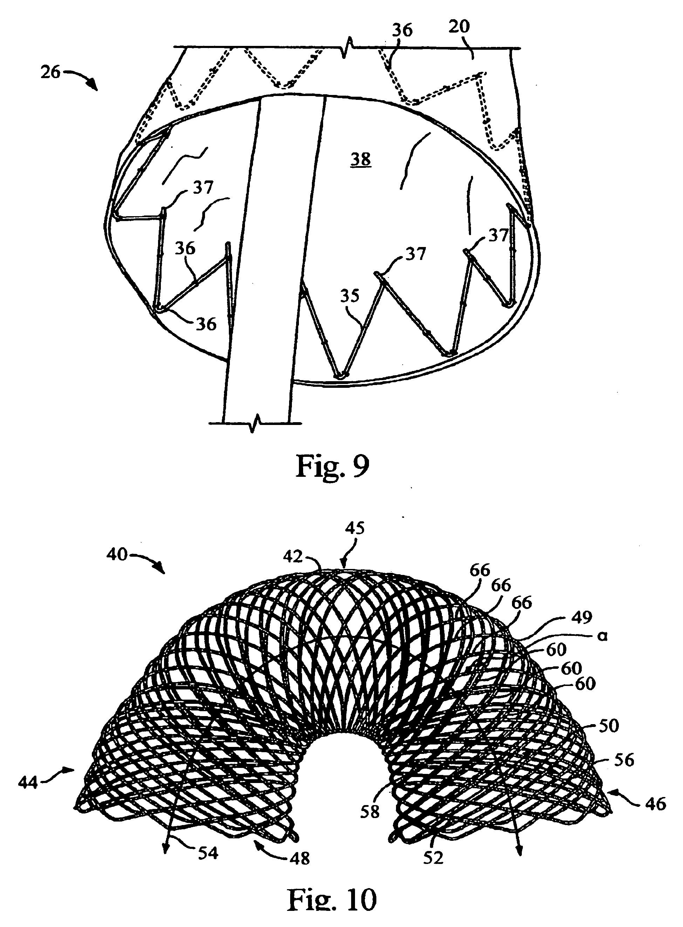

In one embodiment, a locking ring 35 is coupled to graft 20 at distal end 26. As shown in Figure 9, locking ring 35 is coupled to distal end 26 using a suitable coupling mechanism, such as a suture or stitching 36. In one embodiment, locking ring 35 includes at least one projection, such as a plurality of prongs 37, which extend inwardly from locking ring 35 into a passage 38 defined by graft 20. In a particular embodiment, prongs 37 are integrally formed with locking ring 35. Prongs 37 are configured to interfere with and/or couple to a support stent 40 positioned within graft 20 for facilitating maintaining support stent 40 accurately positioned within graft 20. In a particular embodiment, prongs 37 are relatively short and blunt, as opposed to barbs 32 which are relatively longer and sharp or pointed. It will be apparent to those skilled in the art and guided by the teachings herein provided that any suitable component or mechanism may be incorporated into locking ring 35 that is configured to interfere with and/or couple to support stent 40 to maintain support stent 40 accurately positioned within graft 20 without undesirably interfering with blood flow through passage 38. In one embodiment, locking ring 35, with integrally formed prongs 37, is fabricated using a suitable laser cutting process. However, will be apparent to those skilled in the art and guided by the teachings herein provided that any suitable fabrication process may be used to fabricate locking ring 35 with prongs 37 either coupled to or integrally formed with locking ring 35.

### Support Stent

Referring further to Figures 10 and 11, stent graft 10 includes support stent 40 positionable within graft 20 (not shown in these Figures) and coupled to graft proximal end 24 and/or graft distal end 26. Figure 10 shows support stent 40 in an arcuate initial configuration. Figure 11 shows support stent 40 in an insertion configuration and partially deployed, as described in greater detail below. In one embodiment, support stent 40 is a braided stent. Support stent 40 may be fabricated of braided Nitinol wire, which has desirable shape memory properties. In a particular embodiment, support stent 40 is fabricated from a continuous braided Nitinol wire, as described below. In other embodiments, support stent 40 is formed of a suitable biocompatible material including, without limitation, a suitable metal, such as stainless steel, platinum and/or titanium, alloy and/or composite material having suitable elastic properties. In another embodiment, at least a portion of support stent 40 is made of a polymeric material having suitable strength, such as polyetheretherketon (PEEK), polyethersulfon (PES) or polyimide (PI). It will be apparent to those skilled in the art and guided by the teachings herein provided that support stent 40 may include any suitable biocompatible synthetic and/or biological material, which is suitable for repair of the injured or diseased blood vessel. In other embodiments, support stent 40 is fabricated using any suitable process including, without limitation, annealing a straight stent into an arcuate configuration, laser cutting a bent or curved tube to form a continuous laser cut arcuate stent or casting a polymeric material to form a polymer cast arcuate stent.

Support stent 40 has a body 42 that defines a proximal end 44, a midsection 45 and an opposing distal end 46. In one embodiment, an external diameter of proximal end 44 and/or an external diameter of distal end 46 is greater than an external diameter of midsection 45. Further, the external diameter of proximal end 44 may be similar to or different from the external diameter of distal end 46. In one embodiment, body 42 has a tubular configuration and is expandable in a radial direction, as represent by directional arrow 47 in Figure 11, with respect to a longitudinal axis of support stent 40 that corresponds to longitudinal axis 12.

In one embodiment, support stent 40 is positioned within graft 20 and stent graft 10 is delivered to the lesion site using a suitable delivery device, such as a catheter, that is configured to retain stent graft 10 in a compressed insertion configuration as stent graft 10 is delivered through the patient's vascular system to the lesion site. At the lesion site, stent graft 10 is partially deployed. More specifically, support stent proximal end 44, coupled to graft proximal end 24, such as by sewing or stitching stent 40 to graft 20, is deployed. In one embodiment, anchor stent 30 expands radially outwardly with respect to graft 20 such that barbs 32 penetrate and/or imbed into the blood vessel wall. In a particular embodiment, with support stent proximal end 44 coupled to graft proximal end 24, support stent distal end 46 defines a freely movable end portion of support stent 40, e.g., support stent distal end 46 is not directly coupled or attached to graft 20. In one embodiment, with stent graft proximal end 18 coupled to the blood vessel wall, support stent distal end 46 is deployed. In another embodiment, stent graft distal end 16 is deployed. With stent graft distal end 16 contacting the blood vessel wall, stent graft proximal end 18 is deployed.

Support stent distal end 46 is expandable to contact an inner surface of graft 20 and couple to graft distal end 26. Support stent distal end 46 interferes with and/or couples to prongs 37 extending radially inwardly from locking ring 35 coupled to graft distal end 26. In this embodiment, prongs 37 interfere with support stent distal end 46 to align and maintain support stent 40 accurately positioned within graft 20. In the deployed configuration, support stent 40 defines a passage 48 through which blood flows, as shown in Figures 10 and 11.

Support stent 40 has a suitable length extending between support stent proximal end 44 and support stent distal end 46 and along the length of graft 20. In one embodiment, the length of support stent body 42 is greater than the length of graft body 22. In a particular embodiment, the length of support stent body 42 is at least 1 cm greater than the length of graft body 22. For example, support stent distal end 46 extends at least 1 cm in a distal direction along longitudinal axis 12 beyond graft distal end 26. In certain embodiments, support stent 40 is extendable over a variable range of lengths beyond graft distal end 26, as required by certain applications to cover a dissected portion of the aorta. Such length may approach at least about 30 cm in certain applications.

Referring further to Figures 10 and 11, in one embodiment, support stent 40 is a braided stent. As shown in Figure 10, braided stent 40 has an arcuate initial configuration, which is configured to correspond to a curvature of the blood vessel. In one embodiment, braided stent 40 includes a continuous structural wire 49 forming a first helical wire portion 50 having a first translational direction, as shown by direction arrow 52 in Figure 10, about an axis 54 of stent 40. Structural wire 49 further forms a second helical wire portion 56 having a second translational direction, as shown by direction arrow 58 in Figure 10, about axis 54 opposite the first translational direction and inter-wound with first helical wire portion 50. In one embodiment, first helical wire portion 50 and second helical wire portion 56 form a double helix wherein first helical wire portion 50 and second helical wire portion 56 are congruent helixes with a same axis, namely axis 54. Further, first helical wire portion 50 intersects and/or is wound with second helical wire portion 56 at a braiding angle α as shown in Figure 10. In one embodiment, braiding angle α is at least about 120°.

In another embodiment, braided stent 40 includes a first helical wire having a first translational direction, as shown by direction arrow 52 in Figure 10, about axis 54 of stent 40. Braided stent 40 further includes a second helical wire having a second translational direction, as shown by direction arrow 58 in Figure 10, about axis 54 opposite the first translational direction and inter-wound with the first helical wire. In one embodiment, the first helical wire and the second helical wire form a double helix wherein the first helical wire and the second helical wire are congruent helixes with a same axis, namely axis 54.

As shown in Figures 10 and 11, first helical wire portion 50 generally includes a plurality of coil segments or windings 60. Additionally, second helical wire portion 56 includes a plurality of coil segments or windings 66. Each coil winding 60 is movable with respect to adjacent coil windings 60 and/or each coil winding 66 is movable with respect to adjacent coil windings 66 to contact and form or adjust to an inner surface of the corresponding curved portion of the blood vessel. In one embodiment, first helical wire portion 50 and second helical wire portion 56 have an equal number of coil windings 60 and 66, respectively, such that in the deployed configuration, braided stent 40 smoothly approximates the curvature of the interior wall of the blood vessel. In this embodiment, support stent 40 is movable from the initial configuration to the deployed configuration to correspond with the curvature of the interior wall of the blood vessel, while eliminating or limiting individual stress points or areas exerted by support stent 40 on the interior wall of the blood vessel. Further, because support stent 40 has an arcuate initial configuration, support stent 40 does not exert undesirable forces against the interior vessel wall while positioned at the lesion site within the curved portion.

In one embodiment, support stent 40 is heat-treated to give the support stent 40 an arcuate initial configuration. In this embodiment, a force applied to support stent 40 may deform support stent 40. However, when the deformation force is removed, support stent 40 returns to the initial configuration. In a particular embodiment, support stent 40 includes an annealed material. In this particular embodiment, support stent 40 is annealed to form this in the arcuate initial configuration. In one embodiment, support stent 40 is fabricated by forming continuous structural wire 49 into first helical wire portion 50 and second helical wire portion 56. The formed support stent 40 is then annealed to move and retain the stent at the arcuate initial configuration. In this embodiment, axis 54 defines a curvature of support stent 40. During the annealing process, the material is exposed to an elevated temperature for an extended period of time and then slowly cooled. The microstructure of the material is changed as the material is heated and then slowly cooled to alter the mechanical properties of the material. The annealing process further negates any internal stresses developed within the material during the machining and/or casting processes. It will be apparent to those skilled in the art and guided by the teachings herein provided that any suitable process or treatment may be used to form support stent 40 in the arcuate initial configuration, including the methods described above.

In one embodiment, body 42 of support stent 40 has a differential compliance, e.g., a compliance that varies along a length of body 42, for facilitating adjustment to a curvature of the blood vessel at the lesion site. In a particular embodiment, proximal end 44 has a "soft" compliance or stiffness that at least approaches or approximates the physiological compliance of the blood vessel for facilitating positioning support stent 40 within a curved or angular portion of the blood vessel. The stiffness of proximal end 44 approaches or approximates the stiffness of the blood vessel to prevent or limit erosion of the blood vessel due to a radial force exerted by support stent 40 against the interior wall of the blood vessel with support stent 40 deployed. In this embodiment, distal end 46 has a greater stiffness than the stiffness of proximal end 44.

In one embodiment, a suitable heat treatment process facilitates adjusting a radial strength of at least a portion of body 42 to produce support stent 40 having differential compliance. In another embodiment, proximal end 44 is made of a softer material than a material used to make body 42 including distal end 46. Suitable materials include, without limitation, a metal material, an alloy material, such as a Nitinol material, or a polymeric material. In this embodiment, proximal end 44 is made of a material having a stiffness that complies with a stiffness of the blood vessel and distal end 46 is made of a material having a greater stiffness than the stiffness of proximal end 44. In yet another embodiment, distal end 46 is made of a material having a stiffness less than a stiffness of proximal end 44.

### Delivery System

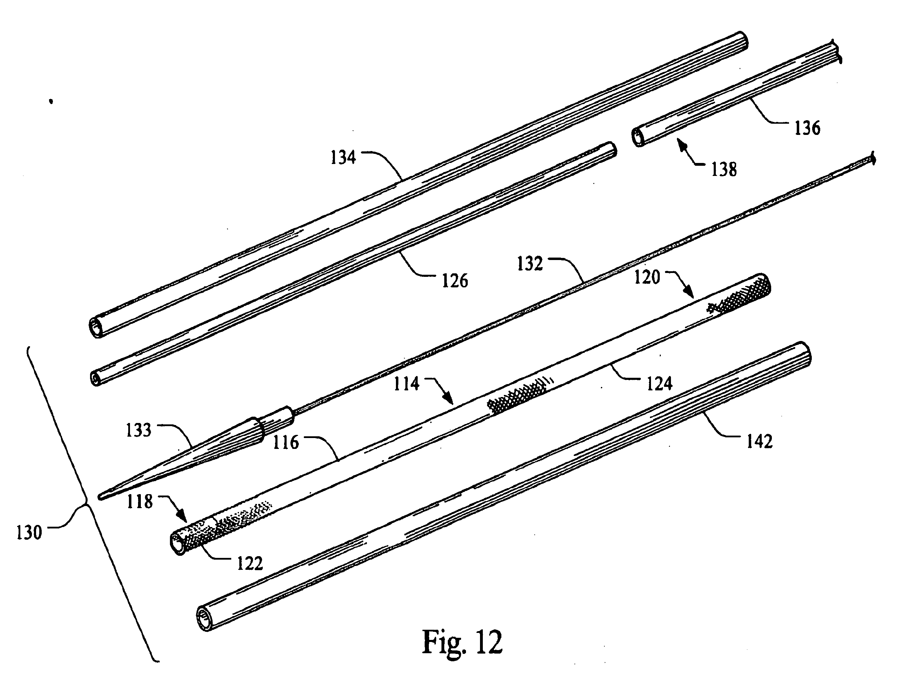

Figures 12-18 show a delivery system for delivering and/or deploying a prosthetic implant, such as a stent or a stent graft, at a lesion site during a thoracic aortic aneurysm repair procedure. During a thoracic aortic aneurysm repair procedure, a delivery system 130 delivers and/or positions a stent graft, for example stent graft 110, with respect to the lesion site at or near the aneurysm. Delivery system 130 includes a wire lumen 132 slidably positionable about a guide wire (not shown) initially positioned within a vessel of a patient. In one embodiment, the guide wire is advanced by the surgeon through the vessel from the patient's femoral artery and positioned within the aorta. Wire lumen 132 defines a passage (not shown) therethrough such that wire lumen 132 is slidably positioned about the guide wire. In one embodiment, a nose cone 133 is coupled to or integrated with wire lumen 132 for facilitating advancing the stent graft to the lesion site.

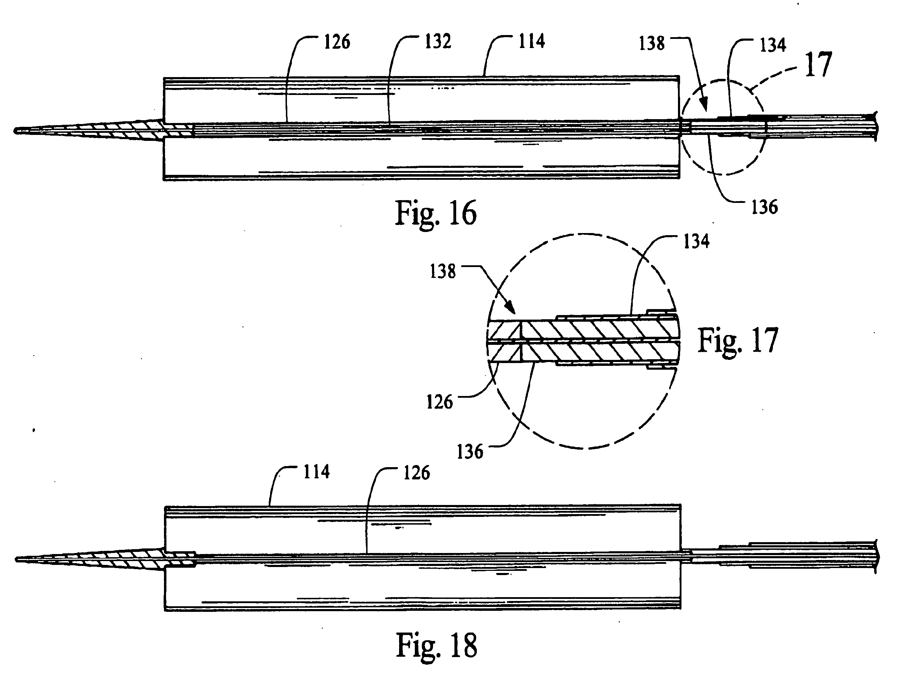

Referring further to Figures 16 and 17, in one embodiment support stent 126 is slidably positioned about wire lumen 132. An inner sheath 134 is retractably positioned about support stent 126 with support stent 126 in the compressed insertion configuration. Inner sheath 134 is positioned about at least a portion of support stent 126 to maintain support stent 126 in the compressed insertion configuration as stent graft 110 is advanced to the lesion site. With stent graft 110 positioned within the vessel as desired, inner sheath 134 is retractable for facilitating deployment of support stent 126 from the compressed insertion configuration to the expanded deployed configuration, as described in greater detail below.

In one embodiment, delivery system 130 includes a support member 136 slidably positioned about wire lumen 132. Support member 136 defines a proximal end 138 and an opposing distal end 140. Proximal end 138 contacts a distal end of support stent 126 with support stent 126 in the compressed insertion configuration, as shown in Figures 16 and 17. In one embodiment, support member 136 maintains a substantially constant force against support stent 126 as inner sheath 134 is retracted from about support stent 126 to prevent or limit undesirable movement of support stent 126 in the distal direction and retain support stent 126 properly positioned at the lesion site. In a particular embodiment, support stent 126 expands as inner sheath 134 is retracted with respect to support stent 126. In various embodiments, inner sheath 134 and support member 136 move in opposite directions to facilitate minimizing a foreshortening of support stent 126, such as a braided stent. In a particular embodiment, a ratio of opposing movement is about 1:1 to about 1:3.

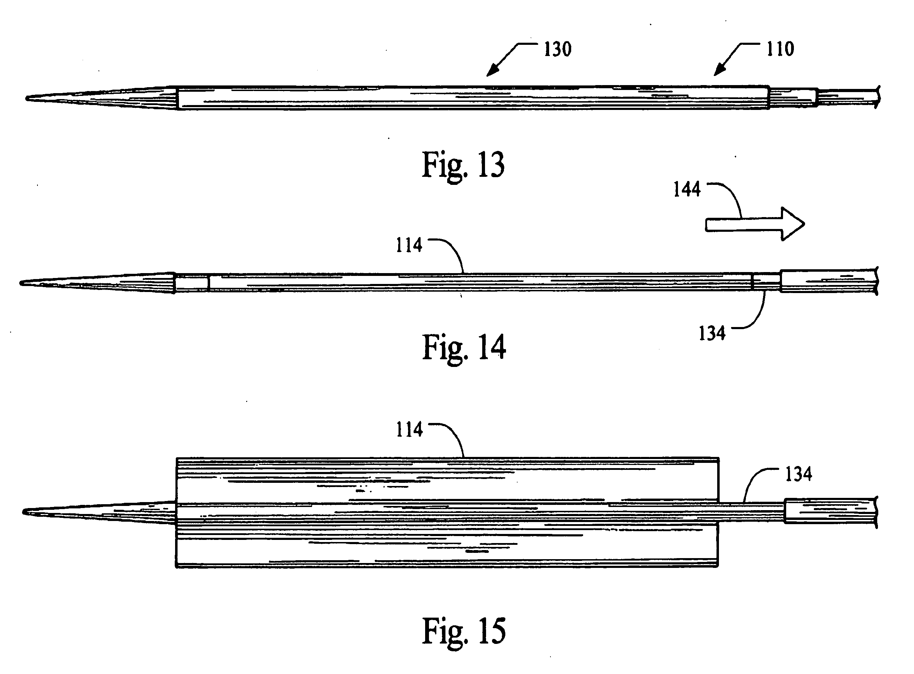

As shown in Figure 14, graft 114 is slidably positioned about inner sheath 134. In one embodiment, graft 114 includes anchor stent 30, and locking ring 35, as described above. An outer sheath 142 is retractably positioned about graft 114 with graft 114 in the delivery configuration. Outer sheath 142 is positioned about at least a portion of graft 114 to maintain graft 114 in the delivery configuration as stent graft 110 is advanced to the lesion site. With stent graft 110 positioned within the vessel as desired, outer sheath 142 is retractable for facilitating deployment of graft 114 from the delivery configuration to the deployed configuration, as described in greater detail below.

Referring to Figures 12-18, during a thoracic aortic aneurysm repair procedure, stent graft 110 is delivered to and deployed at the lesion site. In one embodiment, a guide wire is inserted through a patient's vasculature structure. With stent graft 110 positioned within delivery system 130 as shown in Figure 13, delivery system 130 is advanced to the lesion site along the guide wire. Delivery system 130 is positioned about the guide wire through the passage defined by lumen 132 with nose cone 133 at a leading end of delivery system 130.

With delivery system 130 at the lesion site, outer sheath 142 is moved in a distal direction, as shown by directional arrow 144 in Figure 14, to retract outer sheath 142 and expose at least a portion of graft 114. As shown in Figure 15, graft 114 is deployed at the lesion site. Graft 114 expands in a radial direction with respect to lumen 1 32 between the delivery configuration and the deployed configuration. In the deployed configuration, an outer radial surface of graft 114 contacts the interior surface of the vessel wall at the lesion site and graft 114 defines a passage therethrough. Proximal end 118 of graft 114 is positioned proximal to the aneurysm and distal end 120 is positioned distal to the aneurysm. In one embodiment, graft 114 includes anchor stent 30 and locking ring 35. Anchor stent 30 is positioned proximal to the aneurysm and locking ring 35 is positioned distal to the aneurysm.

In one embodiment, an actuator is operatively coupled to outer sheath 142, graft 114, inner sheath 134 and/or support stent 126. The actuator is activated, as described in greater detail below, to deploy graft 114 from the insertion configuration to a deployed configuration at the lesion site, as shown in Figure 15. In one embodiment, the actuator includes a handle configured to retract outer sheath 142 and deploy graft 114. In this embodiment, the actuator is also operatively coupled to inner sheath 134 and configured to retract inner sheath 134 to deploy support stent 126.

With the deployed graft 114 properly positioned at the lesion site, inner sheath 134 is retracted from about support stent 126 for facilitating expansion of support stent 126 from the compressed delivery configuration to the expanded deployed configuration, as shown in Figure 18. In the deployed configuration, an outer surface of support stent 126 contacts an inner surface of the graft 114. As shown in Figures 16 and 17, in one embodiment support member 136 is positioned about wire lumen 132 and contacts support stent 126 as inner sheath 134 is retracted to prevent or limit undesirable movement of support stent 126 with respect to the lesion site and maintain support stent 126 positioned at the lesion site. In a particular embodiment, support member 136 is movable in the proximal direction along the guide wire to contact support stent 126 as inner sheath 134 is retracted in the opposing distal direction as shown by directional arrow 144 (Figure 14). With graft 114 and support stent 126 deployed at the lesion site, the guide wire is retracted from within the vessel.

### "Bottom-Up" Deployment

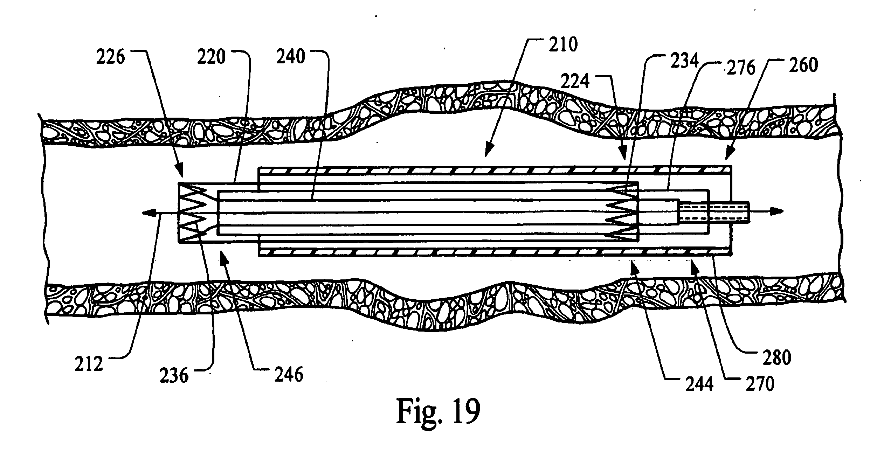

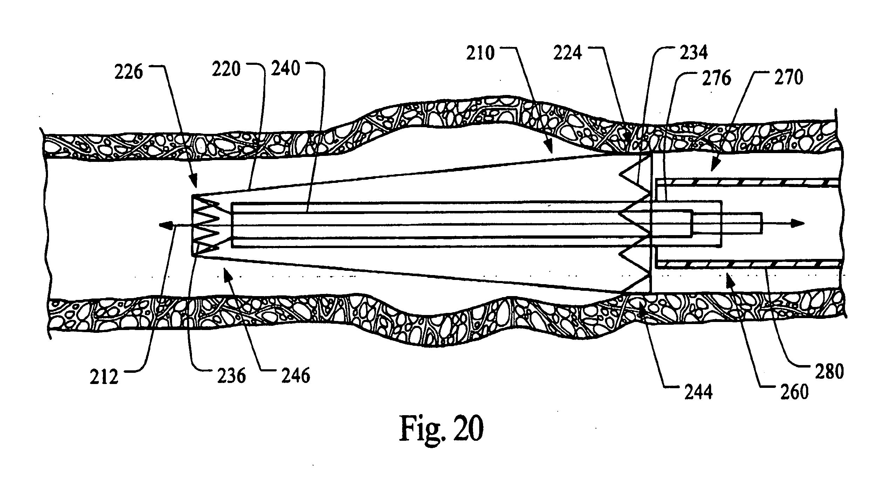

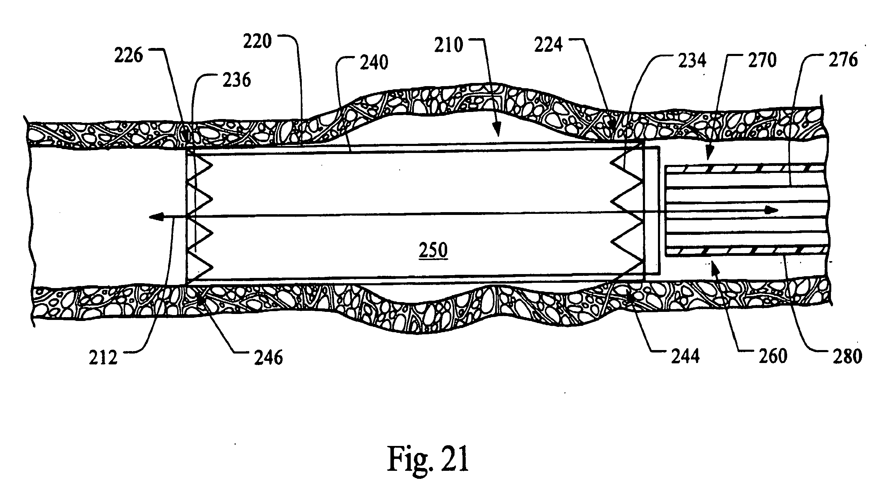

Referring to Figures 19-21, an apparatus 260 for delivering stent graft 210 to a lesion site during an endovascular procedure is provided. In one embodiment, outer sheath 280 covers at least a portion of graft 220 during delivery of stent graft 210 to the lesion site. Further, inner sheath 276 is positioned within outer sheath 280 and covers at least a portion of support stent 240 during delivery of stent graft 210 to the lesion site. At the lesion site, outer sheath 280 is movable in a distal direction with respect to longitudinal axis 212 to at least partially expose and/or deploy graft 220. In this embodiment, with graft 220 at least partially deployed, distal ring 234 contacts and/or anchors to the interior wall surface of the vessel. Inner sheath 276 is independently movable in the distal direction with respect to longitudinal axis 212 to deploy graft 220 and at least partially expose and/or deploy support stent 240. With support stent 240 at least partially deployed, anchor stent 236 is anchored to the interior wall surface. Support stent 240, including freely movable distal end 244, expands in an outward radial direction with respect to longitudinal axis 212 to contact an inner surface of graft 220 and form or define passage 250.

In one embodiment, a method of deploying a stent graft (which is not part of the invention) with respect to a lesion site during an endovascular procedure is provided. During the endovascular procedure, a small incision into the patient's skin is made above the femoral artery. The surgeon guides a guide wire into the femoral artery and advances the guide wire through the tortuous vascular structure to the aneurysm, e.g., the lesion site. In this embodiment, stent graft 210 is loaded into delivery device 270. Delivery device 270 is inserted over the guide wire and inserted into the femoral artery to advance stent graft 210 to the lesion site. Delivery device 270 is configured to retain stent graft 210 in a compressed or delivery configuration during delivery of stent graft 210 to the lesion site. In one embodiment, imaging equipment, such as an angiogram imaging system, is used to facilitate proper positioning of stent graft 210 with respect to the lesion site. Delivery device 270 carries stent graft 210 in the insertion configuration for facilitating advancement of stent graft 210 through the vascular structure, including the blood vessels.

With stent graft 210 positioned at or near the lesion site, the surgeon is able to move delivery device 270 in a proximal direction and/or a distal direction with respect to a position of the patient's heart to position distal ring 234 of stent graft 210 at a desired distal anchoring location with respect to the lesion site. In a particular embodiment, outer sheath 280 is partially withdrawn to partially deploy proximal end 226 of graft 220 before moving delivery device 270 to position locking ring 234. Outer sheath 280 is moved in the distal direction to withdraw outer sheath 280 from delivery device 270 and deploy distal end 224 of graft 220 including locking ring 234. Locking ring 234 moves radially outwardly with respect to longitudinal axis 212 to contact the interior wall surface of the vessel at the distal anchor location. Locking ring 234 contacts and/or is anchored to the interior wall surface. In one embodiment, locking ring 234 contacts and/or anchors to the interior wall surface proximal to an artery, such as the celiac artery, to prevent or limit obstruction of blood flow through the artery.

With locking ring 234 anchored at the distal.anchoring location, inner sheath 276 is moved in the distal direction to withdraw inner sheath 276 from delivery device 270 and deploy proximal end 226 of graft 220 including anchor stent 236 and proximal end 246 of support stent 240. Anchor stent 236 moves radially outwardly with respect to longitudinal axis 212 to contact the interior wall surface of the vessel at a proximal anchor location. Anchor stent 236 is then sealingly anchored to the interior wall surface. In one embodiment, anchor stent 236 is positioned and anchored distal to the right carotid artery to prevent or limit obstruction of blood flow through the carotid artery. Locking ring 234 and anchor stent 236 are anchored to the interior wall surface of the vessel using a suitable procedure known to those skilled in the art and guided by the teachings herein provided that facilitates forming a seal between the outer surface of locking ring 234, anchor stent 236 and the interior wall surface such that blood flows through passage 250 formed in stent graft 210 in the deployed configuration without allowing blood flow between the outer surface of graft 220 and the interior wall surface. Upon deployment of stent graft 210 with respect to the lesion site, delivery device 270 is withdrawn from the lesion site through the femoral artery. In another embodiment, outer sheath 280 is partially deployed to a position retaining locking ring 234. Outer sheath 280 and inner sheath 276 are withdrawn substantially simultaneously to deploy locking ring 234 and anchor stent 236.

### Capture Mechanism

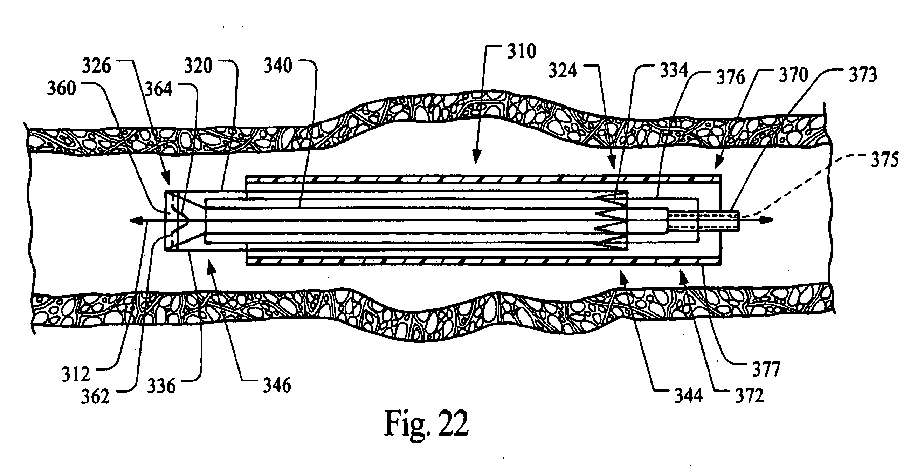

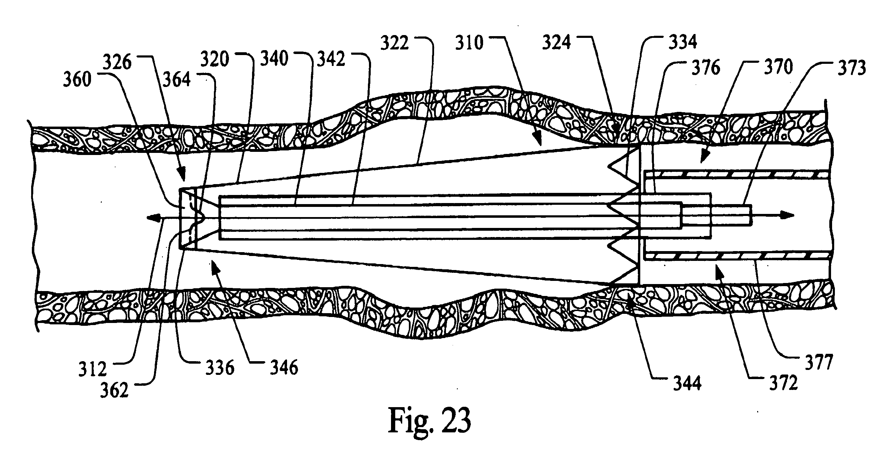

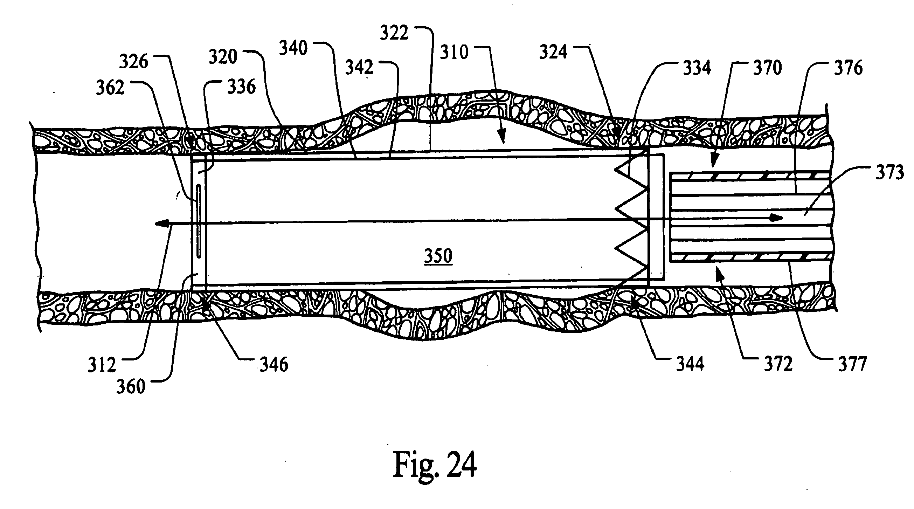

As shown in Figures 22-24, stent graft 310 includes a capture mechanism 360 operatively coupled to graft 320 and/or support stent 340. In one embodiment, capture mechanism 360 is coupled to graft proximal end 326 and/or support stent proximal end 346. Capture mechanism 360 is initially configured to retain graft proximal end 326 in the insertion configuration. As described in greater detail below, capture mechanism 360 is actuatable to release graft proximal end 326 for facilitating radial expansion of graft 320 and/or support stent 340 as the proximal end of stent graft 310 is deployed to the deployed configuration.

In one embodiment, capture mechanism 360 includes an integrated suture 362 (as shown in Figures 22-24) forming a plurality of suture loops 364 coupled to proximal end 326. In one particular embodiment, suture 362 includes a plurality of suture loops 364 sewn into or otherwise coupled to anchor stent 336. Suture 362 is movable with respect to proximal end 326 for facilitating retaining proximal end 326 in the delivery configuration and allowing proximal end 326 to move toward the deployed configuration. In this embodiment, a length of each suture loop 364 may be made shorter or longer to decrease or increase, respectively, a cross-sectional area of the proximal end of stent graft 310. Further, each suture loop 364 is initially operatively coupled to an inner sheath of a delivery device, as described in greater detail below. More specifically, each suture loop 364 is coupled to a corresponding capture wire coupled to the inner sheath.

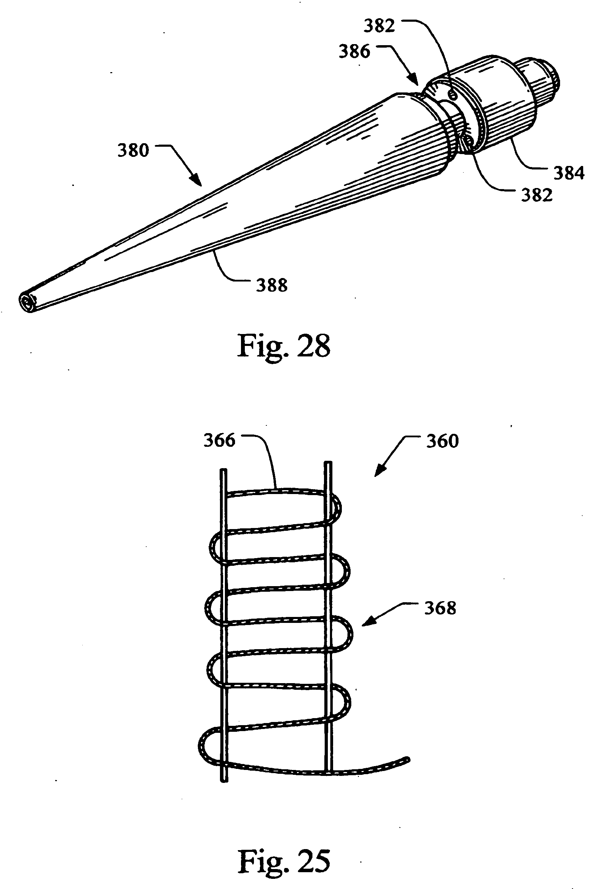

In another embodiment, capture mechanism 360 includes a string 366 (as shown in Figure 25), wrapped about an outer surface of stent graft 310. String 366 may include, for example, suture ribbons, filaments, yarns, threads, wires, strands, as well as any suitable alternative. String 366 includes a plurality of locking knots 368 configured to initially retain graft proximal end 326 in the insertion configuration. In a particular embodiment, string 366 is initially operatively coupled to the inner sheath, such as by being releasably coupled to the capture wires, and configured to release graft proximal end 326 for facilitating radial expansion of graft proximal end 326 toward the deployed configuration. In this other embodiment, string 366 is initially operatively coupled to the inner sheath of a delivery device and releasable from the inner sheath to release the graft proximal end.

Referring further to Figures 25-28, an apparatus 370 for delivering stent graft 310 to and deploying stent graft 310 at a lesion site during an endovascular procedure is provided. Although apparatus 370 is described herein as an apparatus for delivering a stent graft to a lesion site during an endovascular procedure, apparatus 370 or components thereof may be suitable for use with other delivery procedures known to those skilled in the art and guided by the teachings herein provided.

Apparatus 370 includes stent graft 310 (as shown in Figures 22-24) and a delivery device 372 defining a longitudinal axis 373. Delivery device 372 is configured to deliver stent graft 310 to the lesion site within the blood vessel and deploy stent graft 310 at the lesion site. In one embodiment, delivery device 372 includes a wire lumen 374, extending generally along longitudinal axis 373 and defining a passage 375 (shown in Figure 22) configured to receive a guide wire (not shown) and advance delivery device 372, as well as stent graft 310, to the lesion site. An inner sheath 376 is positioned about wire lumen 374 to contact at least a portion of an outer surface of wire lumen 374. Inner sheath 376 is movable in a proximal direction and a distal direction with respect to wire lumen 374 and longitudinal axis 373. An outer sheath 377 is positioned about inner sheath 376 to contact at least a portion of inner sheath 376. Outer sheath 377 is independently movable in the proximal direction and the distal direction with respect to wire lumen 374 and inner sheath 376 along longitudinal axis 373.

In one embodiment, outer sheath 377 covers at least a portion of the length of graft 320 during delivery of stent graft 310 to the lesion site. Further, inner sheath 376 is positioned within outer sheath 377 and covers at least a portion of the length of support stent 340 during delivery of stent graft 310 to the lesion site. At the lesion site, outer sheath 377 is movable in a distal direction with respect to longitudinal axis 373 to at least partially expose and deploy graft 320. In this embodiment, with graft 320 at least partially deployed, distal ring 334 is anchored to the interior wall surface of the vessel. Inner sheath 376 is independently movable in the distal direction with respect to longitudinal axis 373 to at least partially expose and deploy support stent 340. In a particular embodiment, with support stent 340 at least partially deployed, anchor stent 336 is anchored to the interior wall surface. Support stent 340, including freely movable distal end 344, expands in an outward radial direction with respect to longitudinal axis 373 to contact an inner surface of graft 320 and form or define passage 375.

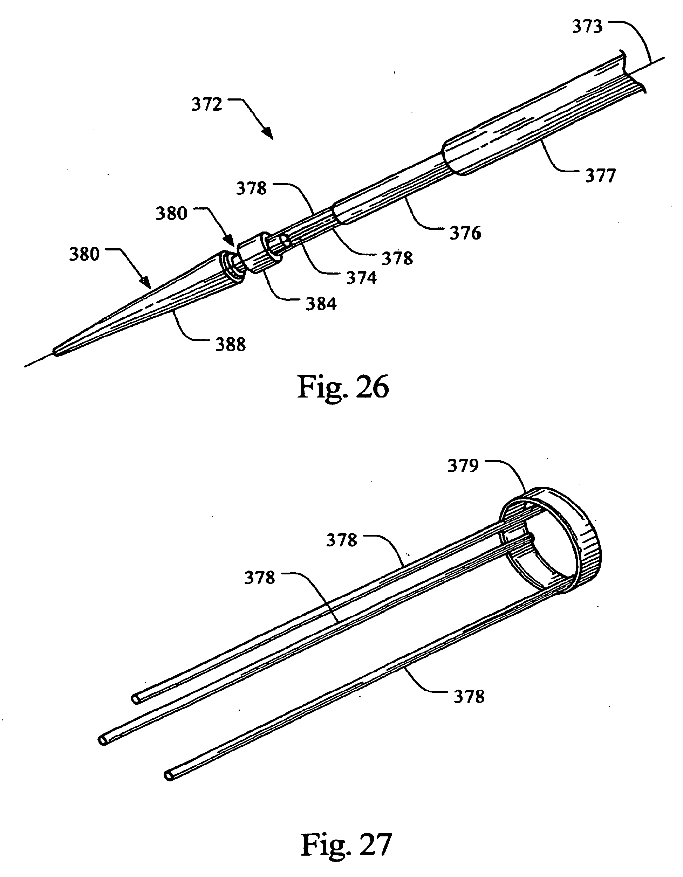

In one embodiment, capture mechanism 360 is initially configured to retain graft proximal end 326 in the insertion configuration. Capture mechanism 360 is actuatable to release graft proximal end 326 for facilitating radial expansion of graft 320. As shown in Figure 26, a plurality of capture wires 378 are coupled to inner sheath 376. Each capture wire 378 is coupled at a distal end to inner sheath 376 and releasably coupled at an opposing proximal end to capture mechanism 360. In a particular embodiment, each capture wire 378 is coupled at a distal end to a ring 379, as shown in Figure 27. Ring 379 is integrated with or coupled to inner sheath 376 using a suitable coupler, such as a suture and/or another suitable coupler known to those skilled in the art and guided by the teachings herein provided. Further, in this particular embodiment, each capture wire 378 is releasably coupled at the proximal end to a corresponding suture loop 364 formed by integrated suture 362 of capture mechanism 360.

In another example, wherein capture mechanism 360 includes a string 366 wrapped about an outer surface of stent graft 310, string 366 is operatively coupled to each capture wire 378. More specifically, string 366 includes a plurality of locking knots 368 initially configured to retain graft proximal end 326 in an insertion configuration, as shown in Figure 25. String 366 is configured such that locking knots 368 decouple from each capture wire 378 for facilitating releasing graft proximal end 326 to allow proximal end 326 to move radially outward toward the deployed configuration.

Referring further to Figures 26 and 28, delivery device 372 includes a nose cone 380 positioned proximal to outer sheath 377 and inner sheath 376. Nose cone 380 includes a plurality of capture wire channels 382 defined within a shaft portion 384 of nose cone 380. In one embodiment, each capture wire channel 382 is positioned radially about and extends parallel to longitudinal axis 373 of deliver device 372. In a particular embodiment, each capture wire channel 382 is radially positioned at about 120° with respect to adjacent capture wire channels 382. It is apparent to those skilled in the art and guided by the teachings herein provided that any suitable number of capture wire channels 382 may be defined within shaft portion 384 such that a suitable number of capture wires 378 may be fed through a corresponding capture wire channel 382 and releasably coupled to a corresponding suture loop 364 formed in capture mechanism 360.

In one example integrated suture 362 forms three (3) suture loops 364. In other examples, integrated suture 362 forms at least six (6) suture loops 364 to twenty-four (24) suture loops 364. It is apparent to those skilled in the art and guided by the teachings herein provided that any suitable number of suture loops 364 (and corresponding capture wires 378) may be provided to retain the proximal end of stent graft 310 in the insertion configuration or a partially deployed configuration, as desired, without undesirably increasing the loading profile. A plurality of suture loops 364 facilitates uniform capturing of graft proximal end 326 and/or uniform releasing of graft proximal end 326 at the desired proximal anchor location for facilitating proper placement of stent graft 310 with respect to the lesion site.

As shown in Figures 26 and 28, a suture capture groove 386 is defined within nose cone 380 between shaft portion 384 and a lead portion 388 of nose cone 380. Suture capture groove 386 extends radially about nose cone 380 and substantially perpendicular to longitudinal axis 373. Suture capture groove 386 intersects each capture wire channel 382 to provide communication between each capture wire channel 382 and suture capture groove 386. In one embodiment, each capture wire 378 extends through corresponding capture wire channel 382, from a distal end to a proximal end of capture wire channel 382, and into suture capture groove 386. Each suture loop 364 formed by capture mechanism 360 is releasably coupled within suture capture groove 386 to a corresponding capture wire 378.

In this example, outer sheath 377 is movable in a distal direction along longitudinal axis 373 to deploy graft distal end 324. With graft distal end 324 deployed, distal ring 334 is anchored to the interior wall surface of the vessel. Inner sheath 376 is then movable in a distal direction along longitudinal axis 373 to deploy graft proximal end 326 and/or anchor stent 336. As inner sheath 376 is moved in the distal direction, each capture wire 378 is decoupled from corresponding suture loop 364. As each capture wire 378 is decoupled from suture loop 364, proximal end 326 of graft 320 moves radially outward toward the deployment configuration. By retaining proximal end 326 in the insertion configuration or a partially deployed configuration as graft distal end 324 is deployed, proximal end 326 can be accurately positioned before stent graft 310 is completely deployed and anchored to the interior wall surface of the vessel.

Referring further to Figure 25, in another example, locking knots 368 of capture ribbon 366 are initially releasably coupled to each capture wire 378. As inner sheath 376 is moved in the distal direction, each capture wire 378 is decoupled from string 366. As each capture wire 378 is decoupled from string 366, proximal end 326 of graft 320 moves radially outward toward the deployment configuration. By retaining proximal end 326 in the delivery configuration or a partially deployed configuration as graft distal end 324 is deployed, proximal end 326 can be accurately positioned before stent graft 310 is completely deployed and anchored to the interior wall surface of the vessel.

In one embodiment, the method includes initially retaining the proximal end of stent graft 310 in the insertion configuration as outer sheath 377 is withdrawn to deploy the distal end of stent graft 310 including distal ring 334. Distal ring 334 is anchored to the vessel wall. Inner sheath 376 of delivery device 372 is then withdrawn to deploy the proximal end of stent graft 310 including anchor stent 336, and anchor stent 336 is anchored to the vessel wall at the proximal anchor location.

In this example capture mechanism 360 is operatively coupled to the proximal end of stent graft 310 and to a plurality of capture wires 378, which are independently coupled to inner sheath 376. Capture mechanism 360 initially retains graft proximal end 326 in the delivery configuration. With the proximal end of stent graft 310 retained in the delivery configuration, the proximal end of stent graft 310 is positioned with respect to the lesion site at a desirable proximal anchor location. Capture mechanism 360 is actuated to release graft proximal end 326 for facilitating radially expanding the proximal end of the stent graft. Inner sheath 376 is withdrawn to deploy the proximal end of stent graft 310 such that capture wires 378 coupled to the proximal end of inner sheath 376 are released from capture mechanism 360.

In a particular example, capture mechanism 360 includes integrated suture 362 coupled to graft proximal end 326. Integrated suture 362 is sewn into graft proximal end 326 and/or anchor stent 336 to form suture loops 364. Each capture wire 378 is releasably coupled to a corresponding suture loop 364. Inner sheath 376 is moved in a distal direction to decouple each capture wire 378 from a corresponding suture loop 364 formed on capture mechanism 360 to actuate capture mechanism 360 and release graft proximal end 326.

In one example, nose cone 380 of delivery device 372 defines a suitable number of capture wire channels 382. Each capture wire channel 382 is positioned radially about and extends parallel to longitudinal axis 373 of deliver device 372. Suture capture groove 386 is defined within nose cone 380. Suture capture groove 386 extends radially about nose cone 380 and substantially perpendicular to longitudinal axis 373. Suture capture groove 386 intersects each capture wire channel 382 to provide communication between each capture wire channel 382 and suture capture groove 386. Each capture wire 378 is initially fed through a corresponding capture wire channel 382 and into suture capture groove 386, wherein each capture wire 378 is coupled within suture capture groove 386, to a corresponding suture loop 364 formed in capture mechanism 360.

### Delivery Device Actuator

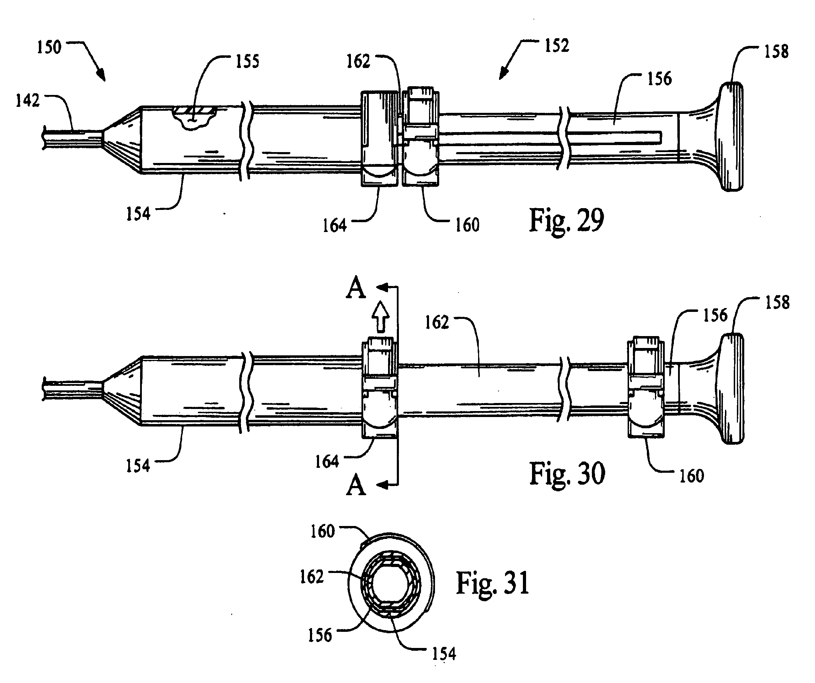

Referring to Figures 29-31, in one example delivery system 130 includes an actuator 150. Actuator 150 has a handle 152 operatively coupled to inner sheath 134 and outer sheath 142. Handle 152 includes a housing 154 defining a chamber 155. Handle 152 further includes an outer sheath retraction tube 156 that is slidably positioned within chamber 155 and coupled at a proximal end to outer sheath 142. A retraction element 158 is coupled to a distal end of outer sheath retraction tube 1 56 for facilitating moving outer sheath retraction tube 156 with respect to housing 154. As shown in Figure 29, outer sheath retraction tube 156 is slidably movable with respect to housing 154 in the distal direction to retract outer sheath 142 and deploy graft 114. In this embodiment, as outer sheath 142 is retracted, graft 114 expands in a radial direction to contact an interior surface of the vessel wall. In another embodiment; actuator 150 is activated to deploy graft 114 from the delivery configuration to a deployed configuration at the lesion site.

As shown in Figure 29, a first locking element 160 is positioned about outer sheath retraction tube 156 and configured to lock outer sheath retraction tube 1 56 in a locked position to prevent or limit movement of outer sheath retraction tube 156 within housing 154 as stent graft 110 is delivered and/or positioned at the lesion site. With stent graft 110 properly positioned at the lesion site, first lucking element 160 is unlocked and outer sheath retraction tube 156 is drawn in a distal direction with respect to housing 154 to retract outer sheath 142.

Handle 152 also includes an inner sheath retraction tube 162 that is slidably positioned about outer sheath retraction tube 156, as shown in Figure 30. Inner sheath retraction tube 162 is coupled at a proximal end to inner sheath 134 and first locking element 160 is coupled to an opposing distal end of inner sheath retraction tube 162. As shown in Figure 30, inner sheath retraction tube 162 is slidably movable with respect to outer sheath retraction tube 156 in a distal direction to retract inner sheath 134 and deploy support stent 126. In one embodiment, a second locking element 164 is coupled to housing 154 and configured to lock inner sheath retraction tube 162 in a locked position to prevent or limit movement of inner sheath retraction tube 162 with respect to outer sheath retraction tube 1 56 as stent graft 110 is delivered and/or positioned at the lesion site. With stent graft 110 properly positioned at the lesion site, second locking element 164 is unlocked and inner sheath retraction tube 162 is drawn in a distal direction with respect to outer sheath retraction tube 156 to retract inner sheath 134.

Referring to Figure 31, in one example outer sheath retraction tube 156 and/or inner sheath retraction tube 162 has a non-circular cross-sectional area configured to prevent or limit undesirable rotational movement of outer sheath retraction tube 1 56 and/or inner sheath retraction tube 162.

In this embodiment, with stent graft 110 properly positioned at the lesion site, first locking element 160 is unlocked. Retraction element 158, and outer sheath retraction tube 156 coupled thereto, is slid in a distal direction to retract outer sheath 142 to deploy graft 114 at a lesion site. Second locking element 164 is then unlocked and first locking element 160, and inner sheath retraction tube 162 coupled thereto, is slid in the distal direction to retract inner sheath 134 positioned about support stent 126. As inner sheath 134 is retracted, support stent 126 expands from the compressed delivery configuration to an expanded deployed configuration. In the deployed configuration, an outer surface of support stent 126 contacts an inner surface of graft 114. In a particular embodiment, first locking element 160 and second locking element 164 are unlocked and retraction element 158 and first locking element 160 are slid in the distal direction substantially simultaneously to deploy graft 114 and support stent 126 at the lesion site.

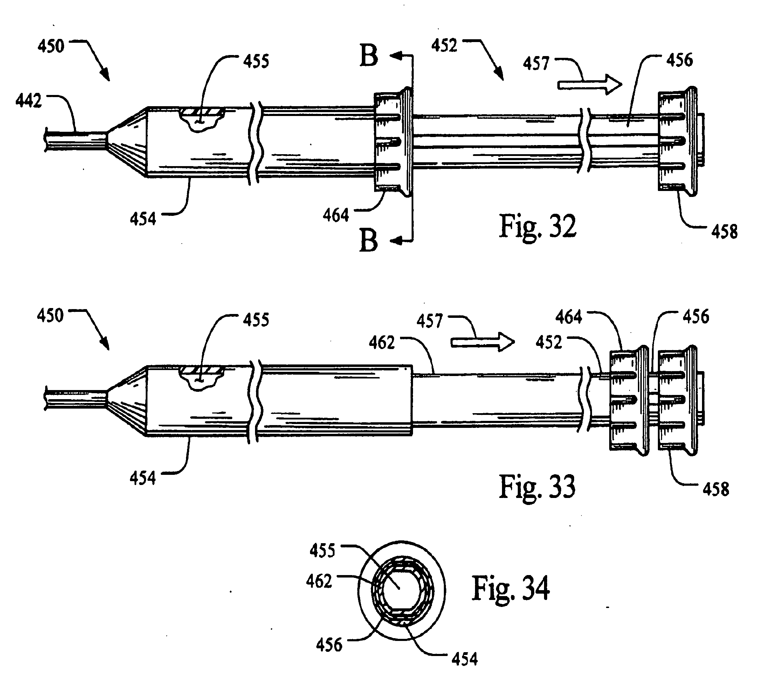

Referring to Figures 32-41, in one example an actuator 450 includes a handle 452 operatively coupled to inner sheath 434 and outer sheath 442. Handle 452 includes a housing 454 defining a chamber 455. Handle 452 also includes an outer sheath retraction tube 456 that is slidably positioned within housing 454 and coupled to a distal end of outer sheath 442. A first retraction element 458 is coupled to a distal end of outer sheath retraction tube 456 for facilitating moving outer sheath retraction tube 456 with respect to housing 454. As shown in Figure 32, outer sheath retraction tube 456 is slidably movable with respect to housing 454 in a distal direction as shown by directional arrow 457 to retract outer sheath 442 and deploy graft 414. In one embodiment, first retraction element 458 is configured to lock outer sheath retraction tube 456 in a locked position to prevent or limit movement of outer sheath retraction tube 456 within housing 454.

As shown in Figure 32, outer sheath retraction tube 456 is slidably movable with respect to housing 454 in the distal direction to retract outer sheath 442 and deploy graft 414. In this embodiment, as outer sheath 442 is retracted, graft 414 expands in a radial direction to contact an interior surface of the vessel wall. In another embodiment, actuator 450 is activated to deploy graft 414 from the insertion configuration to a deployed configuration at the lesion site.

First retraction element 458 is positioned about outer sheath retraction tube 456 and configured to lock outer sheath retraction tube 456 in a locked position to prevent or limit movement of outer sheath retraction tube 456 within housing 454 as stent graft 410 is delivered and/or positioned at the lesion site. With stent graft 410 properly positioned at the lesion site, first retraction element 458 is rotated with respect to outer sheath retraction tube 456 to unlock outer sheath retraction tube 456. Outer sheath retraction tube 456 is then drawn in the distal direction with respect to housing 454 to retract outer sheath 442.

Handle 452 also includes an inner sheath retraction tube 462 that is slidably positioned about outer sheath retraction tube 456, as shown in Figure 33. Inner sheath retraction tube 462 is coupled at a proximal end to inner sheath 434 and a second retraction element 464 is coupled to an opposing distal end of inner sheath retraction tube 462. As shown in Figure 33, inner sheath retraction tube 462 is slidably movable with respect to outer sheath retraction tube 456 in a distal direction to retract inner sheath 434 and deploy support stent 426. In one embodiment, second retraction element 464 is configured to lock inner sheath retraction tube 462 in a locked position to prevent or limit movement of inner sheath retraction tube 462 with respect to outer sheath retraction tube 456 as stent graft 410 is delivered and/or positioned at the lesion site. With stent graft 410 properly positioned at the lesion site, second retraction element 464 is rotated to an unlocked position and inner sheath retraction tube 462 is drawn in the distal direction with respect to outer sheath retraction tube 456 to retract outer sheath 442.

Referring to Figure 34, in one example outer sheath retraction tube 456 and/or inner sheath retraction tube 462 has a non-circular cross-sectional area configured to prevent or limit undesirable rotational movement of outer sheath retraction tube 456 and/or inner sheath retraction tube 462.

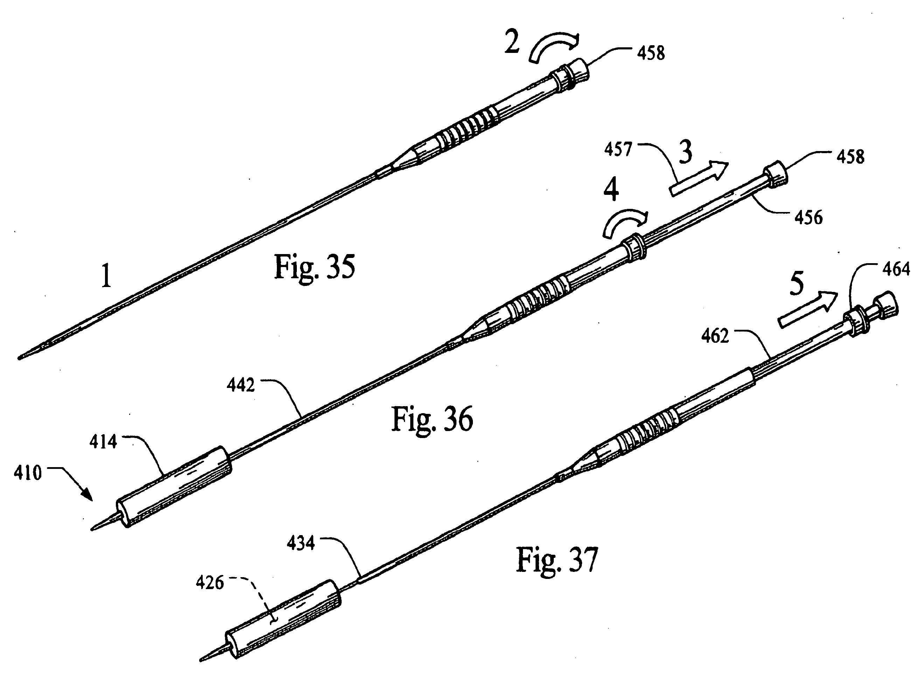

Referring further to Figures 35-41, with stent graft 410 properly positioned at the lesion site, first retraction element 458 is rotated to an unlocked position, as shown in Figure 35. Outer sheath retraction tube 456 is slid with respect to housing 454 in distal direction 457 to retract outer sheath 442 positioned about graft 414 in the delivery configuration to deploy graft 414 at a lesion site, as shown in Figure 36. Second retraction element 464 is then rotated to an unlocked position and inner sheath retraction tube 462 is slid in the distal direction to retract inner sheath 434 positioned about support stent 426 in a compressed delivery configuration, as shown in Figure 37. As inner sheath 434 is retracted, support stent 426 expands from the compressed delivery configuration to an expanded deployed configuration. In the deployed configuration, an outer surface of support stent 426 contacts an inner surface of graft 414. In a particular embodiment, first retraction element 458 and second retraction element 464 are unlocked and outer sheath retraction tube 456 and inner sheath retraction tube 462 are slid in the distal direction substantially simultaneously to deploy graft 414 and support stent 426 at the lesion site.

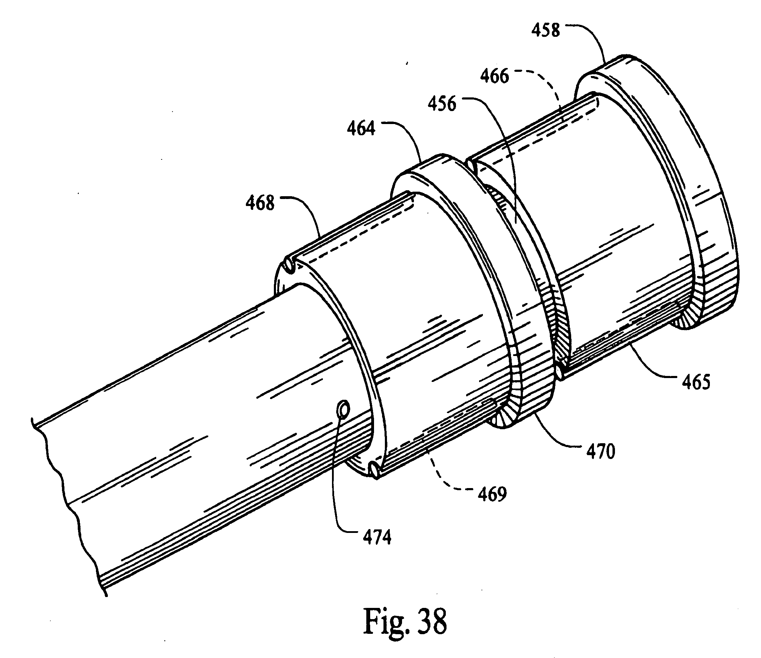

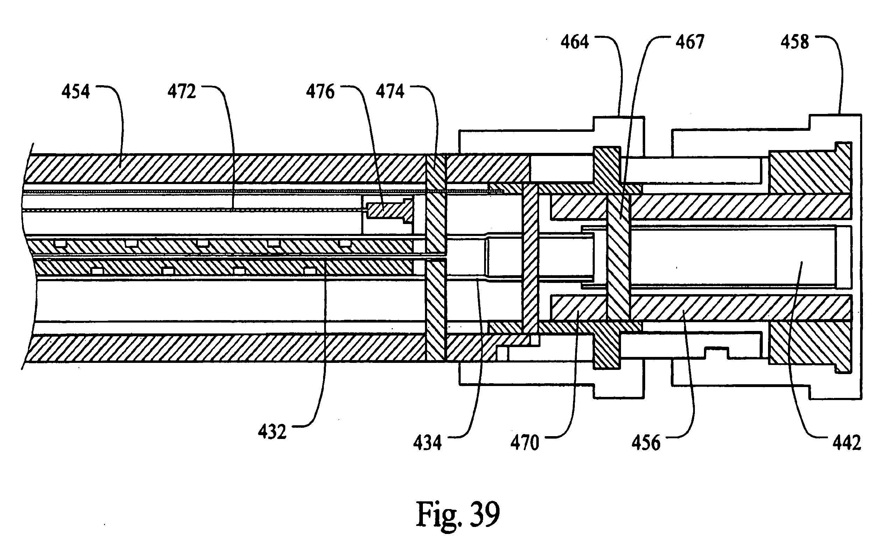

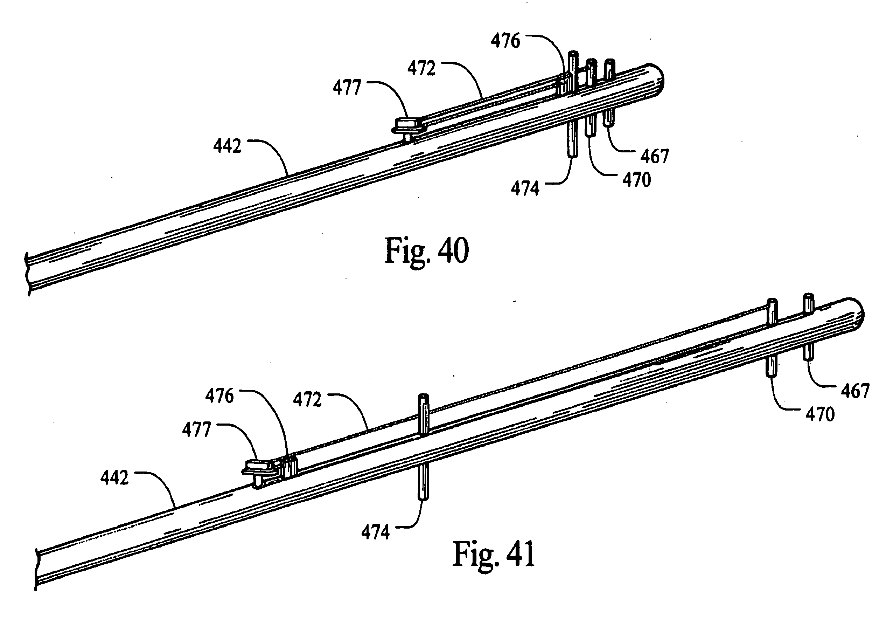

As shown in Figure 38, in one example first retraction element 458 forms a projection, such as pin 465, that is movably positioned within a slot 466 defined within outer sheath retraction tube 456. First retraction element 458 is rotated such that pin 465 travels along slot 466 to move first retraction element 458 between a locked position and an unlocked position, as shown in Figure 38. With first retraction element 458 in the unlocked position, outer sheath retraction tube 456 is drawn in the distal direction to retract outer sheath 442. In this embodiment, pin 465 interferes with a post 467 coupled to outer sheath 442, as shown in Figure 39, to retract outer sheath 442 as outer sheath retraction tube 456 is drawn or pulled in the distal direction. Similarly, second retraction element 464 forms a projection, such as pin 468 shown in Figure 38, which is movably positioned within a slot 469 defined within inner sheath retraction tube 462. Second retraction element 464 is rotated such that pin 468 travels along slot 469 to move second retraction element 464 between a locked position, as shown in Figure 38, and an unlocked position. With second retraction element 464 in the unlocked position, inner sheath retraction tube 462 is drawn or pulled in the distal direction to retract inner sheath 434. Referring to Figures 38-41, pin 468 interferes with a post 470 coupled to inner sheath 434, as shown in Figure 39, to retract inner sheath 434 as inner sheath retraction tube 462 is drawn. Further, as shown in Figures 39-41, a suture 472 couples post 470 through an anchor pin 474 to support member 436. In one embodiment, support member 436 includes a projection, such as a block 476, to which suture 472 is coupled. Referring further to Figures 40 and 41, suture 472 is wrapped about a spindle 477 operatively coupled to housing 454. As inner sheath 434 is retracted in the distal direction, support member 436 coupled to inner sheath 434 by suture 472 is moved in an opposing proximal direction to retain support stent 426 properly positioned at the lesion site.

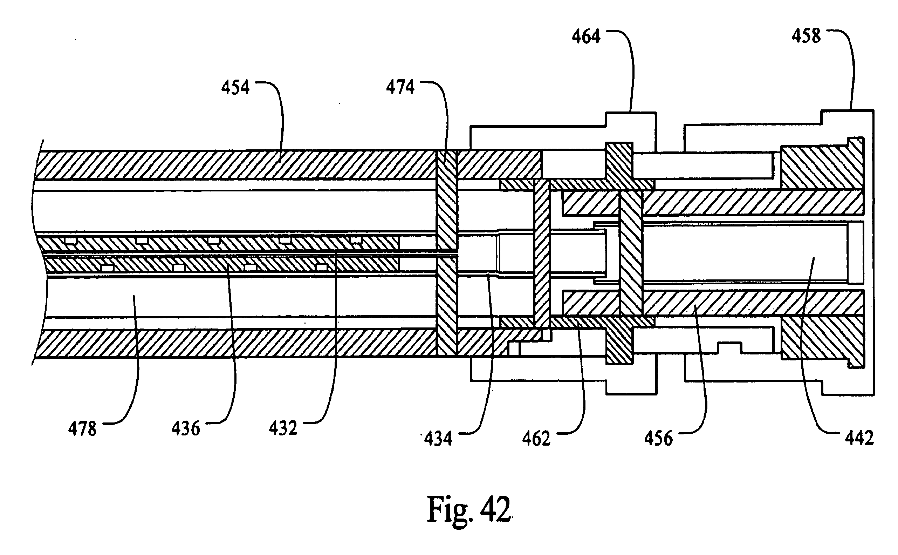

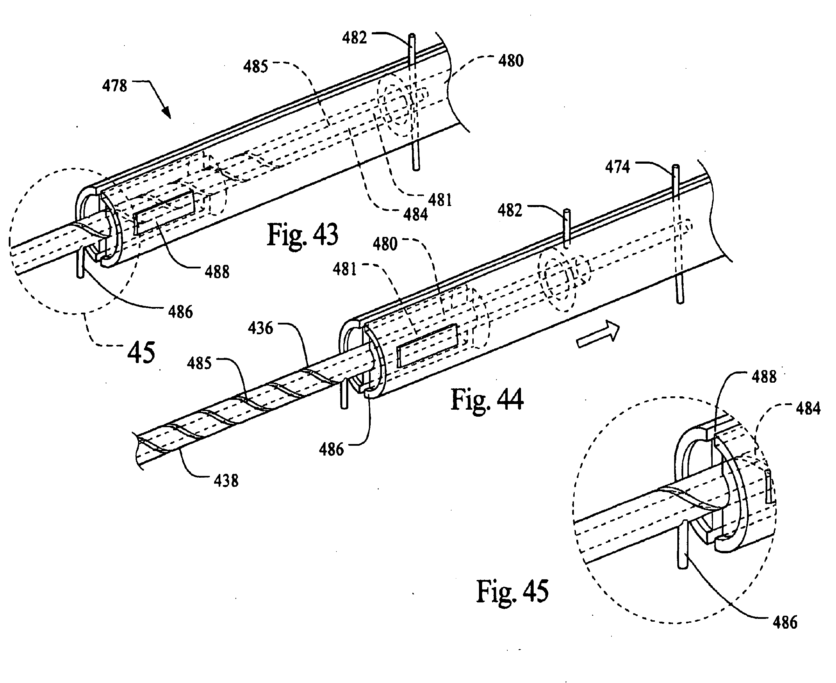

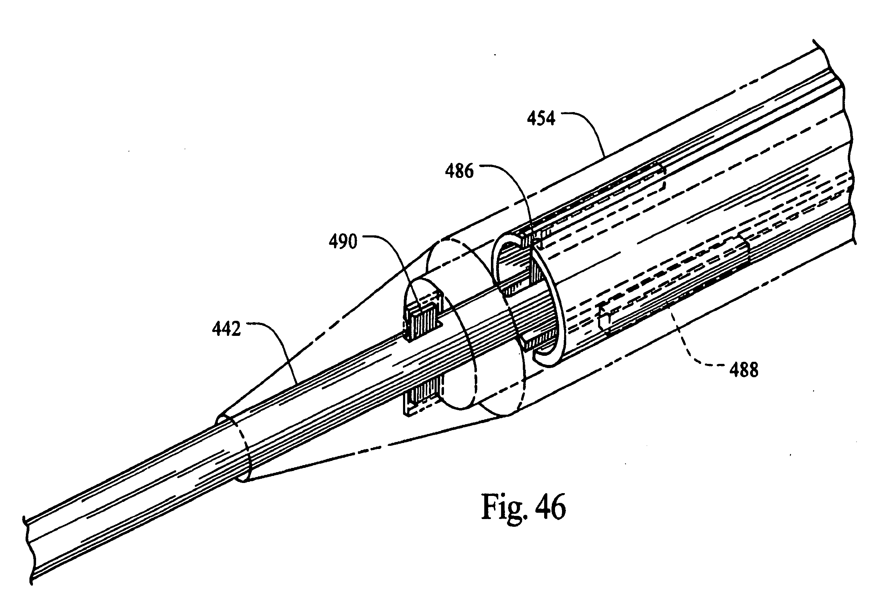

Referring to Figures 42-46, in another example inner sheath retraction tube 462 is retracted to activate a cam system 478 that advances support member 436 as inner sheath retraction tube 462 is retracted. In this embodiment, support member 436 includes a first or distal cam portion 480 forming a helical track 481 that cooperates with an advancement pin 482 that is fixedly coupled to housing 454 as support member 436 is advanced in the proximal direction. The cooperation of first cam portion 480 with advancement pin 482 facilitates advancement of support member 436 in the proximal direction. Support member 436 also includes a second or proximal cam portion 484 forming a helical track 485 that cooperates with a rotation pin 486 that is fixedly coupled to housing 454 as support member 436 advances in the proximal direction. The cooperation of second cam portion 484 with rotation pin 486 facilitates rotation of support member 436. In a particular embodiment, at least one rail 488 is coupled to or formed in housing 454 for facilitating resisting torque stresses and/or rational forces produced by inner sheath retraction tube 462 as inner sheath retraction tube 462 is retracted to activate cam system 478. As shown in Figure 44, proximal end 438 of support member 436 is coupled to second cam portion 484 such that proximal end 438 does not rotate as support member 436 is advanced. Further, in a particular embodiment, a blade 490 is mounted with respect to a proximal end of housing 454, as shown in Figure 46, to cut and/or split outer sheath 442 for facilitating clearing cam system 478 (not shown in Figure 46) without interfering with cam system 478 as outer sheath 442 is retracted.

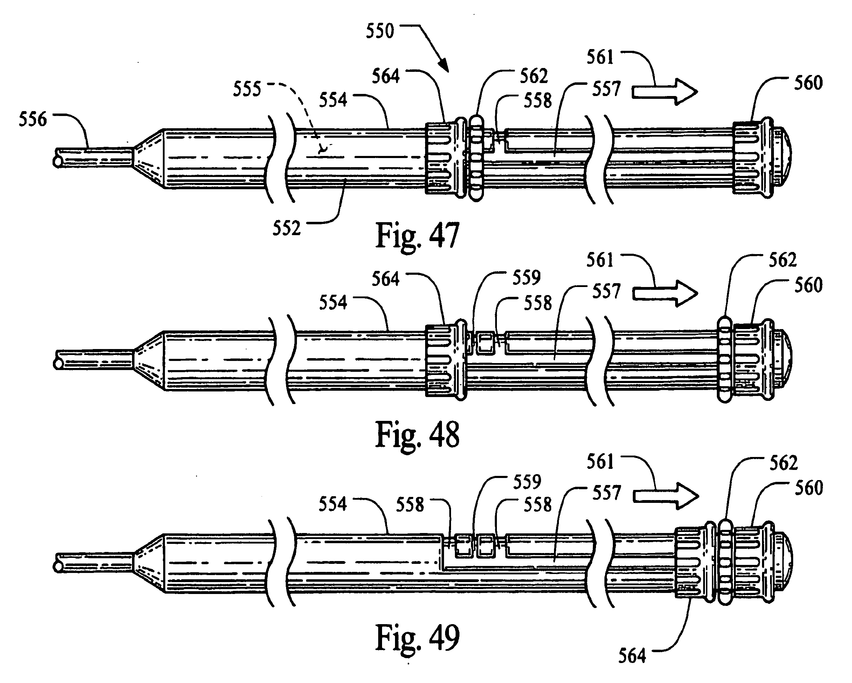

Referring to Figures 47-49, in one example an actuator 550 includes a handle 552 operatively coupled to inner sheath 534 and/or outer sheath 542. Handle 552 includes a housing 554 defining a chamber 555. Housing 554 defines an axis 556 and a track 557 along at least a portion of axis 556, as shown in Figure 47. In one embodiment, track 557 defines or includes at least one locking groove 558 and/or at least one intermediate groove 559.

A first retraction element 560 is positioned about housing 554 and operatively coupled to outer sheath 542. First retraction element 560 is movable, such as by rotating first retraction element 560, between a locked position and an unlocked position. In the locked position, first retraction element 560 is positioned within a first locking groove 558 to prevent or limit movement of outer sheath 542 as stent graft 510 is delivered and/or positioned at the lesion site. With stent graft 510 properly positioned at the lesion site, first retraction element 560 is unlocked and slidably movable within track 557 in a distal direction, as shown by directional arrow 561, to retract outer sheath 542, as shown in Figure 47.

A second retraction element 562 is positioned about housing 554 and operatively coupled to graft 514. Second retraction element 562 is movable, such as by rotating second retraction element 562, between a locked position and an unlocked position. In the locked position, second retraction element 562 is positioned within intermediate locking groove 559 to prevent or limit movement of graft 514 as stent graft 510 is delivered and/or positioned at the lesion site. As shown in Figure 48, with stent graft 510 properly positioned at the lesion site, second retraction element 562 is unlocked and slidably movable with respect to housing 554 in the distal direction to deploy graft 514, as described in greater detail below.

A third retraction element 564 is positioned about housing 554 and operatively coupled to inner sheath 534. Third retraction element 564 is movable, such as by rotating third retraction element 564, between a locked position and an unlocked position. In the locked position, third retraction element 564 is positioned within a locking groove 558 to prevent or limit movement of inner sheath 534 as stent graft 510 is delivered and/or positioned at the lesion site. With stent graft 510 properly positioned at the lesion site, third retraction element 564 is unlocked and slidably movable with respect to housing 554 in the distal direction, as shown in Figure 49, to retract inner sheath 534 and deploy support scent 526.

In this example, with stent graft 510 properly positioned at the lesion site, first retraction element 560 is rotated within corresponding locking groove 558 to unlock first retraction element 560. As shown in Figure 47, first retraction element 560 is drawn or pulled with respect to housing 554 in the distal direction to retract outer sheath 542 positioned about graft 514 in the delivery configuration. Second retraction element 562 is rotated within intermediate groove 559 and is drawn or pulled with respect to housing 554 in the distal direction, as shown in Figure 48, to deploy graft 514 at the lesion site. Third retraction element 564 is rotated within corresponding locking groove 558 to unlock third retraction element 564 positioned about housing 554 and operatively coupled to inner sheath 534. Third retraction element 564 is drawn or pulled with respect to housing 554 in the distal direction to retract inner sheath 534 positioned about support stent 526 in a compressed delivery configuration. With inner sheath 534 in the retracted position, support stent 526 is expandable from the compressed delivery configuration to an expanded configuration, wherein an outer surface of support stent 526 contacts an inner surface of graft 514. In a particular embodiment, first retraction element 560, second retraction element 562 and third retraction element 564 are unlocked and slid in the distal direction substantially simultaneously to deploy graft 514 and support stent 526 at the lesion site.

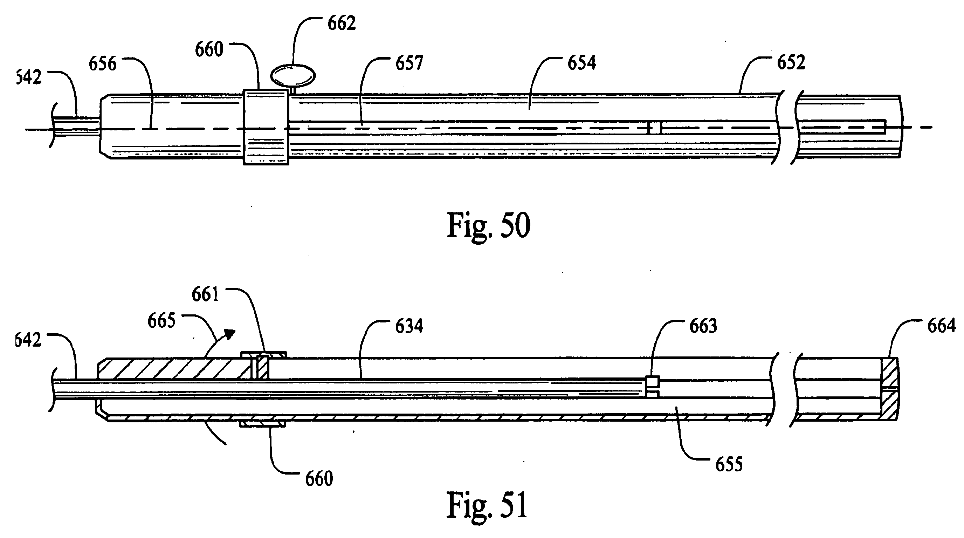

Referring to Figures 50-53, in one example an actuator 650 includes a handle 652 operatively coupled to inner sheath 634 and/or outer sheath 642. Handle 652 includes a housing 654 defining a chamber 655 within which inner sheath 634 and/or outer sheath 642 is positionable in the retracted position. Housing 654 further defines an axis 656 and a track 657 along at least a portion of axis 656, as shown in Figure 50. In one embodiment, track 657 extends through housing 654 and is in communication with chamber 655.

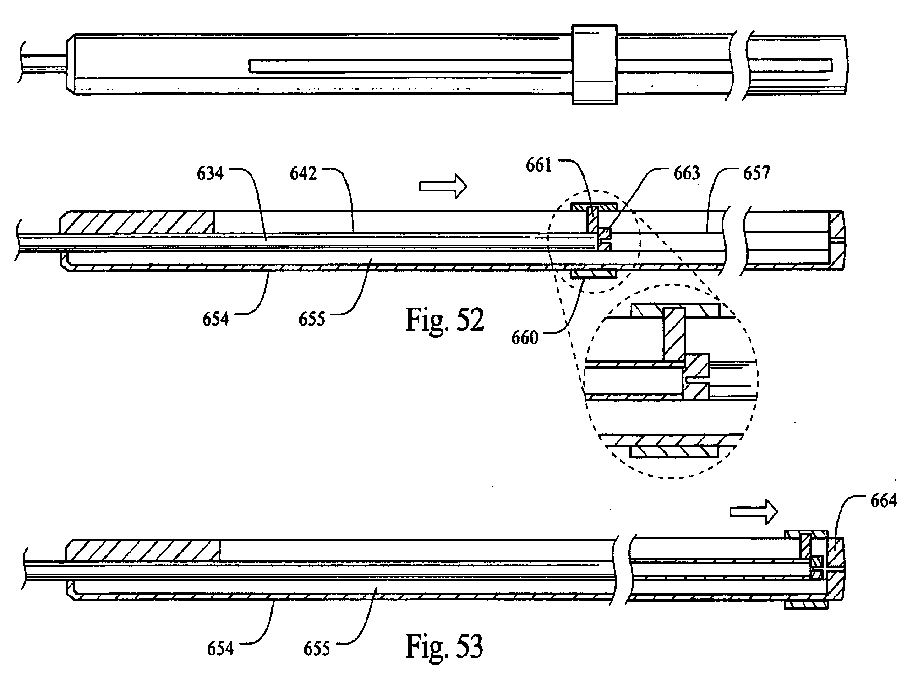

A retraction element 660 is positioned about housing 654 and operatively coupled to outer sheath 642. In one example, a connector 661 couples outer sheath 642 to retraction element 660. As shown in Figure 51, connector 661 is coupled to outer sheath 642 and extends through track 657 to couple to retraction element 660. Retraction element 660 is retained in an initial position along axis 656 by a locking element 662 that extends into an aperture (not shown) defined by housing 654. Locking element 662 is configured to initially prevent or limit movement of retraction element 660 and/or outer sheath 642 along axis 656. In one embodiment, locking element 662 is removable from within housing 654 to allow retraction element 660 to move along a length of housing 654. In another embodiment, locking element 662 is breakable at a coupling point or area with housing 654 to allow retraction element 660 to move along the length of housing 654. Retraction element 660 is rotatable with respect to housing 654 between a locked position and an unlocked position. With locking element 662 removed from the aperture in housing 654 and retraction element 660 rotated to the unlocked position, retraction element 660 is slidably movable with respect to housing 654 in a distal direction between an initial position, as shown in Figure 50, and a first stop position, as shown in Figure 52, to retract outer sheath 642. As shown in Figure 52, at the first stop position connector 661 contacts a connector 663 that is coupled to inner sheath 634. Connector 663 is at least partially positioned within track 657 for facilitating preventing inner sheath 634 from undesirably rotating within chamber 655. In one embodiment, connector 663 is configured to interfere with connector 661 as retraction element 660 is moved from the first stop position to a second or final stop position, as shown in Figure 53. As retraction element 660 moves toward the final stop position, connector 663 moves along track 657 towards a back stop 664 coupled to and/or integrated with a distal end of housing 654 to retract inner sheath 634.

In one example, with stent graft 610 properly positioned at the lesion site, locking element 662 is removed from housing 654, for example by breaking locking element 662 at the housing coupling area. Retraction element 660 is rotated to an unlocked position. In one embodiment, retraction element 660 is rotated in a rotational direction as shown by directional arrow 665 in Figure 51. In another embodiment, retraction element 660 is configured to rotate in a rotational direction opposite the rotational direction shown in Figure 51. Retraction element 660 is drawn or pulled with respect to housing 654 in a distal direction between an initial position, as shown in Figure 51, and the first stop position, has shown in Figure 52, to retract outer sheath 642 and deploy graft 114 at the lesion site. Retraction element 660 is slidably movable with respect to housing 654 in the distal direction between the first stop position and the final stop position at or near back stop 664, as shown in Figure 53, to retract inner sheath 634 and deploy support stent 126 at the lesion site. With graft 114 deployed at the lesion site, support stent 126 is deployed such that at least a portion of an exterior surface of support stent 1 26 contacts at least a portion of an interior surface of graft 114.

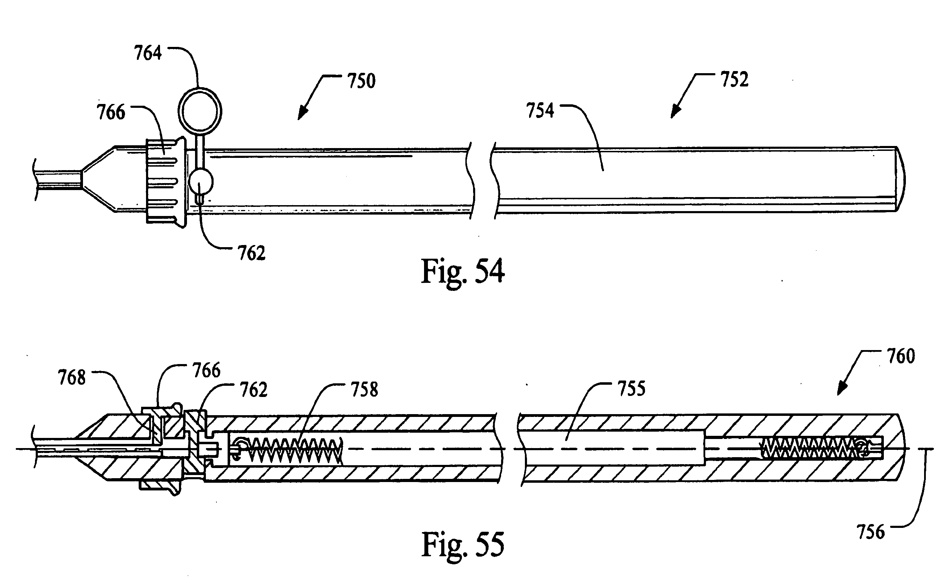

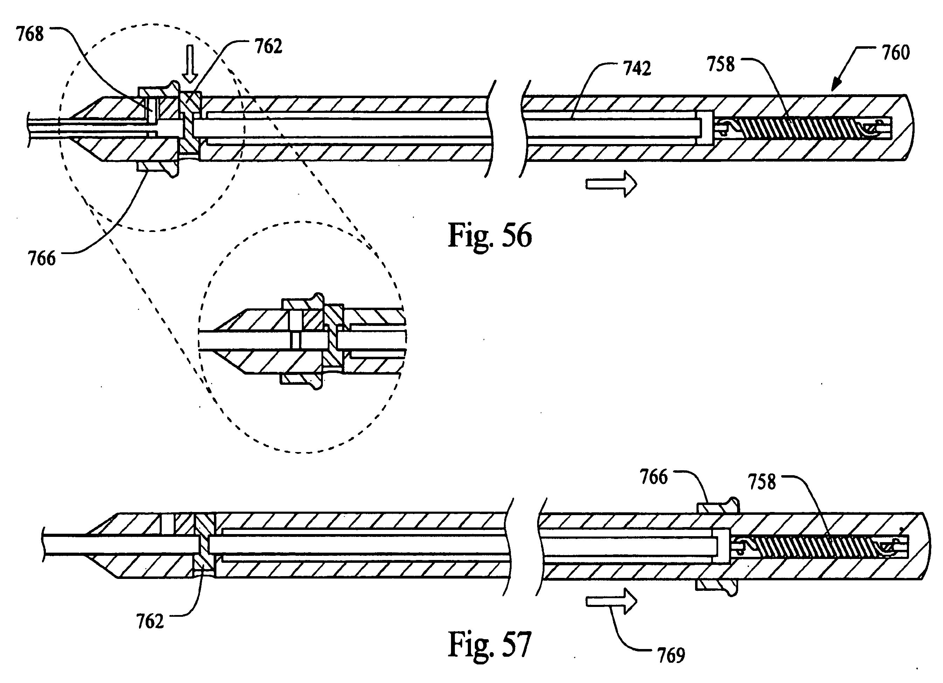

Referring to Figures 54-58, in one embodiment an actuator 750 includes a handle 752 operatively coupled to inner sheath 734 and/or outer sheath 742. Handle 752 includes a housing 754 defining a chamber 755 along at least a portion of a length of housing 754 and an axis 756. Referring further to Figures 56 and 57, at least a portion of inner sheath 734 and/or outer sheath 742 is slidably movable within chamber 755.

In one example, a biasing element 758, such as a spring, is positioned within chamber 755. Biasing element 758 is coupled at a first end to a distal end 760 of housing 754 and at a second end to outer sheath 742. In this embodiment, biasing element 758 biases outer sheath 742 towards distal end 760. A push button 762 is positioned within and/or coupled to housing 754 and configured to retain outer sheath 742 in a delivery configuration. As shown in Figure 55, push button 762 extends into chamber 755 to retain outer sheath 742 in the delivery configuration. Push button 762 is movable between a delivery position wherein push button 762 retains outer sheath 742 in the initial delivery configuration and a depressed position for facilitating retracting outer sheath 742. More specifically, push button 762 is configured to lock or interfere with outer sheath 742 to retain biasing element 758 in an extended position, as shown in Figure 55. In one embodiment, a safety pin 764 is configured to retain push button 762 in an initial position. Push button 762 defines a passage through which safety pin 764 extends to prevent push button 762 from moving inwardly with respect to housing 754.

With safety pin 764 removed, push button 762 is depressed to release outer sheath 742 and allow biasing element 758 to recoil to an inertial position. As biasing element 758 moves toward the inertial position, biasing element 758 biases outer sheath 742 towards distal end 760 to retract outer sheath 742, as shown in Figure 56, and deploy graft 714. In one embodiment, with outer sheath 742 retracted, inner sheath 734 rotates and partially retracts to allow a portion of support stent 126 to expand. A retraction element 766 is positioned about housing 754 and operatively coupled to inner sheath 734. In a particular embodiment, a connector 768 couples retraction element 766 to inner sheath 734, as shown in Figures 55 and 56. Retraction element 766 is rotatable with respect to housing 754 between a locked position and an unlocked position. In the unlocked position, retraction element 766 facilitates aligning connector 768 with a slot or track defined within housing 754. In the unlocked position, retraction element 766 is slidably movable with respect to housing 754 in a distal direction to retract inner sheath 734, as shown by directional arrow 769 in Figure 57.

In one example, a second biasing element 770, such as a spring, is positioned within chamber 755. Biasing element 770 is coupled at a first end to distal end 760 of housing 754 and at a second end to connector 768. In this embodiment, biasing element 770 biases inner sheath 734 towards distal end 760. A second push button (not shown) is positioned within and/or coupled to housing 754 and configured to retain inner sheath 734 in a delivery configuration. In a particular embodiment, the push button extends into chamber 755 to retain inner sheath 734 in the delivery configuration. The push button is movable between a delivery position, wherein the push button retains inner sheath 734 in the initial delivery configuration, and a depressed position for facilitating retracting inner sheath 734.

In one example, with stent graft 110 properly positioned at the lesion site, safety pin 764 is removed from housing 754, which retains push button 762 in an initial position. Push button 762 is pressed to release outer sheath 742 and retract outer sheath 742 to automatically deploy graft 114. By pressing push button 762 to move push button 762 with respect to housing 754, outer sheath 742 is released and spring 758 recoils to retract outer sheath 742. In a particular embodiment, inner sheath 734 is partially retracted to partially deploy support stent 726. Retraction element 766 coupled to inner sheath 734 is rotated to unlock retraction element 766 and align connector 768 with a slot formed in outer sheath 742. Retraction element 766 is slid along housing 754 in the distal direction, to retract inner sheath 734 and deploy support stent 126.

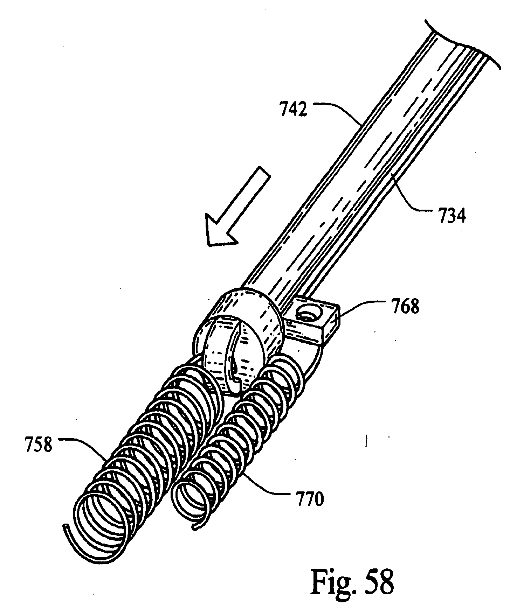

In one example, as shown in Figure 58, each of outer sheath 742 and inner sheath 734 is coupled to a biasing element 758 and 770, respectively, to bias outer sheath 742 and inner sheath 734 towards distal end 760 of housing 754. Each biasing element 758, 770 is operatively coupled to a corresponding push button that is pressed to release respective biasing elements 758, 770 and retract outer sheath 742 and inner sheath 734. In a particular embodiment, the push buttons are pressed substantially simultaneously to release and retract outer sheath 742 and inner sheath 734 and deploy stent graph 110.

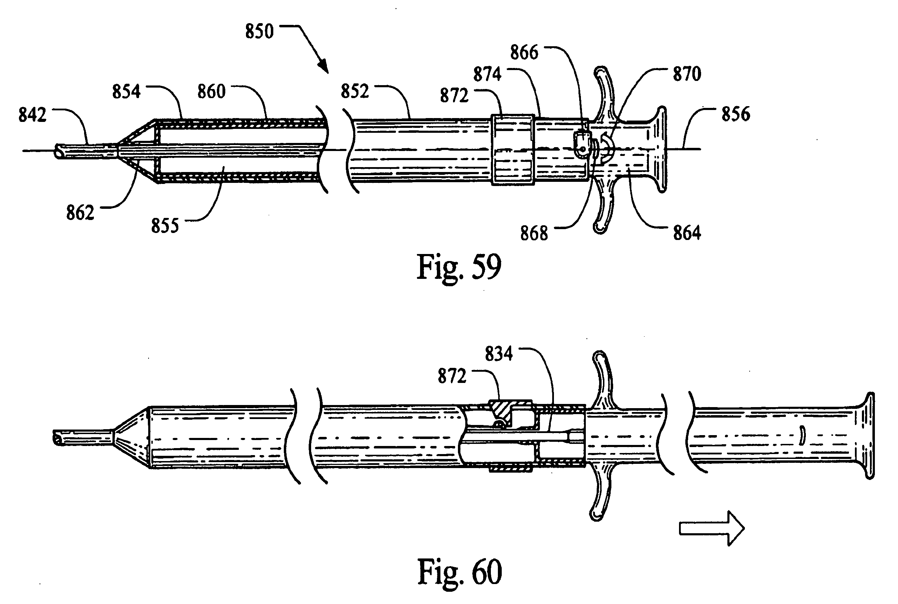

Referring to Figures 59-69, in one embodiment an actuator 850 includes a handle 852 operatively coupled to inner sheath 834 and/or outer sheath 842. Handle 852 includes a housing 854 defining a chamber 855 along at least a portion of a length of housing 854 and an axis 856. As shown in Figures 59-61, at least a portion of inner sheath 834 and/or outer sheath 842 is slidably movable within chamber 855.

In one example, an outer sheath retraction tube 860 is concentrically positioned within housing 854. Outer sheath retraction tube 860 is movable within housing 854 along axis 856 and configured to retract outer sheath 842. As shown in Figure 59, outer sheath 842 is coupled about a nipple 862 formed at a proximal end of outer sheath retraction tube 860. Outer sheath retraction tube 860 transitions into or is coupled to an outer sheath retraction element 864. Outer sheath retraction element 864 is movable along axis 856 to move outer sheath retraction tube 860 in a distal direction along axis 856 and retract outer sheath 842. As shown in Figure 59, an outer sheath locking element 866 is coupled to housing 854 and is configured to prevent or limit movement of outer sheath 842 with respect to axis 856. In one embodiment, outer sheath locking element 866 is rotatably coupled to housing 854. In a locked position, outer sheath locking element 866 contacts a projection 868, such as an arcuate wall, formed on an outer surface of outer sheath retraction grip 864. Outer sheath locking element 866 is rotatable in a rotational direction as shown by directional arrow 870 in Figure 59 to an unlocked position to release outer sheath retraction element 864 for facilitating retracting outer sheath 842.

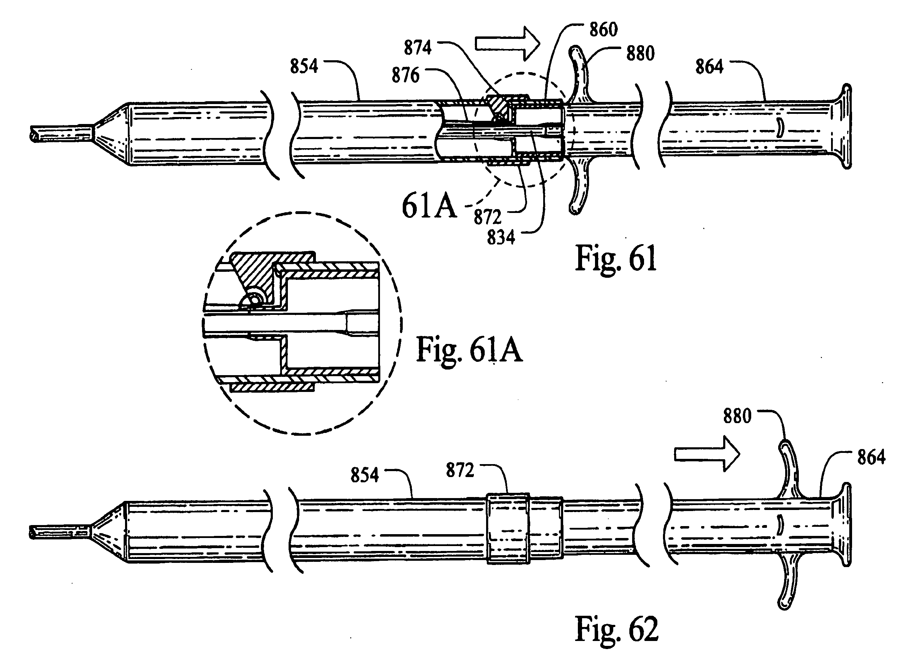

With outer sheath 842 retracted, graft 114 is deployed. In one embodiment, a graft retraction element 872 is coupled to graft 114 and configured to retain graft 114 in a compressed delivery configuration. A graft locking element 874 is formed in or integrated with housing 854. Graft locking element 874 is movable between a locked position, as shown in Figure 59, and an unlocked position, as shown in Figure 61. In the locked position, graft locking element 874 extends from an outer surface of housing 854 to interfere with graft retraction element 872 and prevent or limit movement of graft retraction element 872 along axis 856. Graft locking element 874 is moved inwardly with respect to axis 856 to the unlocked position for facilitating deploying graft 114. As shown in Figure 61, a release suture 876 is coupled between graft retraction element 872 and graft 114 such that as graft retraction element 872 is slide along housing 854, release suture 876 is uncoupled from graft 114 to release graft 114 for deployment.

In one example, an inner sheath retraction element 880 is movably mounted to handle 852 and coupled to inner sheath 834. Inner sheath retraction element 880 is movable along axis 856 and configured to retract inner sheath 834. As inner sheath retraction element 880 is moved in a distal direction along axis 856, inner sheath 834 is retracted and support stent 1 26 is released for deployment.

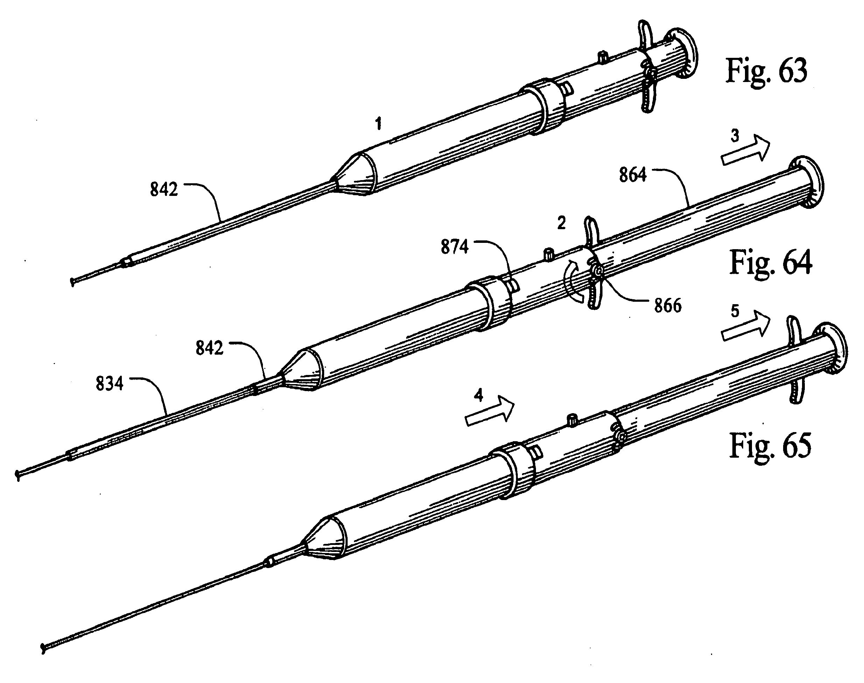

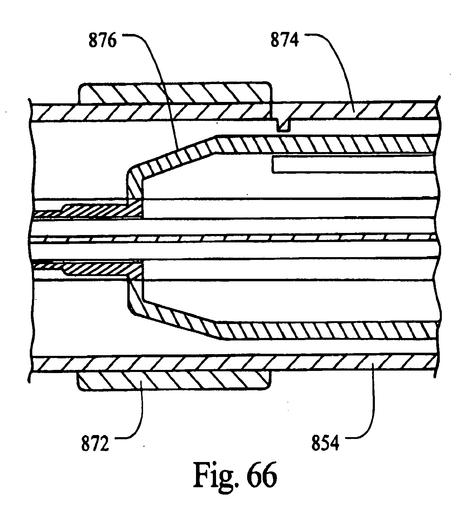

Referring further to Figures 63-69, with stent graft 110 properly positioned at the lesion site, outer sheath retraction tube 860, concentrically positioned within housing 854, is unlocked. In this embodiment, outer sheath locking element 866 is rotated to the unlocked position, as shown in Figure 64. Outer sheath retraction element 864 is pulled in a distal direction along axis 856 to move outer sheath retraction tube 860 within housing 854 and retract outer sheath 842 to expose graft 114. Graft 114 is properly positioned within the vessel at the lesion site and deployed. In one embodiment, graft retraction element 872 is coupled to graft 114 and configured to retain graft 114 in a compressed delivery configuration. Graft locking element 874 is moved from the locked position, as shown in Figure 63, to the unlocked position, as shown in Figures 64 and 66, for facilitating deploying graft 114. Referring further to Figure 66, release suture 876 is uncoupled from graft 114 to release graft 114 for deployment as graft retraction element 872 is slid along housing 854. Inner sheath retraction element 880 is movable in the distal direction along axis 856 to retract inner sheath 834 and release support stent 126 for deployment, as shown in Figure 65.

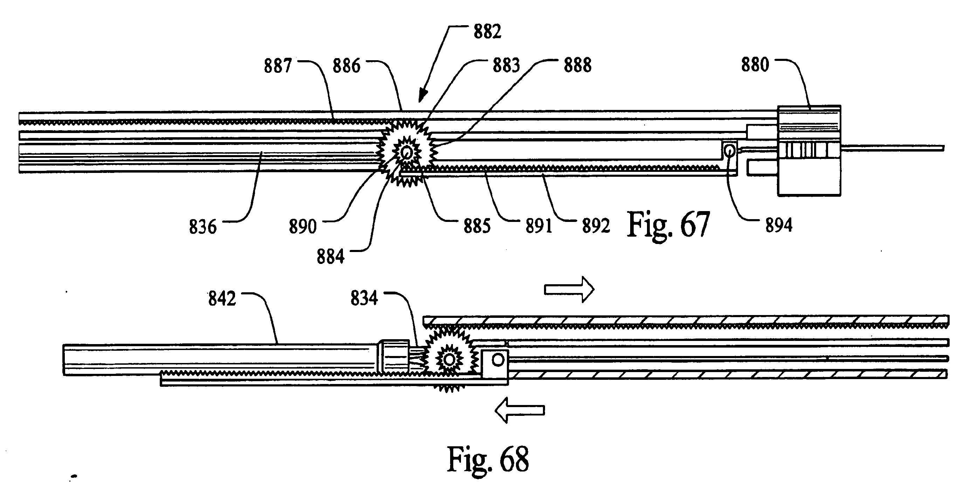

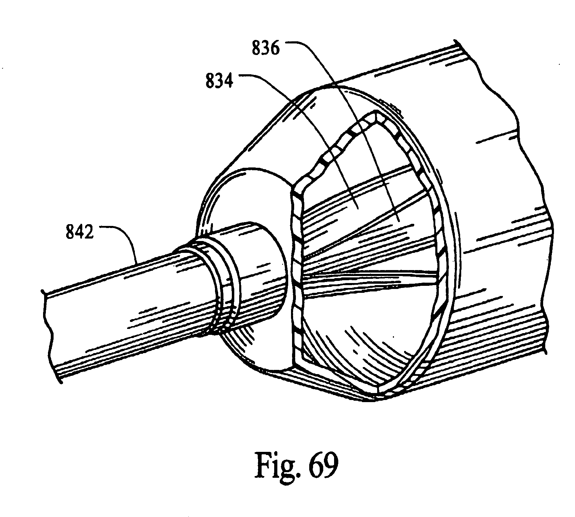

Referring further to Figures 67 and 68, in one example inner sheath retraction element 880 is retracted to activate a gear assembly 882 that advances support member 836 as inner sheath retraction element 880 is retracted. In this embodiment, gear assembly 882 is mounted to housing 854. As shown in Figures 67 and 68, a first gear 883 is rotatably mounted about an axis 884 and a reduction gear 885 is coupled to first gear 883 and coaxially mounted about axis 884. As inner sheath retraction element 880 is drawn or pulled in the distal direction from an initial position, as shown in Figure 67, to a final position, as shown in Figure 68, a rack 886 forming a plurality of teeth 887 cooperates with corresponding teeth 888 formed about a periphery of first gear 883 to rotate first gear 883 about axis 884. As first gear 883 rotates about axis 884, reduction gear 885 coupled to first gear 883 also rotates about axis 884. As shown in Figures 67 and 68, reduction gear 885 forms a plurality of teeth 890 about a periphery of reduction gear 885 that cooperate with a plurality of teeth 891 formed on a rack 892. Rack 892 is coupled to support member 836 at bracket 894. Referring to Figures 67 and 68, as inner sheath retraction element 880 is drawn or pulled in the distal direction to retract inner sheath 834, gear assembly 882 advances support member 836 in an opposing proximal direction to contact support stent 126 and maintain support stent 126 properly positioned at the lesion site. As shown in Figure 69, in one embodiment inner sheath 834 is slotted to accommodate pins and/or support members of gear assembly 882. Inner sheath 834 is coupled to inner sheath retraction element 880 using an interference grip, as shown in Figure 67, or any suitable fitting mechanism. Further, outer sheath retraction tube 860 is slotted to accommodate pins and/or support members of inner sheath retraction element 880. In this example, outer sheath 842 is coupled to outer sheath retraction tube 860 using a barb fitting, as shown in Figure 69, or other suitable fitting.

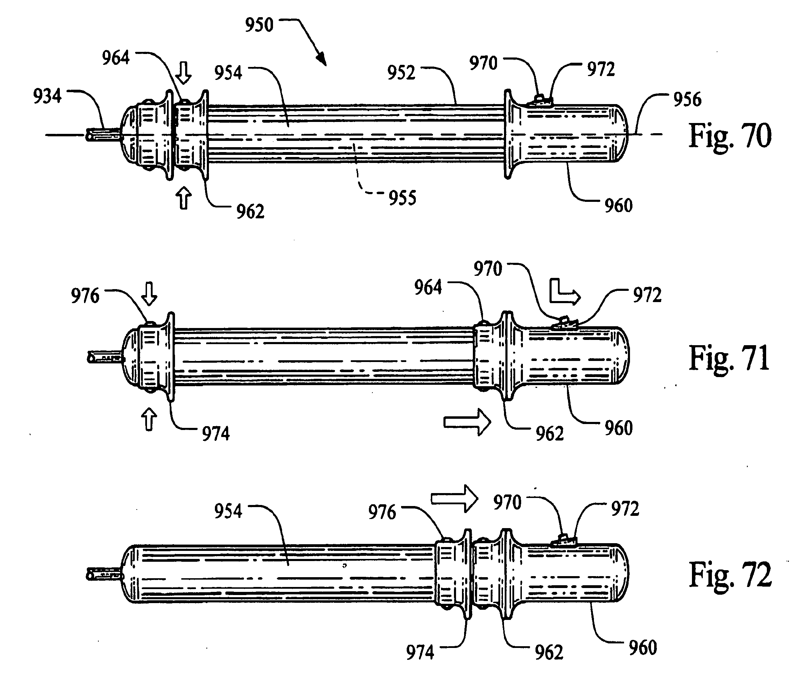

Referring to Figures 70-80, in one example an actuator 950 includes a handle 952 operatively coupled to inner sheath 934 and/or outer sheath 942. Handle 952 includes a housing 954 defining a chamber 955 along at least a portion of a length of housing 954 and an axis 956. A housing grip 960 is coupled to a distal end of housing 954, as shown in Figures 70-72. An outer sheath retraction element 962 is positioned about housing 954 and coupled to outer sheath 942. Outer sheath retraction element 962 is slidably movable along housing 954 with respect to axis 956 between a proximal end of housing 954 and housing grip 960 to retract outer sheath 942. In one embodiment, at least one locking element 964 is coupled to or positioned with respect to outer sheath retraction element 962 and configured to retain outer sheath retraction element 962 in a locked position, as shown in Figure 70. With outer sheath retraction element 962 in the locked position, movement of outer sheath 942 with respect to axis 956 is prevented or limited. By pressing cooperating locking element 964, outer sheath retraction element 962 is released to an unlocked position for facilitating retracting outer sheath 942. In one embodiment, in the unlocked position outer sheath retraction element 962 is movable in a distal direction along axis 956 to retract outer sheath 942 and expose graft 114.

With outer sheath 942 retracted, graft 114 is deployed. In one embodiment, a graft release locking element 970 is mounted to housing grip 960 and is configured to control and/or activate release and/or deployment of graft 114. A graft retraction element 972 is operatively coupled to graft release locking element 970. Further, graft retraction element 972 is operatively coupled to graft 114. Movement of graft retraction element 972 initiates deployment of graft 114.

Referring to Figure 70, graft release locking element 970 initially retains graft retraction element 972 in a locked position and graft 114 in a delivery configuration. Graft release locking element 970 is movable between a biased position and a release position, such as pressing graft release locking element 970, to move graft retraction element 972 to an unlocked position, as shown in Figure 71. In the unlocked position, graft retraction element 972 is slidably movable with respect to housing grip 960 for facilitating deploying graft 114.

In one example, an inner sheath retraction element 974 is positioned about housing 954 and coupled to inner sheath 934. Inner sheath retraction element 974 is slidably movable along housing 954 with respect to axis 956 between a proximal end of housing 954 and outer sheath retraction element 962 to retract inner sheath 934, as shown in Figure 72. In this embodiment, a locking element 976 is coupled to or positioned with respect to inner sheath retraction element 974 and configured to retain inner sheath retraction element 974 in a locked position, as shown in Figure 71. With inner sheath retraction element 974 in the locked position, movement of inner sheath 934 with respect to axis 956 is prevented or limited. By pressing locking element 976, inner sheath retraction element 974 is released to an unlocked position for facilitating retracting inner sheath 934. In one embodiment, in the unlocked position inner sheath retraction element 974 is movable in a distal direction along axis 956 to retract inner sheath 934 and release and/or deploy support stent 126, as shown in Figure 72.

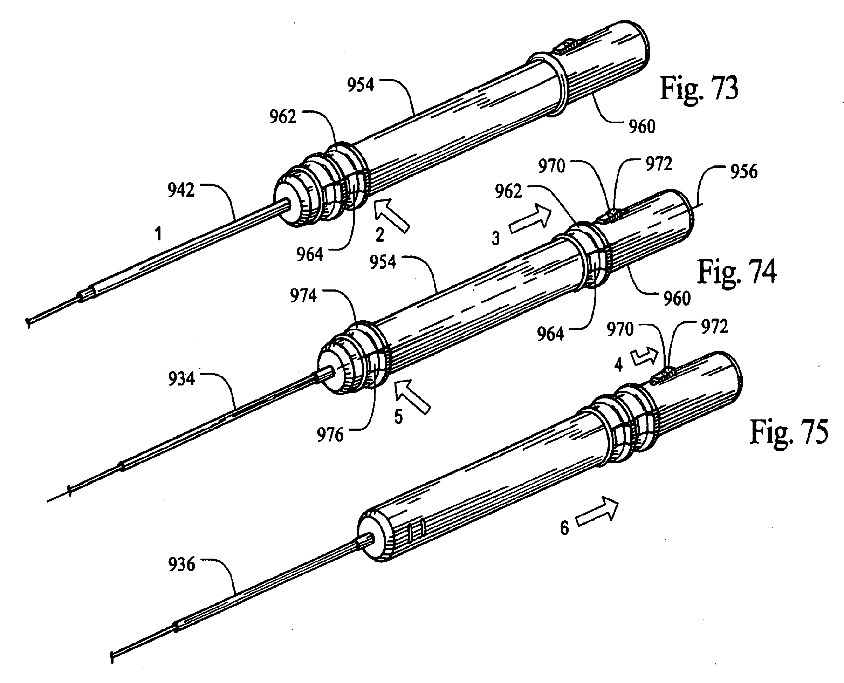

Referring further to Figures 73-80, with stent graft 110 properly positioned at the lesion site, outer sheath retraction element 962, positioned about housing 954 and coupled to outer sheath 942, is unlocked by pressing locking element 964 to release outer sheath retraction element 962, as shown in Figure 73. As shown in Figure 74, outer sheath retraction element 962 is movable in the distal direction along housing 954 with respect to axis 956 between the proximal end of housing 954 and housing grip 960 to retract outer sheath 942 and expose graft 114.

With outer sheath 942 retracted, graft 114 positioned within the vessel at the lesion site is deployed. Graft release locking element 970 is movable between the biased position and the release position, such as pressing graft release locking element 970, to move graft retraction element 972 to an unlocked position, as shown in Figure 75. In the unlocked position, graft retraction element 972 is slidably movable with respect to housing grip 960 to deploy graft 114.

After graft 114 is deployed, inner sheath 934 is retracted to deploy support stent 126. As shown in Figure 74, in one embodiment, inner sheath retraction element 974 is unlocked by pressing locking element 976, which is movable between the locked position and the unlocked position. In the locked position, locking element 976 is configured to limit movement of inner sheath 934 with respect to axis 956. Inner sheath retraction element 974 is moved along housing 954 between a proximal end of housing 954 and outer sheath retraction element 962, as shown in Figure 75, to retract inner sheath 934 and deploy support stent 126.

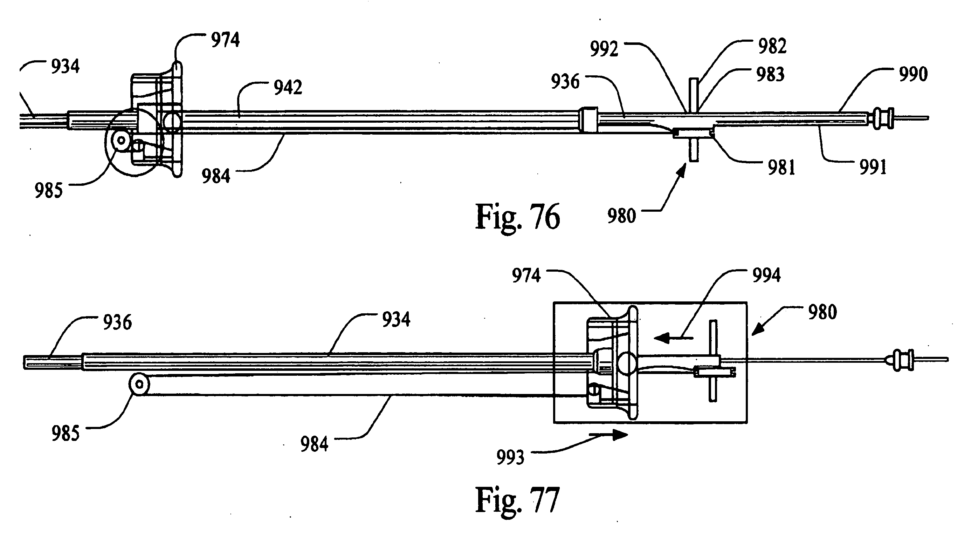

Referring further to Figures 76 and 77, in one example inner sheath retraction element 974 is retracted to activate a rack and pinion assembly 980 that advances support member 936 as inner sheath retraction element 974 is moved in the distal direction along housing 954. In this embodiment, rack and pinion assembly 980 includes a pulley 981 rotatable mounted about a shaft 982 that is mounted to housing 954. As shown in Figures 76 and 77, a pinion 983 is coupled to pulley 981 and coaxially mounted about shaft 982. A cord 984 is coupled at a first end to a distal end of inner sheath 934 and extends and wraps around pulley 981 of rack and pinion assembly 980. Cord 984 extends in a proximal direction with respect to rack and pinion assembly 980 through inner sheath retraction element 974 to wrap about a second pulley 985 rotatably mounted to housing 954 proximal to inner sheath retraction element 974. As shown in Figures 76 and 77, cord 984 wraps around second pulley 985 and is coupled at a second end to inner sheath retraction element 974. In this embodiment, a support member 936 includes a rack portion 990 forming a plurality of teeth 991 that cooperate with corresponding teeth 992 formed about a periphery of pinion 983. As inner sheath retraction element 974 is drawn or pulled in the distal direction as shown by directional arrow 993, from an initial position as shown in Figure 76 to a final position as shown in Figure 77, cord 984 is drawn or pulled in the distal direction. Pulley 981 rotates as inner sheath 934 is retracted. Pinion 983 coupled to pulley 981 also rotates such that teeth 992 formed on the periphery of pinion 983 cooperate with corresponding teeth 991 formed on rack portion 990 to advance support member 936 in an opposing proximal direction as shown by directional arrow 994 in Figure 77. Support member 936 advances to contact support stent 1 26 and maintain support stent 1 26 properly positioned at the lesion site.

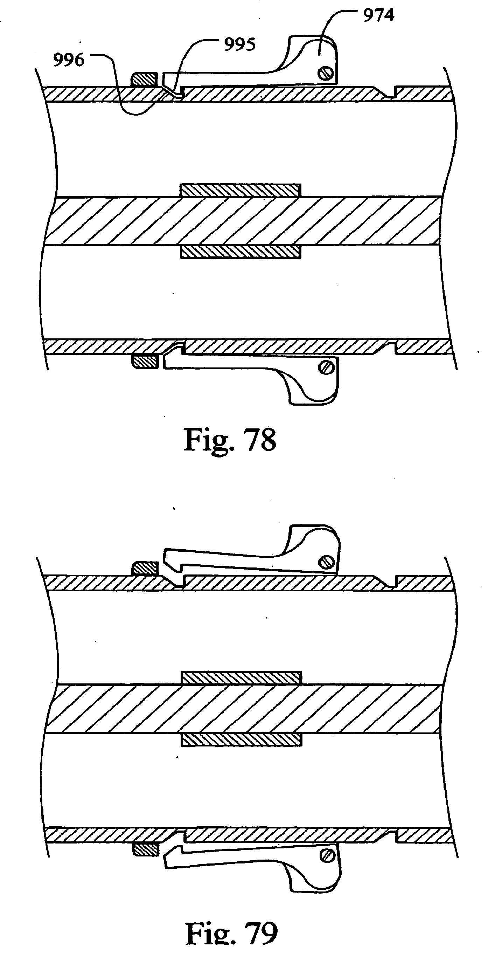

As shown in Figure 78, locking elements 976 are pivotally coupled to inner sheath retraction element 974 such that with inner sheath retraction element 974 in the locked position a snap component 995 formed on locking elements 976 are positioned within a corresponding depression 996 defined in housing 954. By pressing locking elements 976, snap component 995 is released from within corresponding depression 996 and inner sheath retraction element 974 is released to an unlocked position for facilitating retracting inner sheath 934, as shown in Figure 79..

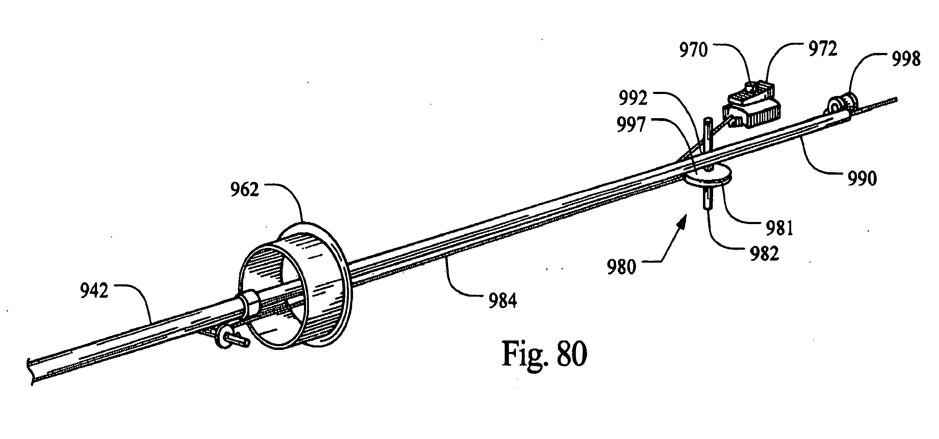

In a particular example, a suture 997 is coupled at a first end to graft retraction element 972, as shown in Figure 80. An opposing second end of suture 997 is coupled about graft 1 14 (not shown) using at least one slip knot or other suitable coupling mechanism or technique. As graft retraction element 972 is pulled, suture 997 is pulled to release the slip knot coupled about graft 114 to release graft 114, which is then deployed to a deployed position at the lesion site. In a further particular embodiment, a luer lock fitting 998 is positioned with respect to chamber 955 defined within housing 954 for facilitating sufficient irrigation during the procedure.

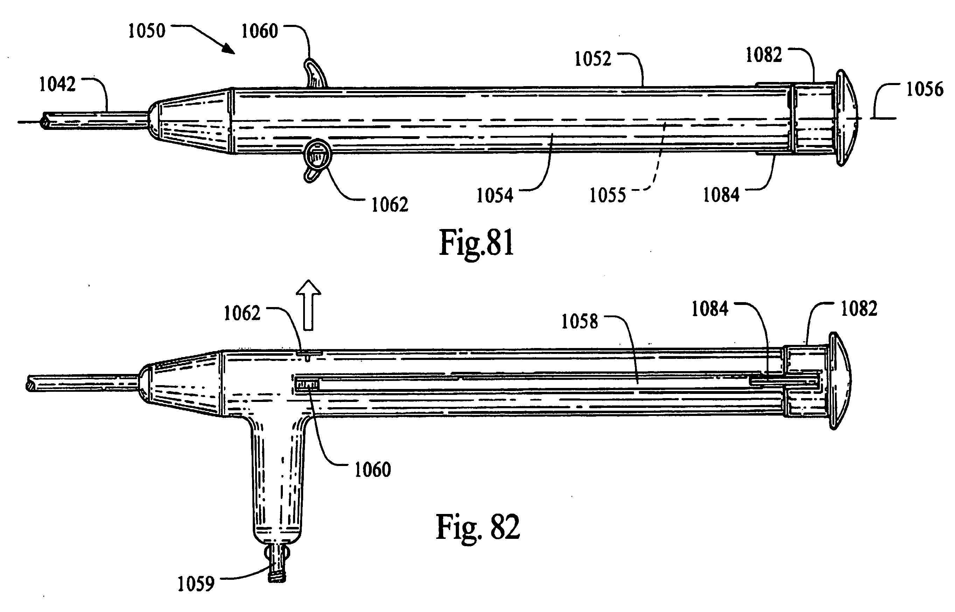

Referring to Figures 81-90, in one example an actuator 1050 includes a handle 1052 operatively coupled to inner sheath 1034 and/or outer sheath 1042. Handle 1052 includes a housing 1054 defining a chamber 1055 along at least a portion of a length of housing 1054 and an axis 1056. As shown in Figure 82, housing 1054 further defines a track 1058 in communication with at least a portion of chamber 1055. In one example, handle 1052 includes an irrigation tube 1059 coupled to or integrated with handle 1052 and in fluid communication with the vessel for facilitating irrigating undesirable fluids and/or air from within the vessel during the procedure.

An outer sheath retraction element 1060 is coupled to outer sheath 1042 and at least partially positioned within track 1058. Outer sheath retraction element 1060 is movable within track 1058 and configured to retract outer sheath 1042. A locking element 1062 is positionable within housing 1054 and configured to lock outer sheath retraction element 1060 to prevent or limit movement of outer sheath retraction element 1060 within track 1058.

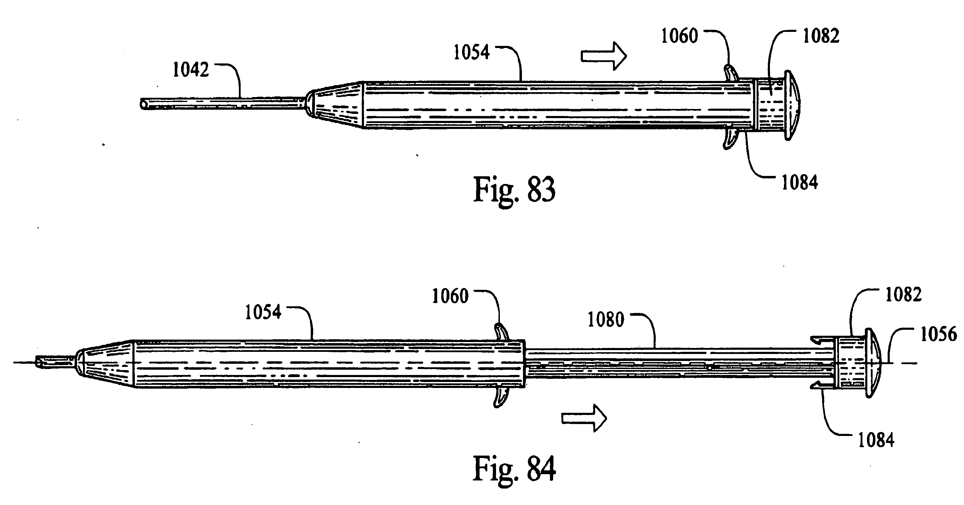

An inner sheath retraction tube 1080 is movably positioned at least partially within chamber 1055 and coupled to inner sheath 1034. Referring to Figure 84, inner sheath retraction tube 1080 is movable within chamber 1055 along axis 1056 for facilitating retracting inner sheath 1034. In one embodiment, a retraction element 1082 is coupled to or integrated with inner sheath retraction tube 1080. Retraction element 1082 is initially coupled to a distal end of housing 1054, as shown in Figures 81-83, to retain inner sheath retraction tube 1080 in a locked position to prevent or limit undesirable movement of inner sheath 1034. In a particular embodiment, retraction element 1082 includes at least one finger 1084 that is initially coupled to the distal end of housing 1054. As shown in Figure 82, finger 1084 is initially positioned within a corresponding track 1058 to couple retraction element 1082 to housing 1054. As outer sheath retraction element 1060 is moved in the distal direction with respect to axis 1056, outer sheath retraction element 1060 contacts fingers 1084 coupling retraction element 1082 to housing 1054. Such contact unlocks inner sheath retraction tube 1080, which is then moved in the distal direction with respect housing 1054 along axis 1056 to retract inner sheath 1034 and release and/or deploy support stent 126.

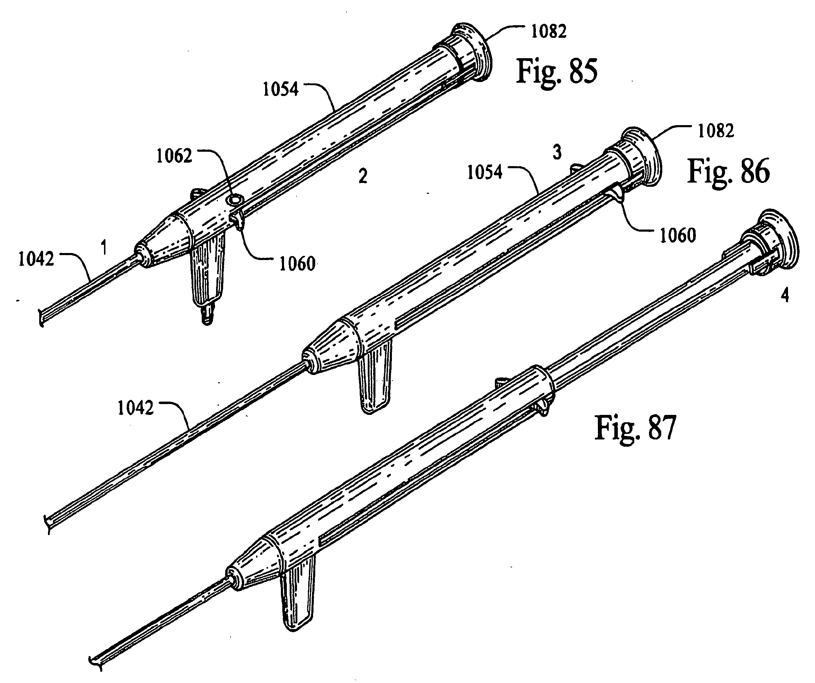

Referring to Figures 85-90, with stent graft 110 properly positioned at the lesion site, the delivery system is unlocked by removing locking element 1062 from within housing 1054. Locking element 1062 is initially coupled through housing 1054 to outer sheath retraction element 1060 and is configured to retain outer sheath 1042 and inner sheath 1034 in a delivery configuration, as shown in Figure 85. As shown in Figure 86, outer sheath retraction element 1060 is movable in the distal direction along housing 1054 with respect to axis 1056 between the proximal end of housing 1054 and retraction element 1082 coupled to the distal end of housing 1054 to retract outer sheath 1042 and automatically release graft 114. As outer sheath retraction element 1060 is moved along housing 1054, outer sheath retraction element 1060 contacts retraction element 1082 to decouple retraction element 1082 from housing 1054. As shown in Figure 87, retraction element 1082 is then moved along axis 1056 in a distal direction to retract inner sheath 1034 and release and/or deploy support stent 126.

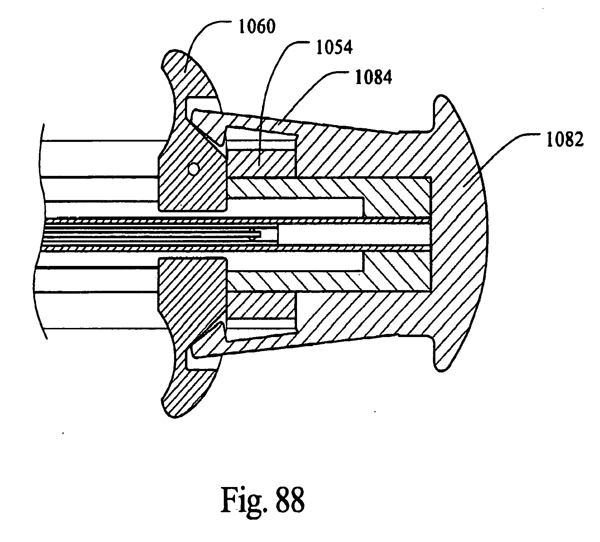

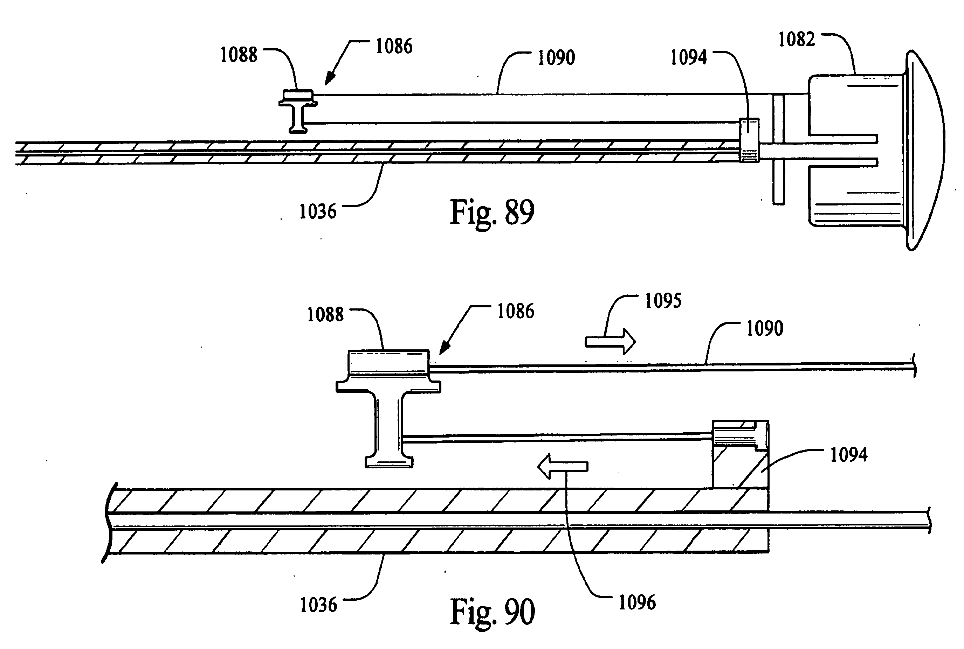

Referring further to Figures 88-90, in one example with outer sheath retraction element 1060 in a retracted position, outer sheath retraction element 1060 contacts fingers 1084 to unlock fingers 1084 from housing 1054 and decouple retraction element 1082 from housing 1054. Inner sheath retraction tube 1080 is then moved in the distal direction with respect housing 1054 along axis 1056 to retract inner sheath 1034 and release and/or deploy support stent 126. As shown in Figures 89 and 90, retraction element 1082 is retracted to activate a spindle arrangement 1086 for facilitating advancing support member 1036 as inner sheath retraction tube 1080 is moved in the distal direction along housing 1054. In this example, spindle arrangement 1086 includes a spindle 1088 rotatably mounted to housing 1054. A cord 1090 is coupled at a first end to retraction element 1082 and extends in the proximal direction to wrap around and/or through spindle 1088. Cord 1090 extends in the distal direction with respect to spindle 1088 and is coupled at an opposing second end to support member 1036. In one example, support member 1036 includes a block 1094 to which cord 1090 is coupled. In this embodiment, as retraction element 1082, and inner sheath retraction tube 1080 coupled thereto, is drawn in the distal direction as shown by directional arrow 1095, from an initial position as shown in Figure 89 to a final position as shown in Figure 90, cord 1090 is drawn or pulled in the distal direction, which causes support member 1036 to advance in an opposing proximal direction as shown by directional arrow 1096. Cord 1090 moves about spindle 1088 causing spindle 1088 to rotate for facilitating smooth retraction of inner sheath 1034 and accurate deployment of support stent 126 at the lesion site.

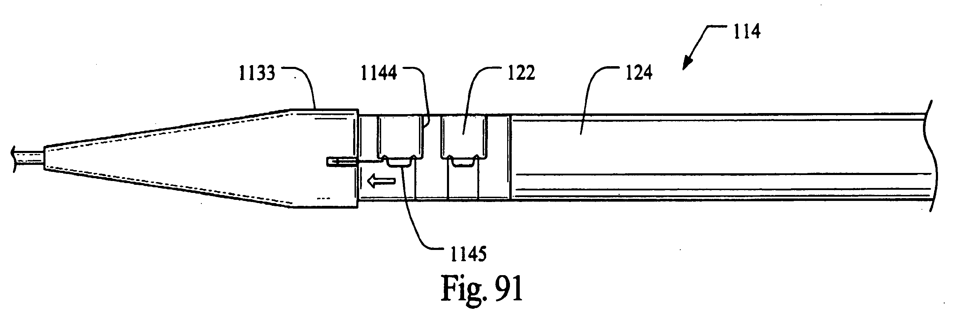

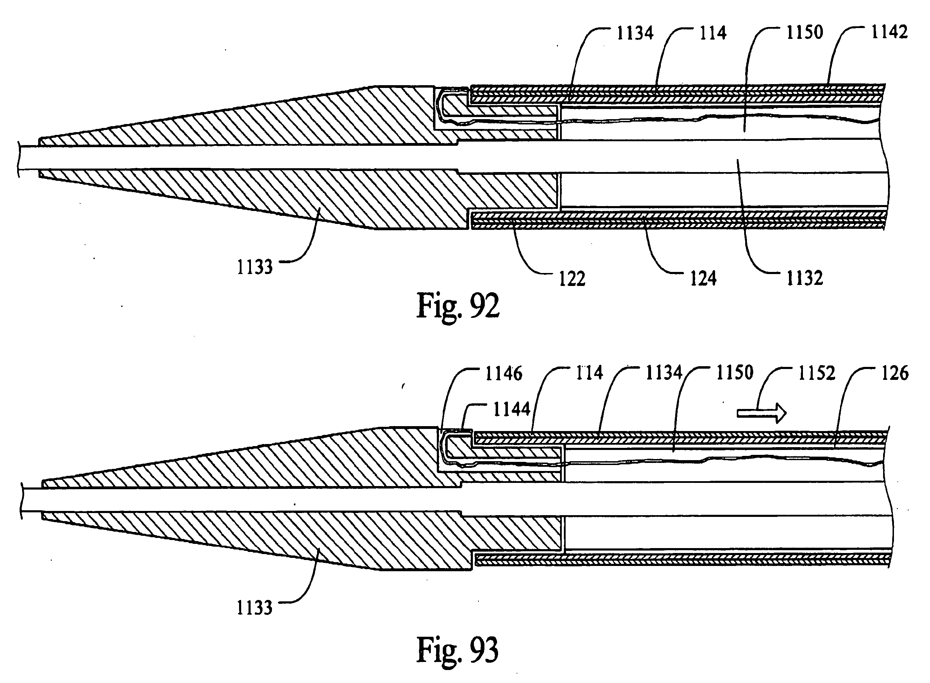

Referring now to Figures 91-93, during a thoracic aortic aneurysm repair procedure, a delivery system 1130 delivers and/or positions stent graft 110 with respect to the lesion site at or near the aneurysm. As shown in Figure 92 and 93, graft 114 is slidably positioned about inner sheath 1134. A portion of inner sheath 1134 is coupled to nose cone 1133. Outer sheath 1142 is retractably positioned about graft 114 with graft 114 in the delivery configuration to maintain graft 114 in the delivery configuration as stent graft 110 is advanced to the lesion site. With stent graft 110 positioned within the vessel as desired, outer sheath 1142 is retractable for facilitating deployment of graft 114 from the delivery configuration to the deployed configuration.

In one embodiment, a pull suture 1144 is positioned about at least a portion of graft 114, such as graft portion 122, and configured to temporarily maintain graft 114 in the compressed delivery configuration after outer sheath 1142 is retracted from about graft 114. As shown in Figure 91, pull suture 1144 includes at least one slip knot 1145 to maintain graft 114 in the compressed delivery configuration. Pull suture 1144 is fed through a passage 1146 formed in nose cone 1133 and into passage 1150 defined between an inner surface of support stent 126 and an outer surface of wire lumen 1132. Pull suture 1100 is coupled to an actuator, such as described above, that is configured to pull or drawn pull suture 1100 to release graft 114, which then expands from the delivery configuration to the deployed configuration.

With delivery system 1130 at the lesion site, outer sheath 1142 is moved in a distal direction, as shown by directional arrow 1152 in Figure 93, to retract outer sheath 1142 and expose at least a portion of graft 114. The actuator is activated to release pull suture 1144 from about graft 114 and graft 114 expands in a radial direction with respect to wire lumen 1132 between the delivery configuration and the deployed configuration. In the deployed configuration, an outer radial surface of graft 114 contacts the interior surface of the vessel wall at the lesion site and graft 114 defines a passage therethrough. Proximal end 118 of graft 114 is positioned proximal to the aneurysm and distal end 120 is positioned distal to the aneurysm.

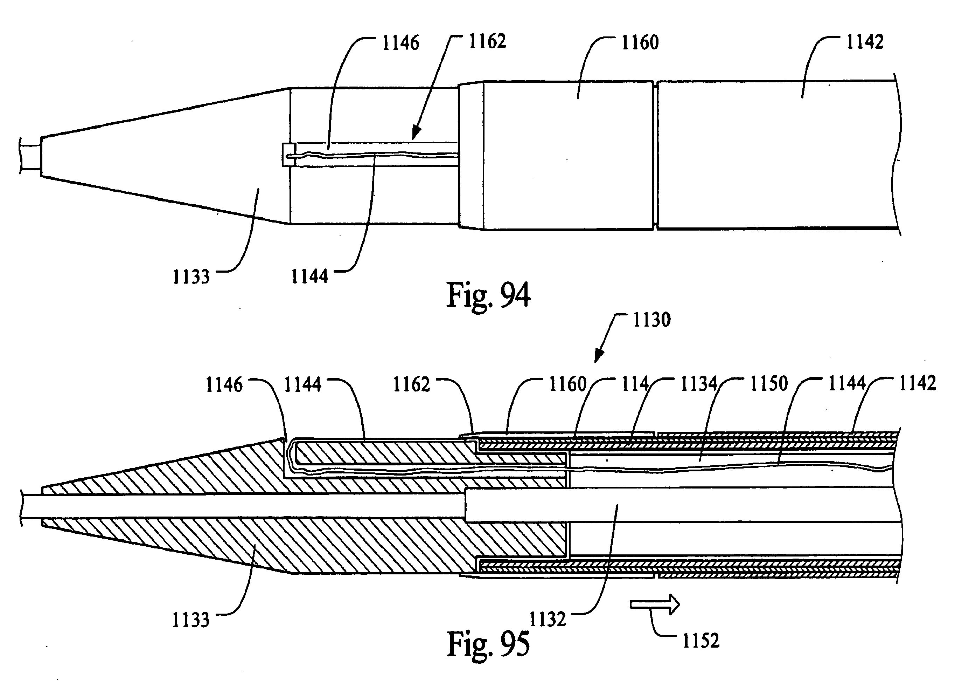

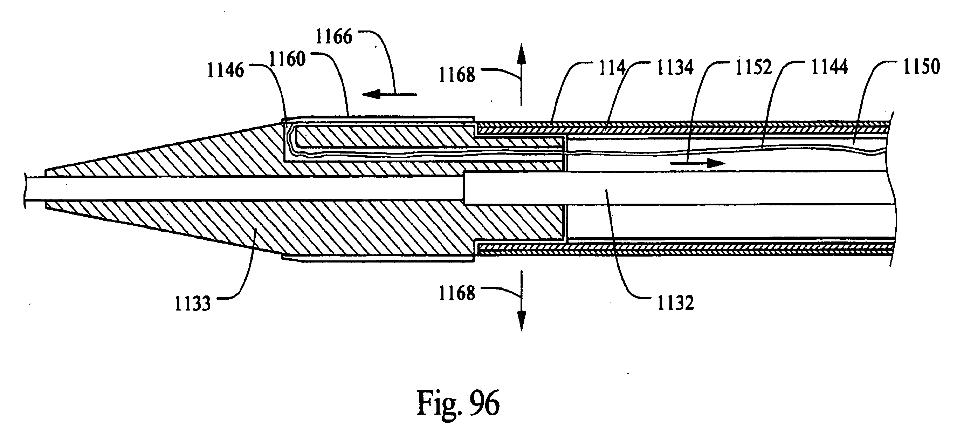

In another example, as shown in Figures 94-96, pull suture 1144 is coupled to a retaining ring 1160 positioned about at least a portion of graft 114, such as graft portion 122. Retaining ring 1160 is slidably movable with respect to nose cone 1133 between an initial position and a release position. In the initial position, retaining ring 1160 is configured to temporarily maintain graft 114 in the compressed delivery configuration after outer sheath 1142 is retracted from about graft 114, as shown in Figures 94 and 95. In the release position, as shown in Figure 96, retaining ring is configured for facilitating deployment of graft 114. Pull suture 1144 is coupled at a first end 1162 to retaining ring 1160 and fed through passage 1146 defined by nose cone 1133, as shown in Figure 94, and into passage 1150 defined between an inner surface of support stent 126 and an outer surface of wire lumen 1132, as shown in Figures 95 and 96. A second end 1164 of pull suture 1144 is coupled to an actuator, such as described above, that is configured to pull or draw pull suture 1144 in the distal direction, as shown by directional arrow 1152 in Figure 96, and move retaining ring 1160 in an opposing proximal direction, as shown by directional arrow 1166 in Figure 96, to release graft 114. Released graft 114 expands in a radial direction, as shown by directional arrows 1168 in Figure 96, from the delivery configuration to the deployed configuration.

With delivery system 1130 at the lesion site, outer sheath 1142 is moved in the distal direction, as shown by directional arrow 1152 in Figure 95, to retract outer sheath 1142 and expose at least a portion of graft 114. The actuator is activated to pull or draw pull suture 1144 in the distal direction and move retaining ring 1160 in the proximal direction to release graft 114, as shown in Figure 96. Graft 114 expands in a radial direction with respect to wire lumen 1132 between the delivery configuration and the deployed configuration. In the deployed configuration, an outer radial surface of graft 114 contacts the interior surface of the vessel wall at the lesion site and graft 114 defines a passage therethrough. Proximal end 118 of graft 114 is positioned proximal to the aneurysm and distal end 120 is positioned distal to the aneurysm.

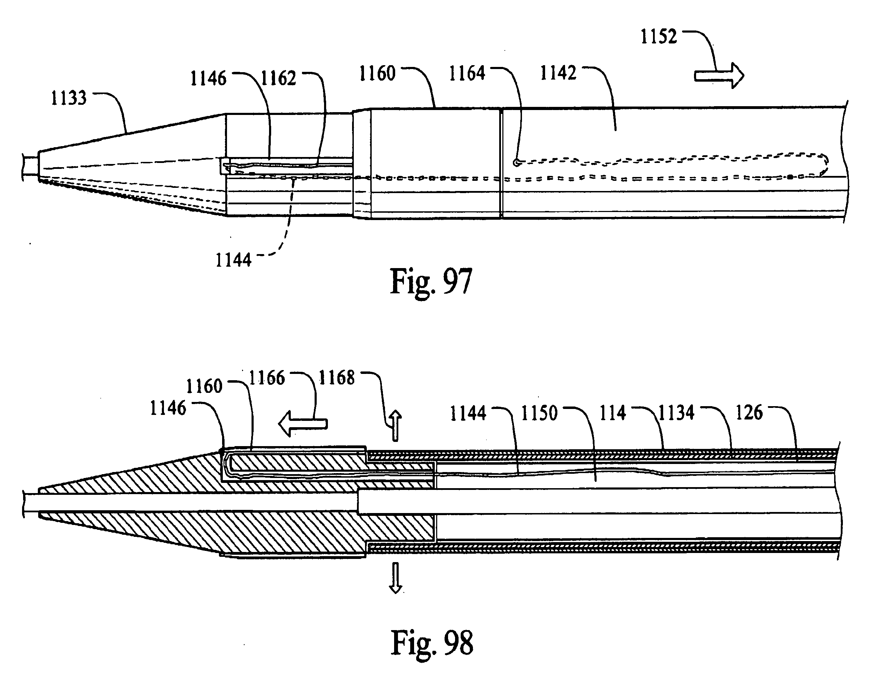

In another example, as shown in Figures 97 and 98, second end 1164 of pull suture 1144 is coupled to outer sheath 1142. With delivery system 1130 at the lesion site, outer sheath 1142 is moved in the distal direction, as shown by directional arrow 1152 in Figure 97, to retract outer sheath 1142 and expose at least a portion of graft 114. As outer sheath 1142 is retracted, outer sheath 1142 draws pull suture 1144 in the distal direction and moves retaining ring 1160 in the proximal direction to release graft 114, as shown in Figure 98. Graft 114 expands in a radial direction with respect to wire lumen 1132 between the delivery configuration and the deployed configuration.

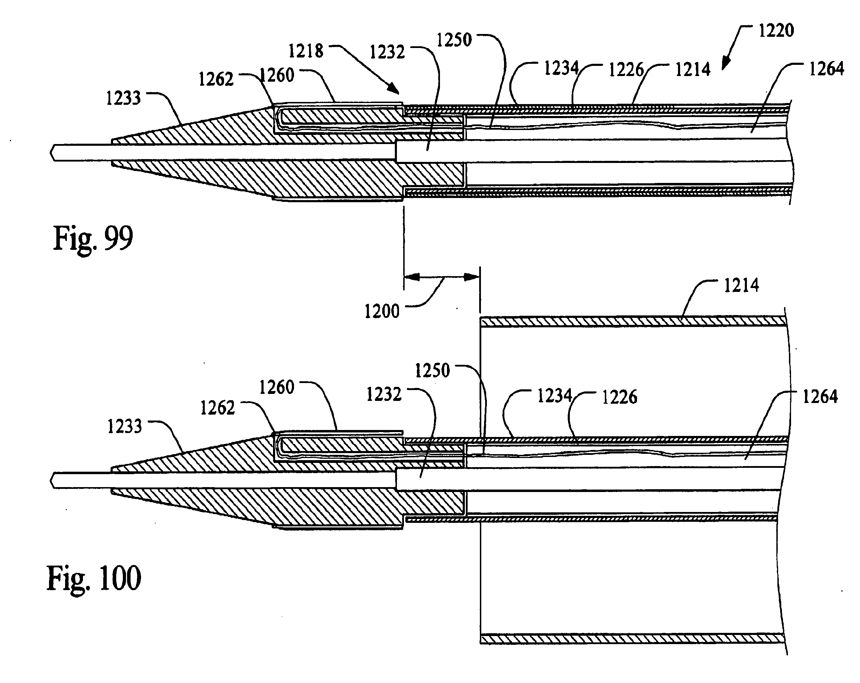

Referring to Figures 99-103, in yet another example, graft 114 is deployed in two stages to prevent undesirable axial migration of graft 114 upon deployment. For example, referring further to Figures 99 and 100, the aorta has a diameter of about 1.12 inches (2.84 cm) and a cross-section area of about O. 1284 in² (0.8239 cm²). Blood flows through the aorta at a velocity of about 12.99 in/sec (32.99 cm/sec). As a result, upon deployment of graft 114, graft 114 will be displaced in a distal direction a distance 1200, as shown in Figure 99, based on several parameters including, without limitation, blood flow rate, dimensions of the aorta section, resisting surface area and/or deployment time.

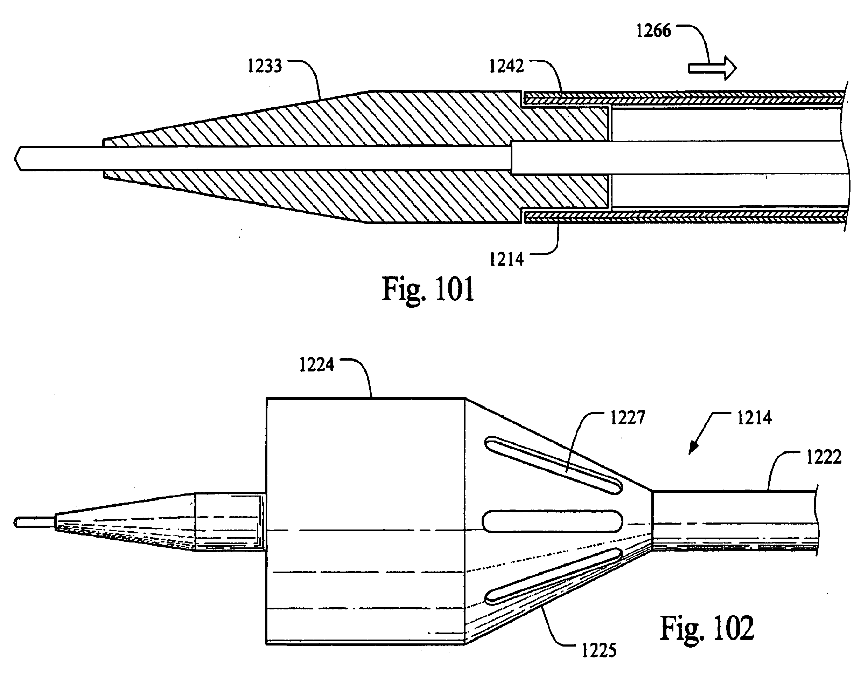

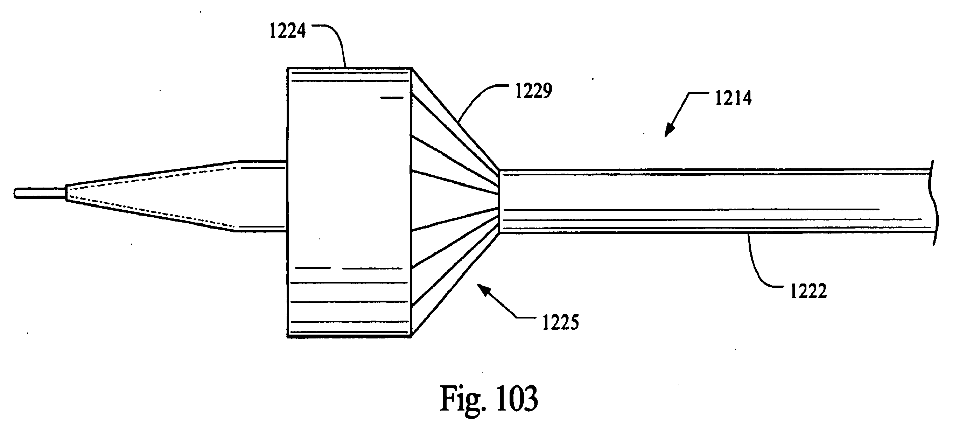

Referring now to Figures 101-103, in this example, a graft 1214 is deployed in two stages to prevent undesirable axial migration of graft 1214 upon deployment. In a first stage, outer sheath 1242 is retracted a first distance, such as about 1.0 inch (about 2.5 cm) to about 2.0 inches (about 5.0 cm), for facilitating partial deployment of graft 1214 to increase the accuracy of placement of graft 1214 without a graft portion 1222 free to migrate. During the first stage, an anchor portion 1224 of graft 1214 is deployed, as shown in Figure 102. Upon deployment of anchor portion 1224, outer sheath 1242 is retracted during a second stage to deploy the remaining portion of graft 1214. During the second stage, a speed at which outer sheath 1242 is retracted is substantially equal to a blood flow rate through the vessel to minimize pressure on graft 1214 during deployment. In a particular embodiment, graft 1214 includes a transition portion 1225 coupling anchor portion 1224 to graft portion 1222. Transition portion 1225 defines a plurality of perforations 1227, as shown in Figure 102, for facilitating blood flow through graft 1214 as graft 1214 expands to engage the inner wall of the aorta. In another embodiment, transition portion 1225 includes a plurality of sutures 1229 that couple anchor portion 1224 to graft portion 1222, as shown in Figure 103, for facilitating blood flow through graft 1214 as graft 1214 expands.

For example, referring further to Figures 99 and 100, a pull suture 1250 is coupled to a retaining ring 1260 positioned about at least a portion of graft 1214, such as graft portion 122. Retaining ring 1260 is slidably movable with respect to nose cone 1233 between an initial position configured to temporarily maintain graft 1214 in the compressed delivery configuration after outer sheath 1242 is retracted from about graft 1214, as shown in Figure 99, and a release position, as shown in Figure 100, for facilitating deployment of graft 1214. Pull suture 1250 is coupled at a first end to retaining ring 1260 and fed through passage 1262 defined by nose cone 1233, as shown in Figures 99 and 100, and into passage 1264 defined between an inner surface of support stent 1226 and an outer surface of wire lumen 1232, as shown in Figures 99 and 100. A second end (not shown) of pull suture 1250 is coupled to an actuator, such as described above, that is configured to draw pull suture 1250 in the distal direction, as shown by directional arrow 1266 in Figure 101, and move retaining ring 1260 in an opposing proximal direction, to release graft 1214. Released graft 1214 expands in a radial direction, from the delivery configuration to the deployed configuration.

With delivery system 1230 at the lesion site, outer sheath 1242 is moved in the distal direction, to retract outer sheath 1242 and expose at least a portion of graft 1214. The actuator is activated to pull or draw pull suture 1250 in the distal direction and move retaining ring 1260 in the proximal direction to release graft 1214, as shown in Figure 99. Graft 1214 expands in a radial direction with respect to wire lumen 1232 between the delivery configuration and the deployed configuration. In the deployed configuration, an outer radial surface of graft 1214 contacts the interior surface of the vessel wall at the lesion site and graft 1214 defines a passage therethrough. Proximal end 1218 of graft 1214 is positioned proximal to the aneurysm and distal end 1220 is positioned distal to the aneurysm.

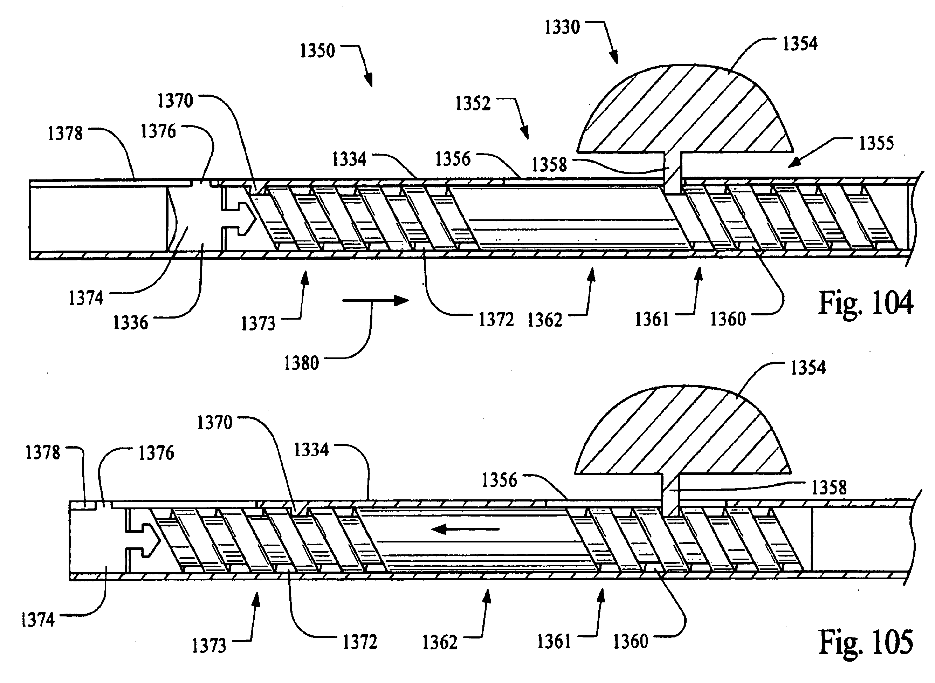

Referring to Figures 104 and 105, in one example a delivery system 1330 includes an actuator 1350 having a handle 1352 operatively coupled to inner sheath 1334 and an outer sheath (not shown). Handle 1352 includes a housing 1354 defining a chamber 1355. Inner sheath 1334 is slidably positioned within chamber 1355 and defines a first slot 1356. As shown in Figures 104 and 105, a first or stationary projection 1358 formed by housing 1354 extends through slot 1356 and is positioned within a helical groove 1360 at least partially forming a first cam 1361 within a distal portion 1362 of support member 1336. A second projection 1370 formed on an inner surface of inner sheath 1334 is positioned within a helical groove 1372 at least partially forming a second cam 1373 within distal portion 1362. A tip portion 1374 of support member 1336 is coupled to distal portion 1362 and includes or forms a key 1376 that extends at least partially into a second slot 1378 defined by inner sheath 1334. Inner sheath 1334 is retracted by moving inner sheath 1334 in a distal direction, as shown by directional arrow 1380 in Figure 104. As inner sheath 1334 is moved in the distal direction, second cam 1373 causes projection 1370 to rotationally advance along helical groove 1372 as first cam 1361 advances with respect to stationary projection 1358 formed on housing 1354. Key 1376 positioned within second slot 1378 prevents tip portion 1374 from rotating as tip portion 1374 moves in the proximal direction. In this embodiment, as inner sheath 1334 is retracted in the distal direction, support member 1336 is advanced in the opposing proximal direction to maintain support stent 126 properly positioned at the lesion site and with respect to graft 114.

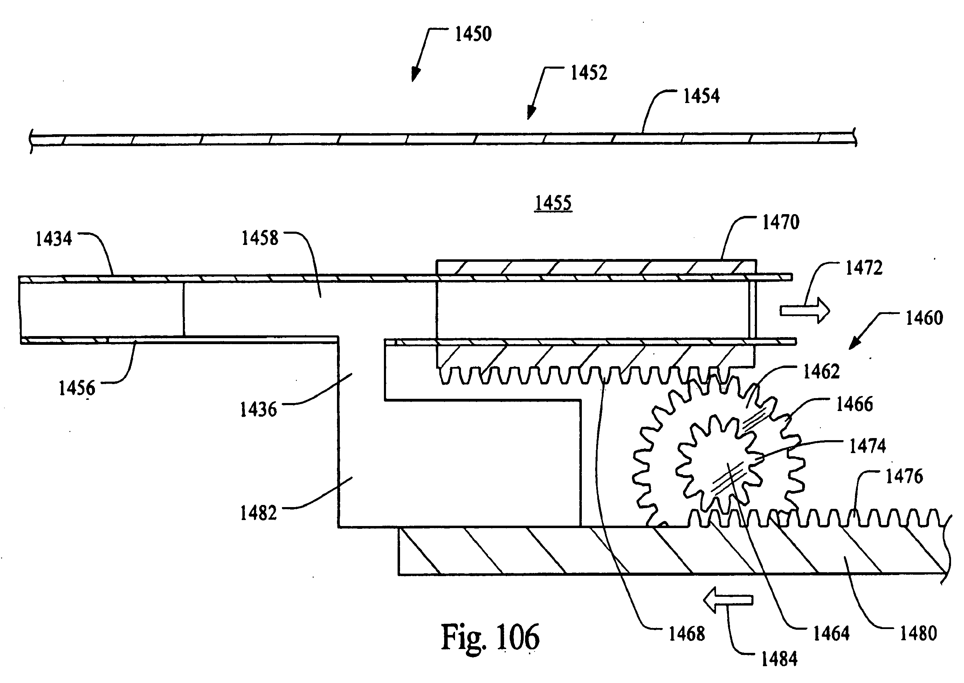

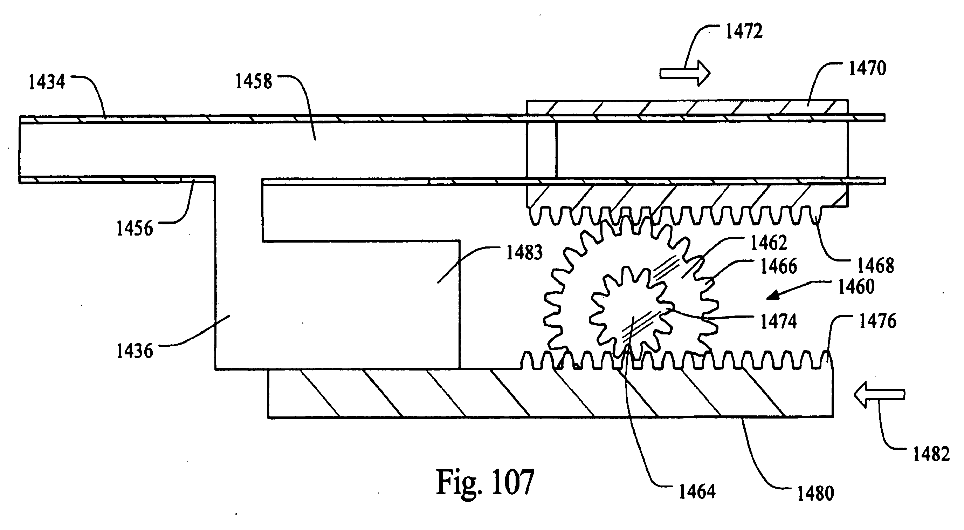

In another example, a delivery system 1430 includes an actuator 1450 having a handle 1452 operatively coupled to inner sheath 1434 and an outer sheath (not shown). Handle 1452 includes a housing 1454 defining a chamber 1455. Inner sheath 1434 is slidably positioned within chamber 1455 and defines a first slot 1456. As shown in Figures 106 and 107, a first portion 1458 of support member 1436 extends through first slot 1456 and is slidably positioned within inner sheath 1434. A gear assembly 1460 is rotatably mounted within housing 1454 and includes a first gear 1462 and a reduction gear 1464. First gear 1462 forms a plurality of teeth 1466 that cooperate with a plurality of teeth 1468 formed on a rack 1470 coupled to inner sheath 134. As inner sheath 134 is moved in a distal direction, as shown by directional arrow 1472 in Figure 106, rack 1470 moves with respect to first gear 1462 causing first gear 1462 to rotate as each tooth 1468 cooperates with corresponding teeth 1466 formed on first gear 1462. Simultaneously, reduction gear 1464 rotates and a plurality of teeth 1474 formed on reduction gear 1464 cooperate with a plurality of teeth 1476 formed on a rack 1480 to cause rack 1480 to move in a proximal direction as shown by directional arrow 1482 in Figure 107. Rack 1480 is coupled to a base portion 1483 of support member 1436 and, thus, movement of rack 1480 in the proximal direction results in advancement of support member 1436 within inner sheath 1434.

In this example, inner sheath 1434 is retracted by moving inner sheath 1434 in the distal direction. As inner sheath 1434 moves in the distal direction, rack 1470 moves with respect to first gear 1462 to cause gear assembly 1460 to rotate. As gear assembly 1460 rotates, rack 1480 moves in the proximal direction as shown by directional arrow 1484, causing first portion 1458 of support member 1436 to advance, as shown in Figure 107. In this embodiment, as inner sheath 1434 is retracted in the distal direction, support member 1436 is advanced in the opposing proximal direction to maintain support stent 126 properly positioned at the lesion site and with respect to graft 114.

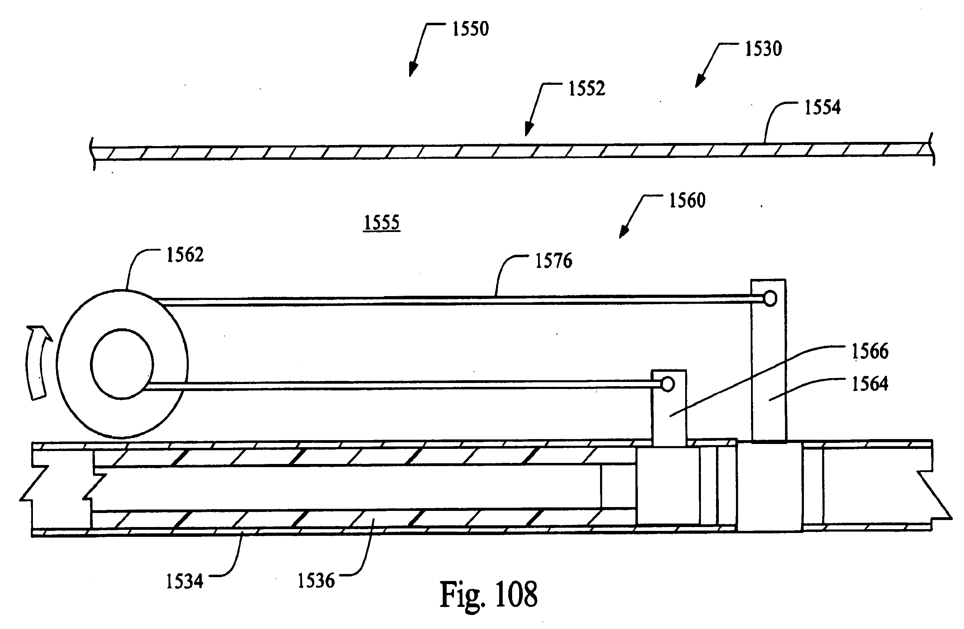

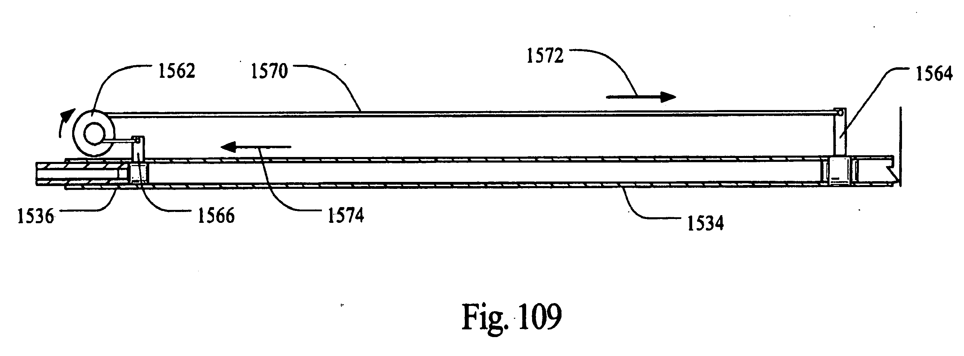

In another example, a delivery system 1530 includes an actuator 1550 having a handle 1552 operatively coupled to inner sheath 1534 and an outer sheath (not shown). Handle 1552 includes a housing 1554 defining a chamber 1555. Inner sheath 1534 is slidably positioned within chamber 1555. As shown in Figures 108 and 109, a pulley assembly 1560 is positioned within housing 1554. Pulley assembly 1560 includes a hub 1562 rotatably mounted to housing 1554. A first bracket 1564 is coupled to inner sheath 1534 and a second bracket 1566 is positioned within inner sheath 1534 to contact support member 1536. A first end of a pull cord 1570 is coupled to first bracket 1564 and wrapped around hub 1562. An opposing second end of pull cord 1570 is coupled to second bracket 1566. As inner sheath 1534 is moved in a distal direction, as shown by directional arrow 1572 in Figure 109, first bracket 1564, coupled to inner sheath 1534, also moves in the distal direction, which causes hub 1562 to rotate and draw second bracket 1566 in an opposing proximal direction, as shown by directional arrow 1574 in Figure 109. As second bracket 1566 moves in the proximal direction, second bracket 1566 contacts support member 1536 and urges support member 1536 to advance in the proximal direction to maintain support stent 126 properly positioned at the lesion site and with respect to graft 114.

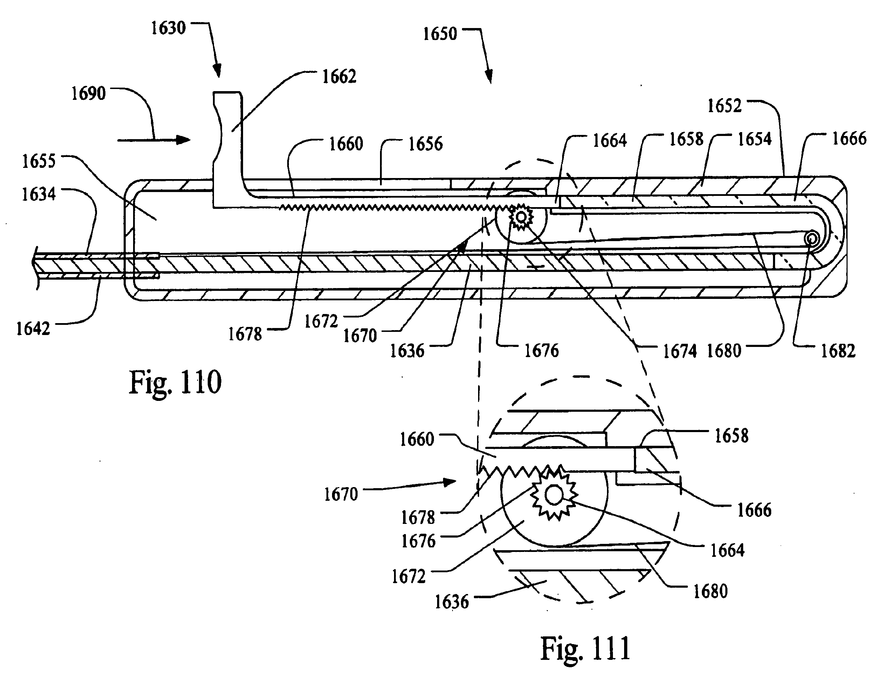

In another example, delivery system 1630 includes an actuator 1650 having a handle 1652 operatively coupled to inner sheath 1634 and outer sheath 1642. Handle 1652 includes a housing 1654 defining a chamber 1655 and a slot 1656 along at least a portion of a length of housing 1654. Further, as shown in Figure 110, housing 1654 defines an inner passage 1658. A retraction element 1660 is positioned within slot 1656. Retraction element 1660 includes a first portion 1662 external to housing 1654 and a second portion 1664 at least partially positioned within inner passage 1658. In one example, a semi-rigid or bendable member 1666 is at least partially positioned within inner passage 1658 between second portion 1664 and support member 1636. A pulley/spindle assembly 1670 is rotatably positioned within housing 1654 and includes a pulley 1672 and a spindle 1674 coaxially coupled to pulley 1672. Spindle 1674 forms a plurality of teeth 1676 that cooperate with a plurality of corresponding teeth 1678 formed on retraction element 1660, as described in greater detail below. Pulley 1672 is coupled to inner sheath 1634 with a pull cord 1680. Pull cord 1680 is coupled at a first end to pulley 1672 and is positioned about a pulley 1682. A second end of pull cord 1680 is coupled to inner sheath 1634.

Referring to Figures 110 and 111, retraction element 1 660 is moved in a distal direction as shown by directional arrow 1690 in Figure 110. As retraction element 1660 is moved, teeth 1678 cooperate with teeth 1676 of spindle 1674 to rotate pulley/spindle assembly 1670. As pulley 1672 rotates, pull cord 1680 is wrapped about an outer periphery of pulley 1672 to retract inner sheath 1634. Simultaneously, retraction element 1660 pushes semi-rigid member 1666 through inner passage 1658 to contact support member 1636. Semi-rigid member 1666 urges support member 1636 to advance in the proximal direction to maintain support stent 126 properly positioned at the lesion site and with respect to graft 114.

In another example, delivery system 1730 includes an actuator 1750 having a handle 1752 operatively coupled to inner sheath 1734 and an outer sheath (not shown). Handle 1752 includes a housing 1754 defining a chamber 1755 and a slot 1756 along at least a portion of a length of housing 1754. Further, as shown in Figure 112, housing 1754 defines an inner passage 1758. Inner passage 1758 includes a sealing member 1759, such as an O-ring or other suitable sealing member known to those skilled in the art and guided by the teachings herein provided, positioned at an inlet end 1760 and a generally opposing outlet end 1762 and configured to sealingly contain a hydraulic fluid, such as water, within inner passage 1758. A retraction element 1764 is positioned within slot 1756. Retraction element 1764 includes a first portion 1766 external to housing 1754 and a second portion 1768 at least partially positioned within inner passage 1758. A pulley/spindle assembly 1770 is rotatably positioned within housing 1754 and includes a pulley 1772 and a spindle 1774 coaxially coupled to pulley 1772. Spindle 1774 forms a plurality of teeth 1776 that cooperate with a plurality of corresponding teeth 1778 formed on second portion 1768 of retraction element 1764, as described in greater detail below. Pulley 1772 is coupled to inner sheath 1734 with a pull cord 1780. Pull cord 1780 is coupled at a first end to pulley 1772 and is positioned about a pulley 1782. A second end of pull cord 1780 is coupled to inner sheath 1734.

Referring to Figures 112 and 113, retraction element 1764 is moved in a distal direction as shown by directional arrow 1790 in Figure 112. As retraction element 1764 is moved, teeth 1778 cooperate with teeth 1776 of spindle 1774 to rotate pulley/spindle assembly 1770. As pulley 1772 rotates, pull cord 1780 is wrapped about an outer periphery of pulley 1772 to retract inner sheath 1734. Simultaneously, retraction element 1764 provides a force against the hydraulic fluid within inner passage 1758 to advance support member 1736. The hydraulic fluid urges support member 1736 to advance in the proximal direction to maintain support stent 126 properly positioned at the lesion site and with respect to graft 114.

### Delivery System for Generic Prosthesis

Figure 114 is a partial sectional view of a delivery system 1810. Components of delivery system 1810 may have any suitable shape, size and/or configuration. Delivery system 1810 can be used in conjunction with a plurality of components including, without limitation, a balloon catheter, a dual balloon catheter a trans-medicinal catheter and/or a multi-branched catheter.

In one example, prosthesis delivery system 1810 includes a catheter 1812 including a support member 1814 and a catheter sheath 1816. In a particular embodiment, delivery system 1810 also includes an expandable balloon (not shown). A prosthesis 1818, such as a stent or stent graft, is positioned on delivery system 1810.

Catheter 1812 has any suitable shape and/or size. Further, catheter 1812 is fabricated using any suitable material that enables catheter 1812 to function as described herein. Catheter 1812 includes an elongate shaft 1820 defining a guide wire passage 1822 extending therethrough from a proximal end 1824 to a distal end 1826 along an axis 1828.

In operation, a guide wire 1830 extends through guide wire passage 1822 to guide delivery system 1810 to a target location or lesion site, as shown in Figure 114. In one embodiment, a nose cone 1832 is coupled to shaft distal end 1826. Nose cone 1832 includes a guide wire passage 1834 extending therethrough. Nose cone 1832 facilitates advancement of catheter 1812 through a body lumen to the lesion site.

In one example, shaft 1820 is slidably coupled to support member 1814 and/or prosthesis 1818. Specifically, at least a portion of shaft 1820, such as distal end 1826, is circumferentially surrounded by support member 1814 and prosthesis 1818. In another embodiment, shaft distal end 1826 is coupled to an expandable balloon (not shown) which extends within prosthesis 1818. In an embodiment, prosthesis 1818 is a tubular, radially expandable prosthesis, such as a stent, a vascular graft, a stent graft composite, a Nitinol stent, a covered stent, a mesh stent, a braided stent, a tapered stent, a Z stent, a Wallstent or a combination thereof. It will be apparent to those skilled in the art and guided by the teachings herein provided that prosthesis 1818 may include any suitable prosthesis. In this embodiment, prosthesis 1818 is radially expandable between a generally unexpanded configuration having an unexpanded insertion diameter and an expanded configuration having an expanded or deployment diameter, which is greater than the insertion diameter. Prosthesis 1818 is flexible and coupled to shaft 1820 in a radially compressed configuration and then expanded at the lesion site. In one embodiment, prosthesis 1818 is fabricated from self-expandable material having a spring-like action and/or memory properties, such as temperature-dependant memory properties. In another embodiment, a balloon positioned with respect to prosthesis 1818 facilitates expansion of prosthesis 1818. Prosthesis 1818 is radially distensible or deformable. It will be apparent to those skilled in the art and guided by the teachings herein provided that any suitable mechanism for expanding and/or enabling the expansion of prosthesis 1818 may be employed without departure from the scope of the appended claims.

Prosthesis 1818 may have any suitable geometry and/or configuration. Further, prosthesis 1818 may be fabricated of any suitable biocompatible material including, without limitation, a suitable metal, such as stainless steel, platinum, gold and titanium, an alloy and/or a polymeric material. In one embodiment, prosthesis 1818 is fabricated from a Nitinol material, which exhibits a spring-like or shape-memory deformation.

In one example, prosthesis 1818 includes an outer surface 1836 in frictional contact with sheath 1816 and an inner surface 1838 in frictional contact with shaft 1820. Prosthesis 1818 is positioned between support member 1814 and nose cone 1832. Prosthesis 1818 is configured to be deployed by support member 1814 and/or sheath 1816.

Support member 1814 defines a distal end 1840 and an opposing proximal end 1842. An elongate body 1844 extends between distal end 1840 and proximal end 1842. In one embodiment, body 1844 was a tubular shape forming a passage through which shaft 1820 extends. In other embodiments, body 1844 has any suitable shape and/or size. In one embodiment, support member 1814 is fabricated from a polyetheramide such as Pebaxe obtainable from Elf Atochem S.A. of Paris, France. In another embodiment, support member 1814 is fabricated from a suitable polymeric material, such as a polyether amide, or any suitable material that enables support member 1814 to function as described herein.

In one example, support member distal end 1840 is positioned adjacent a prosthesis proximal end 1846 and in a contacting relationship with proximal end 1846. In a particular embodiment, support member 1814 is releasably coupled to prosthesis 1818. In one embodiment, support member proximal end 1842 is coupled to a catheter handle 1850, which will be discussed in greater detail below.
Support member body 1844 has a diameter 1852 substantially equal to an unexpanded diameter 1854 of prosthesis 1818 and less than an inner diameter 1856 of sheath 1816. Support member 1814 is sized to fit within sheath 1816 and slidably contact an inner surface 1858 of sheath 1816. Support member 1814 and sheath 1816 are fabricated with tight tolerances such that a frictional force exists between sheath inner surface 1858 and a support member outer surface 1860. Specifically, support member 1814 frictionally contacts sheath 1816, and is movable within sheath 1816. As will be discussed in further detail below, support member 1814 is configured to contact and/or engage and deploy prosthesis 1818 at the lesion site.

Support member 1814 has a suitable length 1862. In one example, length 1862 is greater than a prosthesis length 1864 and less than a sheath length 1866. In other embodiments, lengths 1862, 1864, 1866, and diameters 1852, 1854, 1856, have different lengths and/or diameters than the above-indicated lengths and/or diameters, depending upon the particular application.

Catheter sheath 1816 defines a distal end 1870, and an opposing proximal end 1872. An elongate body 1874 extends between distal end 1870 and proximal end 1872. Body 1874 defines a housing, a sleeve, a sock or any suitable assembly for surrounding and retaining prosthesis 1818 and/or support member 1814 properly position on catheter 1812. In one embodiment, body 1874 has a tubular shape. Sheath 1816 is sized to overlay prosthesis 1818 and support member 1814. In other embodiments, body 1874 has any suitable shape and/or size. In yet another embodiment, sheath 1816 is substantially shorter than support member 1814. In one embodiment, sheath 1816 is retractable. hi another embodiment, sheath 1816 is coupled to handle 1850 and is configured to move in a proximal direction and/or distal direction.

In one embodiment, sheath 1816 is fabricated from a braided, reinforced extruded material. In another embodiment, sheath 1816 is fabricated from Pebax™ material or any suitable polymeric material. In further embodiments, sheath 1816 is fabricated from a suitable material that enables sheath 1816 to function as described herein.

In one example, sheath 1816 is configured to have a yield strength greater than a self-expansion force of prosthesis 1818. As such, sheath 1816 retains prosthesis 1818 in a compressed or unexpanded configuration during delivery of prosthesis 1818. While the yield strength of sheath 1816 is sufficient to maintain prosthesis 1818 in a compressed state, sheath 1816 is configured to axially move over an outside surface 1876 of support member 1814 along axis 1828 during deployment. In one example, sheath 1816 is slidably coupled with prosthesis 1818 and/or support member 1814 for facilitating retaining of prosthesis 1818 adjacent and/or in contacting relationship with support member 1814 during delivery and deployment of prosthesis 1818. In one embodiment, sheath 1816 is releasably coupled to nose cone 1832.

Handle 1850 is configured to simultaneously impart relative movement to support member 1814 and sheath 1816 in opposite directions. More specifically, handle 1850 simultaneously imparts a proximal movement on support member 1814 and a distal movement on sheath 1816 during deployment of prosthesis 1818. This relative movement is in an axial direction and the ratio of movement is based, at least partially, on a predetermined foreshortening percentage of prosthesis 1818. In one embodiment, this relative movement ratio is based on the specific prosthesis included in delivery system 1810. In embodiments, handle 1850 includes an adjustable relative movement control member 1878 configured to vary the amount of axial force according to the predetermined foreshortening percentage of prosthesis 1818 and the specific usage of delivery system 1810.

Figure 1 15 is a sectional view of an exemplary prosthesis delivery system 1810 before deployment. Figure 116 is a sectional view of an exemplary prosthesis delivery system 1810 during deployment. Figure 11 7 is a sectional view of an exemplary prosthesis delivery system 1810 after deployment. Figures 115-117 share common location reference numbers to aid in understanding the deployment of delivery system 1810 at selected stages of deployment. These numbers are for illustration and are not meant to limit in any way the application of prosthesis delivery system 1810.

In one example, prosthesis 1818 is a self-expanding stent 1819 configured to contact and/or engage an interior surface of lumen wall 1900. Before deployment, stent 1819 is releasably coupled to or loaded on shaft 1820 in a compressed configuration. Guide wire 1830 is percutaneously inserted into a patient's lumen or vessel, and guide wire 1830 is guided to a location 1902 proximal to a target location or lesion site 1904 such that guide wire distal end 1906 is positioned at lesion site 1904. Catheter 1812 is then positioned such that guide wire 1830 extends through passage 1822 in nose cone 1832 and shaft 1820. Nose cone 1832 is guided to lesion site 1904 such that stent proximal end 1908 is positioned at a target location proximal end 1910 and stent distal end 1909 is positioned at a target location distal end 1912.

During deployment at lesion site 1904, support member 1814 advances proximal while, simultaneously, sheath 1816 retracts distally and guide wire end 1906 and nose cone 1832 are kept stationary relative to location 1902. More specifically, a first axial force is applied to support member 1814 in a proximal direction 1920 along axis 1828. The first axial force is greater than the frictional force applied against sheath inner surface 1858 by compressed stent 1819 and support member 1814, thus support member 1814 engages stent 1819. Simultaneously, a second axial force is applied in a distal direction 1922 opposite proximal direction 1920 and sheath 1816 releases stent 1819 which begins to expand as stent 1819 exits sheath 1816. The second axial force is greater that the frictional force applied by prosthesis 1818 and/or the interior surface of lumen wall 1900. In this embodiment, "simultaneously" refers to the first and second axial forces imparted substantially concurrently.

The amount of the first axial force is sufficient to maintain stent 1819 stationary. In one embodiment, first axial force and second axial force are determined by the foreshortening percentage of stent 1819 as well as the friction between sheath 1816 and stent 1819 and/or support member 1814. In one embodiment, the first axial force and the second axial force are equal. In another embodiment, the first axial force and the second axial force are different.

After deployment of stent 1819, stent 1819 is fully expanded and accurately positioned at lesion site 1904. Specifically, stent proximal end 1908 is positioned at target location proximal end 1910 and stent distal end 1909 is positioned at target location distal end 1912. Additionally, guide wire end 1906 remains at location 1902. Catheter 1812 including guide wire 1830, nose cone 1832, support member 1814, sheath 1816 and shaft 1820 are withdrawn in distal direction 1922 from the patient, leaving stent 1819 properly positioned.

While Figures 115-177 illustrate a delivery system to facilitate accurate positioning of a self-expanding prosthesis, the advantages apply to all types of prostheses. The system can be sized and configured for use in various body lumens, specifically, any other lumen where accurate location of a stent or prosthesis is desired.

While the invention has been described in terms of various specific embodiments, those skilled in the art will recognize that the invention can be practiced with modification within the scope of the claims.

## Claims

1. A stent graft for deployment within a body vessel, including:
a tubular graft provided with a proximal end and a distal end;
a support stent, at least a portion of which lies within the tubular graft, provided with a proximal end and a distal end;
wherein the support stent includes at least one wire forming a first helical wire portion having a first translational direction about an axis of the support stent and a second helical wire portion having a second translational direction about the axis opposite the first translational direction;
wherein when the support stent is in a compressed delivery configuration, the support stent has a first length, and wherein when the support stent is in an expanded deployed configuration, the support stent has a second length which is less than the first length; and
wherein when in the delivery configuration, the support stent is attached to the graft only at or near the proximal end of the graft and the graft is positioned about an inner sheath of a delivery system, which inner sheath covers at least a portion of the support stent; **characterized in that** the stent graft further includes at least one locking mechanism at or near the distal end of the graft and configured to engage the support stent in the deployed configuration, thereby substantially to fix the diameter and length of the support stent in the deployed configuration.

2. A stent graft according to claim 1, wherein the support stent in the deployed configuration has a curvature which approximates a curvature of the body vessel.

3. A stent graft according to claim 1 or 2, wherein the deployed configuration is set prior to deployment within the body vessel.

4. A stent graft according to any preceding claim, wherein the locking mechanism includes a ring and at least one engagement member configured to engage the support stent.

5. A stent graft according to claim 4, wherein the engagement member includes at least one prong configured to engage at least one of the first helical wire portion and the second helical wire portion.

6. A stent graft according to any preceding claim, wherein the at least one locking mechanism comprises a stent at the distal end of the graft.

7. A stent graft according to any preceding claim, comprising at least one anchor stent attached to at least one end of the graft.

8. A stent graft according to claim 7, wherein the at least one anchor stent includes a stent attached to the graft at or near to the graft proximal end.

9. A stent graft according to any preceding claim, wherein the support stent second length is equal to or greater than the length of the graft.

10. A stent graft according to any preceding claim, including a distal ring attached at or near the distal end of the graft.

11. A stent graft according to claim 10, wherein the distal ring comprises the or a locking mechanism.

12. A stent graft according to any preceding claim, wherein in the deployed configuration the support stent is positioned at least partly within the graft.

13. A stent graft according to any preceding claim, including a capture mechanism operatively coupled to the support stent proximal end, the capture mechanism being configurable to retain the support stent proximal end in the delivery configuration and being actuatable to release the proximal end of the support stent.

14. A stent graft according to any preceding claim, wherein the support stent in the deployed configuration has a curvature of between 0° and 180°.

15. A delivery system including a stent graft as claimed in any preceding claim.

## Patentansprüche

1. Stent-Graft zur Entfaltung innerhalb eines Blutgefäßes, umfassend:
Ein röhrenförmiges synthetisches Gewebe, das mit einem proximalen Ende und einem distalen Ende versehen ist;
Einen Unterstützungs-Stent, von dem wenigstens ein Abschnitt sich innerhalb des röhrenförmigen synthetischen Gewebes befindet, das mit einem proximalen Ende und einem distalen Ende versehen ist;
Wobei der unterstützende-Stent mindestens einen Draht umfasst, der einen ersten spiralförmigen Drahtabschnitt mit einer ersten translatorischen Richtung um eine Achse des Unterstützungs-Stents und einen zweiten spiralförmigen Drahtabschnitt mit einer zweiten translatorischen Richtung um die entgegengesetzte Achse der ersten translatorischen Richtung umfasst;
Wobei, wenn der unterstützende Stent sich in einer komprimierten Einführungs Konfiguration befindet, der unterstützende Stent eine erste Länge hat und wobei, wenn der unterstützende Stent sich in einer ausgedehnten, ausgefahrenen Konfiguration befindet, der unterstützende Stent eine zweite Länge hat, die kürzer ist als die erste Länge; und
Wobei, wenn der unterstützende Stent sich in der Einführungskonfiguration befindet, der unterstützende-Stent nur am synthetischen Gewebe am oder nahe am proximalen Ende des synthetischen Gewebe befestigt ist und das synthetische Gewebe um einen inneren Katheter eines Einführungs -Systems positioniert ist, wobei der genannte innere Katheter wenigstens einen Abschnitt des unterstützenden Stents abdeckt; **dadurch gekennzeichnet, dass** der unterstützende Stent darüber hinaus wenigstens einen Verriegelungsmechanismus am oder nahe am distalen Ende des synthetischen Gewebe umfasst und konfiguriert ist, um den unterstützenden Stent in der ausgefahrenen Konfiguration einzurasten und dabei im Wesentlichen den Durchmesser und die Länge des unterstützenden Stents in der ausgefahrenen Konfiguration zu befestigen.

2. Stent-Graft gemäß Anspruch 1, bei dem der unterstützende Stent in der entfaltenden Konfiguration eine Krümmung hat, die ungefähr einer Krümmung des Blutgefäßes entspricht.

3. Stent-Graft gemäß Anspruch 1 oder 2, bei dem die entfaltende Konfiguration vor der Entfaltung innerhalb des Blutgefäßes eingestellt wird.

4. Stent-Graft gemäß einem der vorhergehenden Ansprüche, bei dem der Verriegelungsmechanismus einen Ring und wenigstens ein Einrastmechanismus umfasst, das zum Einrasten des unterstütenden-Stents konfiguriert ist.

5. Stent-Graft gemäß Anspruch 4, bei dem der Einrastmechanismus wenigstens einen Ausläufer oder Aufhanger umfasst, der konfiguriert ist, um wenigstens einen ersten spiralförmigen Drahtabschnitt und einen zweiten spiralförmigen Drahtabschnitt einrasten lässt.

6. Stent-Graft gemäß einem der vorhergehenden Ansprüche, bei dem wenigstens der eine Einrastmechanismus einen Stent am distalen Ende des Stents umfasst.

7. Stent-Graft gemäß einem der vorhergehenden Ansprüche, der wenigstens einen Verankerungsstent umfasst, der an wenigstens einem Ende des synthetischen Gewebes befestigt ist.

8. Stent-Graft gemäß Anspruch 7, bei dem der wenigstens eine Verankerungsstent am synthetischen Gewebe am oder nahe am proximalen Ende des synthetischen Gewebes befestigt ist.

9. Stent-Graft gemäß einem der vorhergehenden Ansprüche, bei dem die zweite Länge des Stents gleich oder größer als die Länge des synthetischen Gewebes ist.

10. Stent-Graft gemäß einem der vorhergehenden Ansprüche, umfassend einen distalen Ring, der am oder nahe am distalen Ende des synthetischen Gewebes befestigt ist.

11. Stent-Graft gemäß Anspruch 10, bei dem der distale Ring den oder einen Verriegelungsmechanismus umfasst.

12. Stent-Graft gemäß einem der vorhergehenden Ansprüche, bei dem der unterstützende Stent in der entfaltenden Konfiguration wenigstens teilweise im synthetichen Gewebe positioniert ist.

13. Stent-Graft gemäß einem der vorhergehenden Ansprüche, umfassend einen Erfassungsmechanismus, der operativ an das proximale Ende des unterstützenden Stents gekoppelt ist, wobei der Erfassungsmechanismus konfigurierbar ist, um das proximale Ende des unterstützenden Stents in der Einführungs - Konfiguration zurückzuhalten und zu betätigen ist, um das proximale Ende des unterstützenden Stents freizusetzen.

14. Stent-Graft gemäß einem der vorhergehenden Ansprüche, bei dem der unterstützende Stent in der entfaltenden Konfiguration eine Krümmung zwischen 0° und 180° hat.

15. Einführungs -System, umfassend einen Stent Graft gemäß einem der vorhergehenden Ansprüche.

## Revendications

1. Endoprothèse pouvant être déployée dans un vaisseau du corps, comprenant :
un greffon tubulaire comportant une extrémité proximale et une extrémité distale ;
un stent de support, dont au moins une partie se trouve à l'intérieur du greffon tubulaire, comportant une extrémité proximale et une extrémité distale ;
dans laquelle le stent de support comprend au moins un fil formant une première partie de fil hélicoïdal ayant une première direction de translation autour d'un axe du stent de support et une seconde partie de fil hélicoïdal ayant une seconde direction de translation autour de l'axe opposé à la première direction de translation ;
dans laquelle, lorsque le stent de support est dans une configuration d'insertion comprimée, le stent de support présente une première longueur, et dans laquelle lorsque le stent de support est dans une configuration déployée étendue, le stent de support présente une seconde longueur qui est inférieure à la première longueur ; et
dans laquelle, lorsqu'il est dans la configuration d'insertion, le stent de support est attaché au greffon, sensiblement à l'extrémité proximale de ce dernier, et le greffon est positionné autour d'un tube interne d'un système d'introduction, lequel tube interne couvre au moins une partie du stent de support ; **caractérisée en ce que** l'endoprothèse comprend en outre au moins un mécanisme de verrouillage sensiblement à l'extrémité distale du greffon et configuré de sorte à s'unir au stent de support dans la configuration déployée, permettant de manière substantielle de déterminer le diamètre et la longueur du stent de support dans la configuration déployée.

2. Endoprothèse selon la revendication 1, dans laquelle le stent de support dans la configuration déployée a une courbure proche de celle du vaisseau du corps.

3. Endoprothèse selon les revendications 1 ou 2, dans laquelle la configuration déployée est prédéfinie avant déploiement dans le vaisseau du corps.

4. Endoprothèse selon l'une quelconque des revendications précédentes, dans laquelle le mécanisme de verrouillage comprend un anneau et au moins un membre d'engagement configuré pour s'unir au stent de support.

5. Endoprothèse selon la revendication 4, dans lequel le membre d'engagement comprend au moins une pointe configurée pour s'unir à au moins une des première et seconde parties de fil hélicoïdal.

6. Endoprothèse selon l'une quelconque des revendications précédentes, dans laquelle le au moins un mécanisme de verrouillage comprend un stent à l'extrémité distale du greffon.

7. Endoprothèse selon l'une quelconque des revendications précédentes, comprenant au moins un stent d'ancrage fixé à au moins une extrémité du greffon.

8. Endoprothèse selon la revendication 7, dans laquelle la au moins un stent d'ancrage comprend un stent attaché sensiblement à l'extrémité proximale du greffon.

9. Endoprothèse selon l'une quelconque des revendications précédentes, dans laquelle la seconde longueur du stent de support est égale ou supérieure à la longueur du greffon.

10. Endoprothèse selon l'une quelconque des revendications précédentes, comprenant un anneau distal attaché sensiblement à l'extrémité distale du greffon.

11. Endoprothèse selon la revendication 10, dans laquelle l'anneau distal comprend le ou un mécanisme de verrouillage.

12. Endoprothèse selon l'une quelconque des revendications précédentes, dans laquelle, dans la configuration déployée, le stent de support est positionné au moins partiellement à l'intérieur du greffon.

13. Endoprothèse selon l'une quelconque des revendications précédentes, comprenant un mécanisme de capture couplé de façon fonctionnelle avec l'extrémité proximale du stent de support, ce mécanisme de capture étant configuré pour retenir l'extrémité proximale dans la configuration d'insertion et pour être actionnable pour libérer ladite extrémité proximale du stent de support.

14. Endoprothèse selon l'une quelconque des revendications précédentes, dans laquelle le stent de support dans la configuration déployée a une courbure comprise entre 0 et 180°.

15. Système d'introduction comprenant une endoprothèse telle que revendiquée dans l'une quelconque des revendications précédentes.
